# EUROPEAN PATENT APPLICATION

(11) **EP 0 691 343 A1**
(43) Date of publication of application: **10.01.1996**
(21) Application number: 94910527.4
(22) Date of filing: 24.03.1994
(51) Int. Cl.: C07D 501/20, C07D 501/22, A61K 31/38

(54) **CEPHALOSPORIN DERIVATIVE**

(30) Priority: 26.03.1993 JP 92589/93; 29.03.1993 JP 93605/93; 22.12.1993 JP 324707/93; 22.02.1994 JP 24256/94
(71) Applicant: YAMANOUCHI PHARMACEUTICAL CO. LTD., Tokyo 103 (JP)
(72) Inventor: HARA, Ryuichiro, Tsukuba-shi, Ibaraki 305 (JP); ITAHANA, Hirotsune, Tsukuba-shi, Ibaraki 305 (JP); SAKAMOTO, Kenichiro, Tsukuba-shi, Ibaraki 305 (JP); HISAMICHI, Hiroyuki, Tsukuba-shi, Ibaraki 305 (JP); NAGANO, Noriaki, Ryugasaki-shi, Ibaraki 301 (JP)
(74) Representative: Geering, Keith Edwin
(86) International application number: JP9400472
(87) International publication number: WO9422874

(57) **Abstract**

A cephalosporin derivative represented by general formula (I) or a salt thereof, wherein R¹ represents hydrogen, lower alkenyl, lower alkynyl, cycloalkyl, hydroxyl-protecting group, or (un)substituted lower alkyl; R² represents hydrogen, ester residue, or negative charge; R³ represents either (1) a group represented by general formula (a), wherein R⁴ and R⁵ represent each independently lower alkoxy or (un)substituted amino, or R⁴ and R⁵ may be combined together with the neighboring tetrahydropyridazine ring to form an (un)substituted fused 5- or 6-membered ring, or (2) a group represented by general formula (b) or (c), wherein R⁶ and R⁷ represent each independently hydrogen, halogen, amino, azido, carbamoyl, lower alkyl which may be substituted by amino, azido, carbamoyl or halogen, carbamoyl, or (un)substituted 5- or 6-membered heterocyclic group (provided the nitrogen atom of the heterocycle may be substituted to form a quaternary amine); X represents methine (-CH=) or nitrogen; and ring A represents a 4- to 5-membered nitrogenous heterocycle which may contain oxygen.

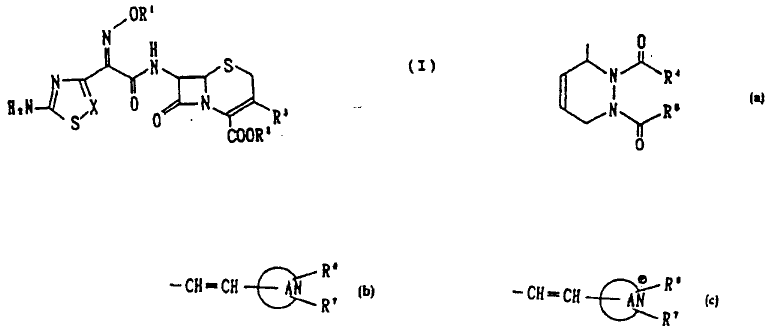

## Description

### TECHNICAL FIELD

This invention relates to cephalosporin derivatives or salts thereof useful as medicines, particularly as antibacterial agents, and to cephalosporin derivative intermediates or salts thereof.

### BACKGROUND ART

Since cephalosporin antibacterial agents show a broad range of antibacterial activities against Gram-positive bacteria, Gram-negative bacteria and the like, a number of studies have been conducted on the synthesis of cephalosporin compounds.

For example, unexamined published Japanese Patent Application (*Kokai*) No. 3-232891 discloses a compound in which isooxazolidine ring is linked to the 3-position of the cephalosporin nucleus, and unexamined published Japanese Patent Application (*Kokai*) No. 56-55392 discloses a compound having a vinyl group substituted with a lower alkoxycarbonyl group or cyano group.

In addition, unexamined published Japanese Patent Application (*Kokai*) No. 1-156984 discloses a compound in which 3-(carbamoylmethylethylmethylammonio)-1-propen-1-yl group is introduced into the 3-position of the cephalosporin nucleus.

However, great concern has been directed toward the development of more excellent cephalosporin antibacterial agents.

### DISCLOSURE OF THE INVENTION

The inventors of the present invention have conducted intensive studies on the synthesis of cephalosporin derivatives and, as the result, found that a compound whose chemical structure is different from the prior art compounds, in which the 3-position of cephalosporin is linked with a vinyl group substituted with a nitrogen-containing hetero ring or a quaternary salt thereof which may contain oxygen atom or with a six-membered heterocyclic group containing 2 nitrogen atoms, shows excellent antibacterial activities against Gram-positive bacteria and Gram-negative bacteria, hence resulting in the accomplishment of the present invention. An object of the present invention is to provide a cephalosporin derivative represented by the following general formula (I) which is effective even against antibiotic resistant strains such as MRSA and the like. Another object is to provide compounds represented by the following general formulae (II) and (A) which are useful as intermediates for use in the synthesis of the inventive cephalosporin derivative (I).

The present invention relates to a cephalosporin derivative represented by the following general formula (I) or a pharmaceutically acceptable salt thereof. 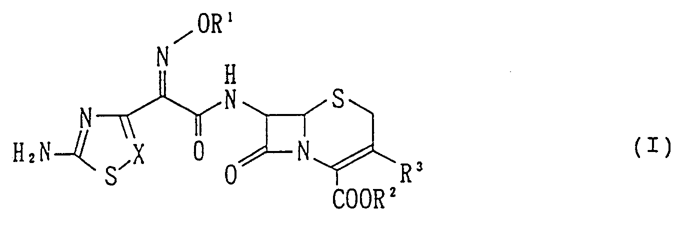
(symbols in the formula have the following meanings.
R¹: a hydrogen atom, a lower alkenyl group, a lower alkynyl group, a cycloalkyl group, an protective group for the hydroxyl group or a substituted or unsubstituted lower alkyl group,
R²: a hydrogen atom, an ester residue or a negative charge,

R³: (1) a group represented by the following formula 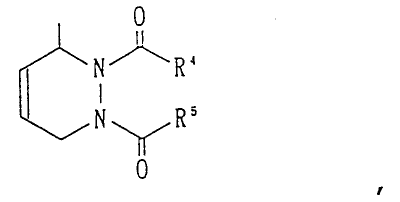
R⁴, R⁵: the same or different from each other and each represents a lower alkoxy group or a substituted or unsubstituted amino group, or R⁴ and R⁵ may be combined together with the neighboring tetrahydropyridazine ring to form a substituted or unsubstituted condensed five- or six-membered cyclic group, or
(2) a group represented by the following formula: 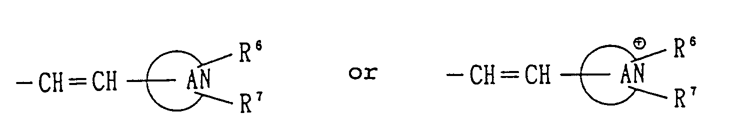
R⁶, R⁷: the same or different from each other and each represents a hydrogen atom, a halogen atom, an amino group, an azide group, an unsubstituted or amino-, azide-, carbamoyl- or halogen-substituted lower alkyl group, a carbamoyl group or a substituted or unsubstituted five- or six-membered heterocyclic group (nitrogen atom of the hetero ring may be substituted to form a quaternary amine together with substituent groups),
X: a methine group (-CH=) or a nitrogen atom, and
ring A: a four- or five-membered nitrogen-containing hetero ring which may contain an oxygen atom, the same shall apply hereinafter);
to a cephalosporin derivative represented by the following general formula (II) 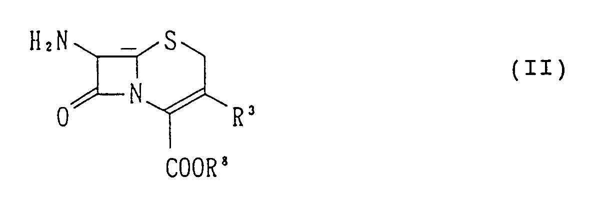
(R⁸ represents a hydrogen atom, a negative charge or a protective group for the carboxyl group, the same shall apply hereinafter); and
a cephalosporin derivative represented by the following formula (A) or a pharmaceutically acceptable salt thereof 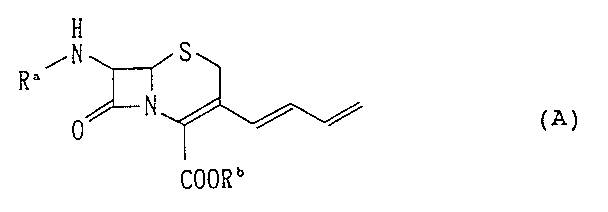
(R^{a}: a hydrogen atom, a phenylacetyl group, a group represented by the following formula 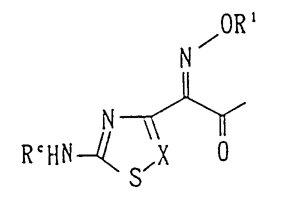
or a protective group for the amino group,
R^{c}: a hydrogen atom or a protective group for the amino group,
R^{b}: a hydrogen atom or a protective group for the carboxyl group, the same shall apply hereinafter).

The following describes the compounds (I), (II) and (A) of the present invention in detail.

Unless otherwise indicated, the term "lower" as used herein in the definition of the general formulae means a straight or branched carbon chain having 1 to 6 carbon atoms.

Illustrative examples of the "unsubstituted lower alkyl group" of R¹ include methyl, ethyl, propyl, isopropyl, butyl, isobutyl, sec-butyl, tert-butyl, pentyl (amyl), isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl and the like. Of these groups, a methyl group, an ethyl group and a propyl group are preferred.

The "lower alkenyl group" is a straight- or branched-chain alkenyl group having 2 to 6 carbon atoms, which illustratively includes vinyl, propenyl, butenyl, methylpropenyl, methylpropenyl, dimethylvinyl, pentenyl, methylbutenyl, dimethylpropenyl, ethylpropenyl, hexenyl, dimethylbutenyl, methylpentenyl and the like. Of these groups, a vinyl group, a propenyl group or a butenyl group is preferred.

The "lower alkynyl group" is a straight- or branched-chain alkynyl group having 2 to 6 carbon atoms, which illustratively includes ethynyl, propynyl, butynyl, methylpropynyl, pentynyl, methylbutynyl, hexynyl and the like. Of these groups, an ethynyl group, a propynyl group, or a butynyl group is preferred.

Illustrative examples of the "cycloalkyl group" include those having 3 to 8 carbon atoms, such as cyclopropyl, cyclobutyl, cyclopentyl, cyclohexyl, cycloheptyl, cyclooctyl and the like. Of these groups, a cyclopentyl group or a cyclohexyl group is preferred.

The "substituted lower alkyl group" means a lower alkyl group substituted at optional positions with 1 or more substituents such as a halogen atom, a carboxyl group, a lower alkoxycarbonyl group, a cycloalkyl group and the like, and the substituent cycloalkyl group may be unsubstituted or substituted with 1 or more substituents such as a lower alkyl group, a lower alkoxy group, a halogen atom, a carboxyl group and a lower alkoxycarbonyl group.

In this connection, the "lower alkyl group" is as defined in the foregoing. Illustrative examples of the "lower alkoxycarbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxy(amyloxy)carbonyl, isopentyloxycarbonyl, tert-pentyloxycarbonyl, neopentyloxycarbonyl, 2-methylbutoxycarbonyl, 1,2-dimethylpropoxycarbonyl, 1-ethylpropoxycarbonyl, hexyloxycarbonyl and the like.

Illustrative examples of the "protective group for the hydroxyl group" include a lower alkylsilyl group such as trimethylsilyl, tert-butyldimethylsilyl or the like; a lower alkoxymethyl group such as methoxymethyl, 2-methoxyethoxymethyl or the like; tetrahydropyranyl group; an aralkyl group such as benzyl, p-methoxybenzyl, 2,4-dimethoxybenzyl, o-nitrobenzyl, p-nitrobenzyl, trityl or the like; an acyl group such as formyl, acetyl or the like; a lower alkoxycarbonyl group such as tert-butoxycarbonyl, 2-iodoethoxycarbonyl, 2,2,2-trichloroethoxycarbonyl or the like; an alkenyloxycarbonyl group such as 2-propenyloxycarbonyl, 2-chloro-2-propenyloxycarbonyl, 3-methoxycarbonyl-2-propenyloxycarbonyl, 2-methyl-2-propenyloxycarbonyl, 2-butenyloxycarbonyl, cinnamyloxycarbonyl or the like; and an aralkyloxycarbonyl such as benzyloxycarbonyl, p-methoxybenzyloxycarbonyl, o-nitrobenzyloxycarbonyl, p-nitrobenzyloxycarbonyl or the like group; of which 2-propenyloxycarbonyl group, p-nitrobenzyloxycarbonyl group, tert-butyldimethylsilyl group and the like are preferred.

The "cycloalkyl group" is as defined in the foregoing, and illustrative examples of the "lower alkoxy group" include methoxy, ethoxy, propoxy, isopropoxy, butoxy, isobutoxy, sec-butoxy, tert-butoxy, pentyloxy (amyloxy), isopentyloxy, tert-pentyloxy, neopentyloxy, 2-methylbutoxy, 1,2-dimethylpropoxy, 1-ethylpropoxy, hexyloxy and the like.

The "halogen atom" means a fluorine atom, a chlorine atom, a bromine atom or an iodine atom.

The "ester residue" of R² is an ester residue which is metabolically hydrolyzed in the living body, and its illustrative examples thereof include commonly used ester residues such as a lower alkanoyloxy-lower alkyl group, a lower alkenoyl-lower alkyl group, a cycloalkylcarbonyloxy-lower alkyl group, a lower alkenoyloxy-lower alkyl group, a lower alkoxy-lower alkanoyloxy-lower alkyl group, a lower alkoxy-lower alkyl group, a lower alkoxy-lower alkoxy-lower alkyl group, a lower alkoxycarbonyloxy-lower alkyl group, a lower alkoxy lower carbonyloxy-lower alkyl group, a benzoyloxy-lower alkyl group, a 2-oxotetrahydrofuran-5-yl group, a 2-oxo-5-alkyl-1,3-dioxolen-4-ylmethyl group, 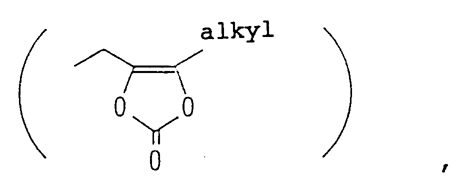
a tetrahydrofuranylcarbonyloxymethyl group, a 3-phthalidyl group, and the like.

With respect to the group represented by the following formula 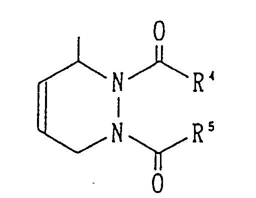
in the definition of R³, the "substituted amino group" of R⁴ and R⁵ is an amino group substituted with, for example, 1 or 2 of the aforementioned alkyl groups, and its illustrative examples include methylamino, ethylamino, propylamino, dimethylamino, diethylamino, methylethylamino and the like.

Illustrative examples of the "lower alkoxy group" are as described in the foregoing, of which methoxy group, ethoxy group and propoxy group are preferred.

Examples of the "substituted or unsubstituted condensed five- or six-membered cyclic group formed by R⁴ and R⁵ taken together with the adjacent tetrahydropyridazinyl group" include the groups represented by 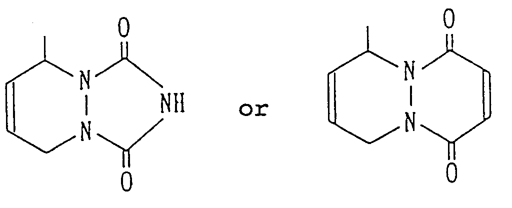
and these rings may have a "lower alkyl group" as a substituent at the optional position, and examples of such a "lower alkyl group" are as described in the foregoing, preferably methyl, ethyl and propyl groups.

With respect to the group represented by the following formula 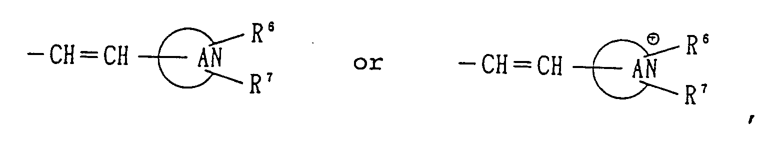
the "halogen atom" in the meaning of R⁶ or R⁷ is a fluorine atom, a chlorine atom, a bromine atom or an iodine atom. Examples of the "lower alkyl group" are as described in the foregoing, preferably methyl, ethyl and propyl groups. Examples of the "substituted or unsubstituted five- or six-membered heterocyclic group" include pyridyl, furyl, 2-nitrofuryl and the like. Examples of the substituent which forms a quaternary amine by linking to the nitrogen atom of the heterocyclic group include trimethylpyridinio, 1-methylpyridinio, 1-ethylpyridinio and the like.

Illustrative examples of the "four- or five-membered nitrogen-containing hetero ring which may contain an oxygen atom" in the meaning of the "ring A" include azetidinyl, pyridyl, oxazolyl, oxazolinyl, oxazolidinyl, isooxazolyl, isooxazolinyl, isooxazolidinyl, pyridinio, oxazolinio, oxazolidinio, isooxazolidinio, isooxazolinio and the like.

Any oxygen atom-containing five-membered nitrogen-containing hetero ring may be used preferably, and particularly preferred groups are those which are not aromatized, such as isooxazolinyl group, isooxazolidinyl group, isooxazolinio group and isooxazolidinio group.

The "protective group for the amino group" in the compounds of the present invention means a protective group generally used by those skilled in the art, and typical examples of the acyl-type protective group for the amino group include a lower alkanoyl group such as formyl, acetyl, propionyl or the like; a lower alkoxycarbonyl group such as methoxycarbonyl, ethoxycarbonyl, tert-butoxycarbonyl or the like; a lower alkanesulfonyl group such as methanesulfonyl, ethanesulfonyl or the like; a lower alkoxycarbonyl group such as tert-butoxycarbonyl (to be referred to as BOC hereinafter) or the like; and an aliphatic acyl group such as methoxyacetyl, methoxypropionyl, benzoyl, benzyloxycarbonyl, p-nitrobenzyloxycarbonyl or the like; or heterocyclic acyl groups including a heterocyclic lower alkanoyl group such as thienylacetyl, thiazolylacetyl, tetrazolylacetyl or the like and a heterocyclic glyoxyloyl group such as azolylglyoxyloyl, thienylglyoxyloyl or the like. Examples of the aralkyl-type protective group for the amino group include benzyl, p-methoxybenzyl (to be referred to as PMB hereinafter), benzhydryl, trityl and the like. Also useful are tri-lower alkylsilyl groups such as a trimethylsilyl group and the like.

Illustrative examples of protective groups for the carboxyl group include a lower alkyl group such as methyl, ethyl, propyl, isopropyl, tert-butyl or the like; a halo-substituted lower alkyl group such as 2,2,2-trichloroethyl, 2,2,2-trifluoroethyl or the like; a lower alkanoyloxyalkyl group such as acetoxymethyl, propionyloxymethyl, pivaloyloxymethyl, 1-acetoxyethyl, 1-propionyloxyethyl or the like; a lower alkoxycarbonyloxyalkyl group such as 1-(methoxycarbonyloxy)ethyl, 1-(ethoxycarbonyloxy)ethyl, 1-(isopropoxycarbonyloxy)ethyl or the like; a lower alkenyl group such as 2-propenyl, 2-chloro-2-propenyl, 3-methoxycarbonyl-2-propenyl, 2-methyl-2-propenyl, 2-butenyl, cinnamyl or the like; an aralkyl group such as benzyl, p-methoxybenzyl, 3,4-dimethoxybenzyl, o-nitrobenzyl, p-nitrobenzyl, benzhydryl, bis(p-methoxyphenyl)methyl or the like; a (5-substituted-2-oxo-1,3-dioxol-4-yl)methyl group such as (5-methyl-2-oxo-1,3-dioxol-4-yl)methyl or the like; a lower alkylsilyl group such as trimethylsilyl, tert-butyldimethylsilyl or the like; and indanyl, phthalidyl, methoxymethyl and the like; of which a 2-propenyl group, a p-nitrobenzyl group, a p-methoxybenzyl group, a benzhydryl group, a tert-butyldimethylsilyl group and the like are preferred.

The compound (I) or (II) of the present invention can form an intramolecular salt between the 4-position carboxyl group and a certain type of the 3-position substituent. In some cases, a salt may be formed with other acid or base when the 4-position has a carboxyl group or depending on the type of the 3-position substituent.

Such a salt is formed with a pharmaceutically acceptable acid or base, and examples of the acid salt include acid addition salts with mineral acids such as hydrochloric acid, hydrobromic acid, hydroiodic acid, sulfuric acid, nitric acid, phosphoric acid and the like or with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, glutamic acid and the like.

Examples of the base salt include salts with inorganic bases such as sodium, potassium, magnesium, calcium, aluminium and the like, organic bases such as methylamine, ethylamine, ethanolamine and the like and basic amino acids such as lysine, arginine, ornithine and the like, as well as an ammonium salt.

The compounds (I) and (A) of the present invention have 2-aminothiazolyl group or 5-aminothiadiazolyl group at the 7-position, and it contains an imino ether-type oxime, so that syn, anti and the like geometrical isomers, optical isomers, tautomers and the like based thereon are all included in the inventive compounds. Also, cis (Z) and trans (E) type geometrical isomers are present, because the 3-position substituent contains a double bond. An optical isomer which shows R, S or RS is also present due to carbon atoms of the four- or five-membered nitrogen-containing hetero ring which may contain an oxygen atom, linked to the vinyl group at the 3-position. These Z and E type geometrical isomers and optical isomers based on the 3-position substituent group, as well as their mixtures, are all included in the compound (II) of the present invention. Also, compounds of the present invention can also form hydrates, solvates with ethanol and the like or polymorphic forms.

In addition, Z and E type geometrical isomers due to the 7-position substituent and their mixture are included in 7β-[2-(2-amino-4-thiazolyl)-2-(hydroxyimino)acetamido]-3-[(E)-2-(2,2-dimethyl-5-isooxazolidinio)vinyl]-3-cephem-4-carboxylate and 7β-[2-(5-amino-1,2,4-thiadiazol-3-yl)-2-hydroxyiminoacetamido]-3-[(E)-2-((RS)-2,2-dimethyl-5-isooxazolidinio)vinyl]-3-cephem-4-carboxylate.

In the compounds (I) and (II) of the present invention, R³ is preferably a group represented by the following general formula 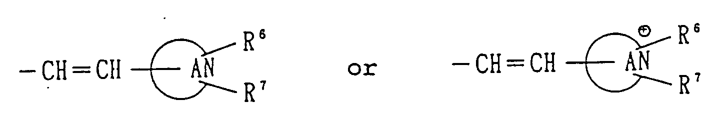
more preferably a group represented by the following general formula 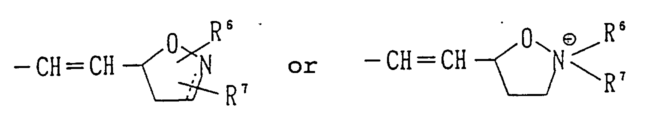
(wherein the dotted line may form a double bond).

### (Production method)

The compounds of the present invention and intermediates thereof can be produced by employing various synthesis methods. The following describes typical production processes thereof.

### Production process 1

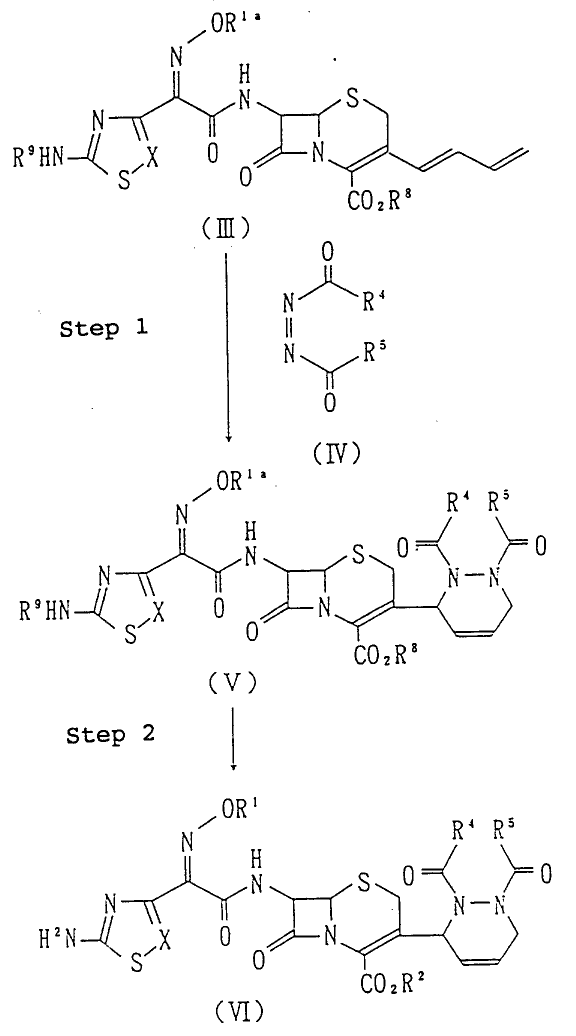

(In the above formulae, R^{1a} represents a group resulting from the elimination of the hydrogen atom from R¹, and R⁹ represents a protective group for the amino group.)

The compound (VI) of the present invention can be produced by carrying out a cyclization reaction of a butadiene compound represented by the general formula (III) with an azodicarboxylate represented by the general formula (IV) to obtain a tetrahydropyridazinyl compound represented by the general formula (V) (first step) and then eliminating the protective group from the compound (V) (second step). The first step is carried out by stirring the compound (III) and its reaction equivalent amount of the compound (IV) in an inert solvent with cooling or heating.

As the inert solvent, an organic solvent such as methylene chloride, chloroform, acetonitrile, benzene, toluene or the like may be used.

Elimination of a protective group in the second step can easily be effected in the usual way by, for example, treatment with water when the protective group is a tri-lower alkylsilyl group or the like. When the protective group is benzhydryl, p-methoxybenzyl, trityl, tert-butyl, formyl or the like, it can be eliminated easily by treating it with an acid such as formic acid, trifluoroacetic acid, a trifluoroacetic acid-anisole mixture, a hydrobromic acid-acetic acid mixture, a hydrochloric acid-dioxane mixture or the like.

### Production process 2

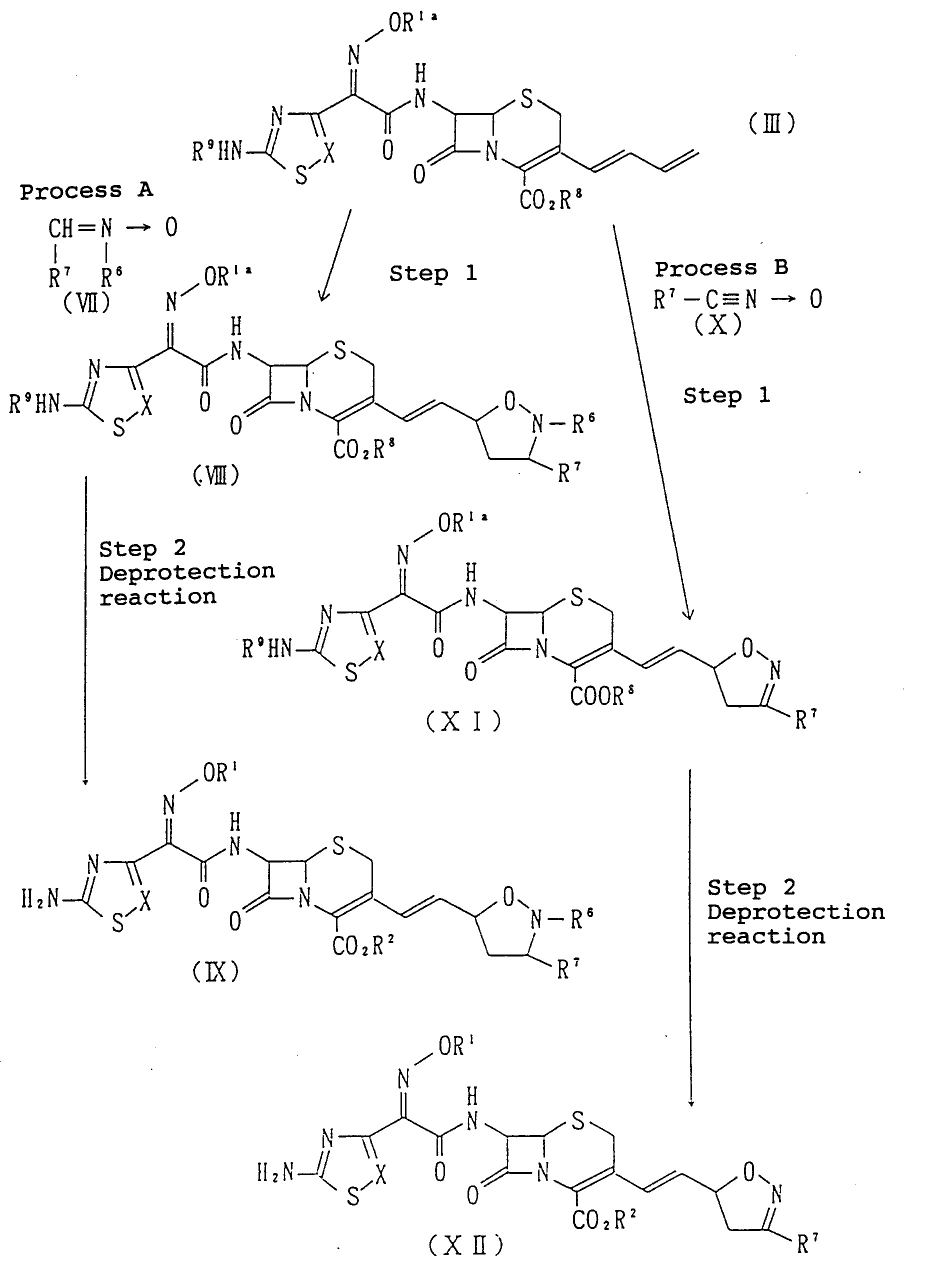

The production process 2 is a process for the production of a compound in which the 3-position of the cephalosporin nucleus has a vinyl group substituted with a nitrogen-containing five-membered hetero ring which contains an oxygen atom.

In the process A, the object compound (IX) of the present invention is produced by carrying out a cyclization reaction of a butadiene compound represented by the general formula (III) with a nitron compound represented by the general formula (VII) to obtain an isooxazolidinyl compound represented by the general formula (VIII) (first step) and then eliminating the protective group from the thus obtained compound (VIII) if desired (second step).

The first step is effected by allowing the compound (III) to react with a reaction equivalent amount of the compound (VII) in an inert solvent at room temperature or with heating.

As the inert solvent, methylene chloride, chloroform, acetonitrile, ethyl acetate, tetrahydrofuran or the like may be used.

Elimination of protective group in the second step can be effected in the same manner as described in the foregoing.

In the process B, the compound (XII) of the present invention is produced by carrying out a cyclization reaction in the same manner as the process A, except that a nitrile oxide compound (X) is used instead of the nitron compound (VII), to obtain an isooxazolinyl compound represented by the general formula (XI) (first step) and then eliminating the protective group from the thus obtained compound (XI) if desired (second step).

The first step is effected by allowing the butadiene compound represented by the general formula (III) to react with a reaction equivalent amount of the compound (X) in an inert solvent such as methylene chloride, chloroform, acetonitrile, ethyl acetate, dioxane or the like with cooling or heating.

Elimination of protective group in the second step can be effected in the same manner as described in the foregoing.

### Production process 3

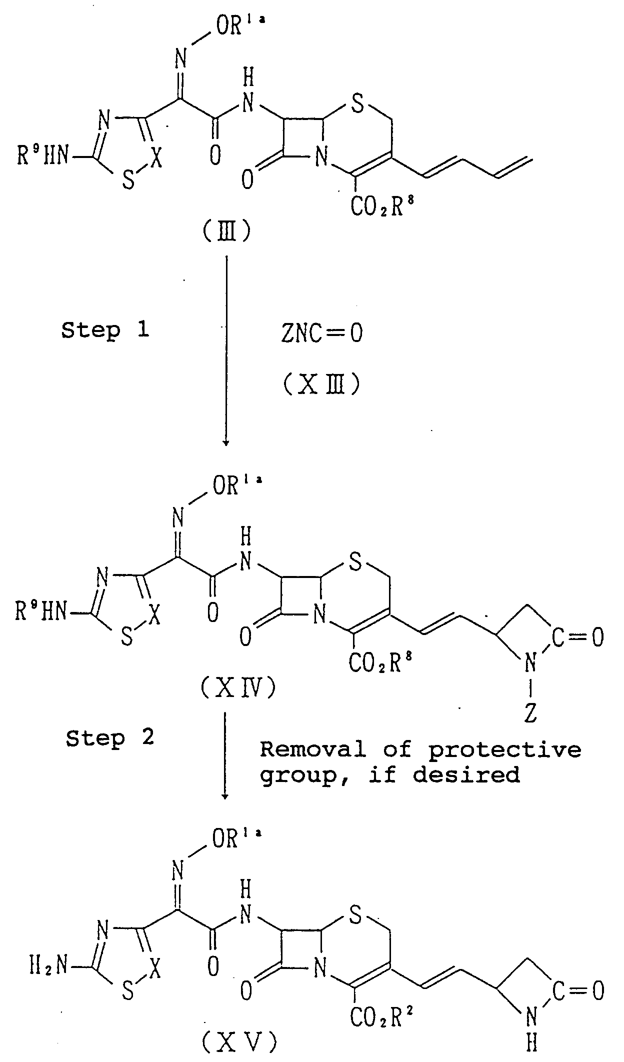

(In the above formulae, Z represents a halogenosulfonyl group.)

The production process 3 is a process for the production of a compound in which the 3-position of the cephalosporin nucleus has a vinyl group substituted with a nitrogen-containing four-membered hetero ring.

The object compound (XV) of the present invention is produced by carrying out a cyclization reaction of a butadiene compound represented by the general formula (III) with an isocyanate compound represented by the general formula (XIII) to obtain a lactam compound represented by the general formula (XIV) (first step) and then eliminating the protective group from the thus obtained compound (XIV) if desired (second step).

Alternatively, the lactam compound may be subjected to an N-alkylation reaction prior to the elimination of protective group, thereby effecting introduction of a lower alkyl group.

The first step is effected by stirring the compound (III) and a reaction equivalent amount of the compound (XIII) in an inert solvent with cooling or at room temperature.

As the inert solvent, methylene chloride, acetonitrile, chloroform, ethyl ether, an aliphatic hydrocarbon, an aromatic hydrocarbon or the like may be used. The N-alkylation reaction can be carried out in the usual way. For example, it may be carried out in accordance with the production process 5 which will be described later.

### Production process 4

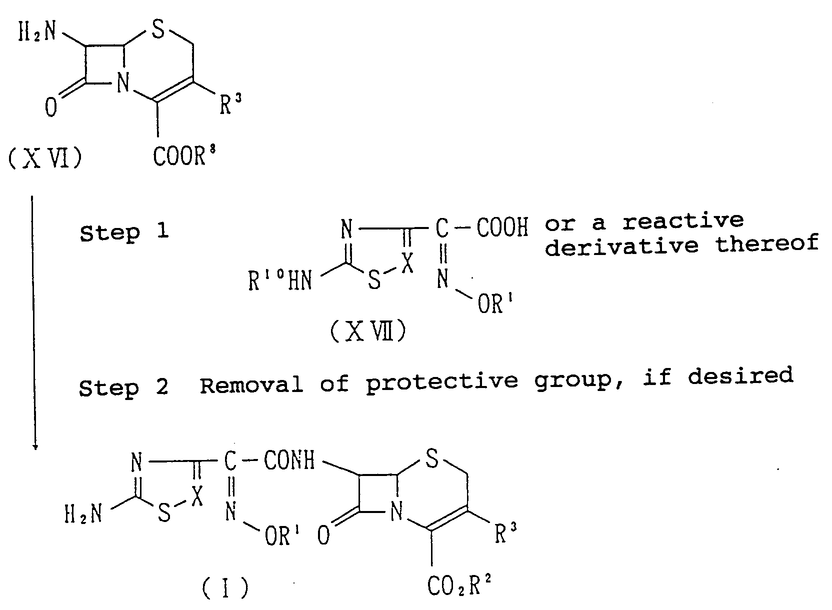

(In the above formulae, R¹⁰ represents an acyl-type protective group for the amino groups in the meaning of R⁹.)

The object compound (I) of the present invention is produced by allowing a 3-substituted-7-amino-3-cephem-4-carboxylic acid (or protected carboxylic acid) represented by the general formula (XVI) to react with an α-(thiazolyl or thiadiazolyl)-α-substituted iminoacetic acid represented by the general formula (XVII) or a reactive derivative thereof and then eliminating the protective group from the reaction product, if necessary.

The reaction of the compound (XVI) with a reaction equivalent amount of the compound (XVII) or its reactive derivative is carried out generally in a solvent with cooling or at room temperature. The solvent is not particularly limited, provided that it does not take part in the reaction. Examples of commonly used solvent include acetone, dioxane, ethyl ether, tetrahydrofuran, methyl ethyl ketone, chloroform, dichloroethane, methylene chloride, ethyl acetate, ethyl formate, dimethylformamide, dimethyl sulfoxide, water and the like. These solvents may optionally be used as an mixture.

The compound (XVII) may be used in the reaction as a form of a free carboxylic acid or as a reactive derivative of the carboxylic acid. Examples of the reactive derivative of the carboxylic acid include an active ester (e.g., benzotriazole ester or the like), a mixed acid anhydrides, an acid halide, an active amide, an acid anhydride, an acid azide and the like. When the compound (XVII) is used in the form of a free carboxylic acid, it is desirable to use a condensing agent such as N,N-dicyclohexylcarbodiimide, N,N-diethylcarbodiimide or the like.

Depending on the type of the reactive derivative of the carboxylic acid to be used, it is desirable in some cases to carry out the reaction in the presence of a base in order to effect smooth progress of the reaction. Examples of the base include inorganic bases such as sodium bicarbonate, potassium bicarbonate, sodium carbonate, potassium carbonate and the like and organic bases such as trimethylamine, triethylamine, dimethylaniline, pyridine and the like.

Elimination of a protective group from the thus obtained product can easily be effected by, for example, treatment with water when the protective group is a tri-lower alkylsilyl group or the like. When the protective group is benzhydryl, p-methoxybenzyl, trityl, tert-butyl, formyl or the like group, it can be eliminated easily by treating it with an acid such as formic acid, a trifluoroacetic acid-anisole mixture, a hydrobromic acid-acetic acid mixture, a hydrochloric acid-dioxane mixture or the like.

### Production process 5 (production of betaine)

When the aforementioned ring A is a betaine (quaternary amine), it can be produced by stirring a nitrogen-containing heterocyclic compound and a lower alkyl halide or a lower alkyl trifurate in an inert solvent such as dimethylformamide, chloroform, benzene or the like at room temperature or with heating.

### Production process 6

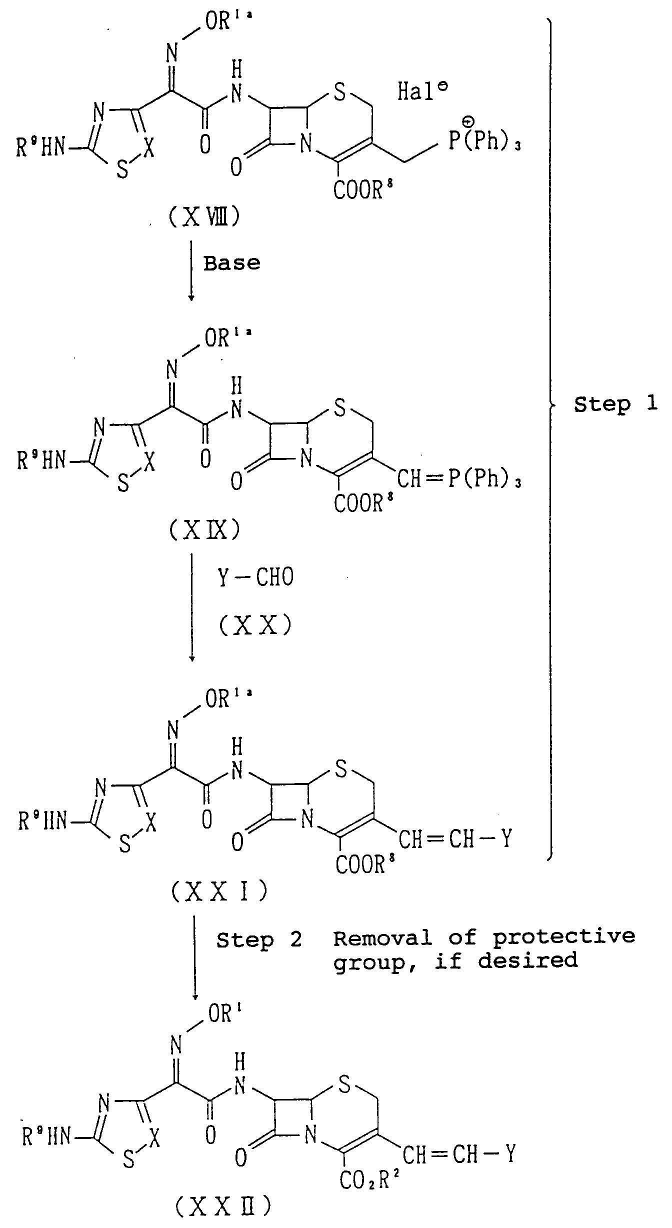

(In the above formulae, Hal represents a halogen atom.)

The object compound (XXII) of the present invention can be produced by allowing a phosphorane compound represented by the general formula (XIX) formed from a triphenylphosphine compound represented by the general formula (XVIII) in the presence of a base to react with a carbonyl compound represented by the general formula (XX) to obtain a lactam compound represented by the general formula (XXI) and then eliminating the protective group of the thus obtained compound.

The first step is the Wittig reaction in which the phosphorane compound represented by the general formula (XIX) is obtained by allowing the compound of general formula (XVIII) to react with a generally used base such as sodium hydroxide, a lithium compound or the like. The compound of general formula (XXI) is then obtained by stirring the thus obtained compound (XIX) and a reaction equivalent amount of the compound of general formula (XX) in an insoluble solvent with cooling or heating. The inert solvent may be selected from methylene chloride, chloroform, tetrahydrofuran, ethyl acetate, dimethyl sulfoxide, benzene, toluene, hexane and the like.

### Production process 7 (salt formation reaction)

Salts of the compound of the present invention can be produced by employing commonly used salt formation reactions.

For example, an alkali metal salt can be produced by adding a butanol solution of an alkali salt of 2-ethylhexanoic acid and then an organic solvent having different solubility, such as ethyl ether, ethyl acetate or the like; a salt with an organic base or basic amino acid can be produced by the reaction with the same or slightly excess amount of an organic base or basic amino acid, such as dicyclohexylamine, triethylamine, cyclohexylamine, diethanolamine, arginine, lysine or the like, and an ammonium salt can be produced by adding an aqueous ammonia.

Isolation and purification of the compound (I) of the present invention and salts thereof can be effected in the usual way by utilizing extraction and crystallization with an organic solvent and separation and purification by column chromatography.

### Production process 8 (production of intermediate)

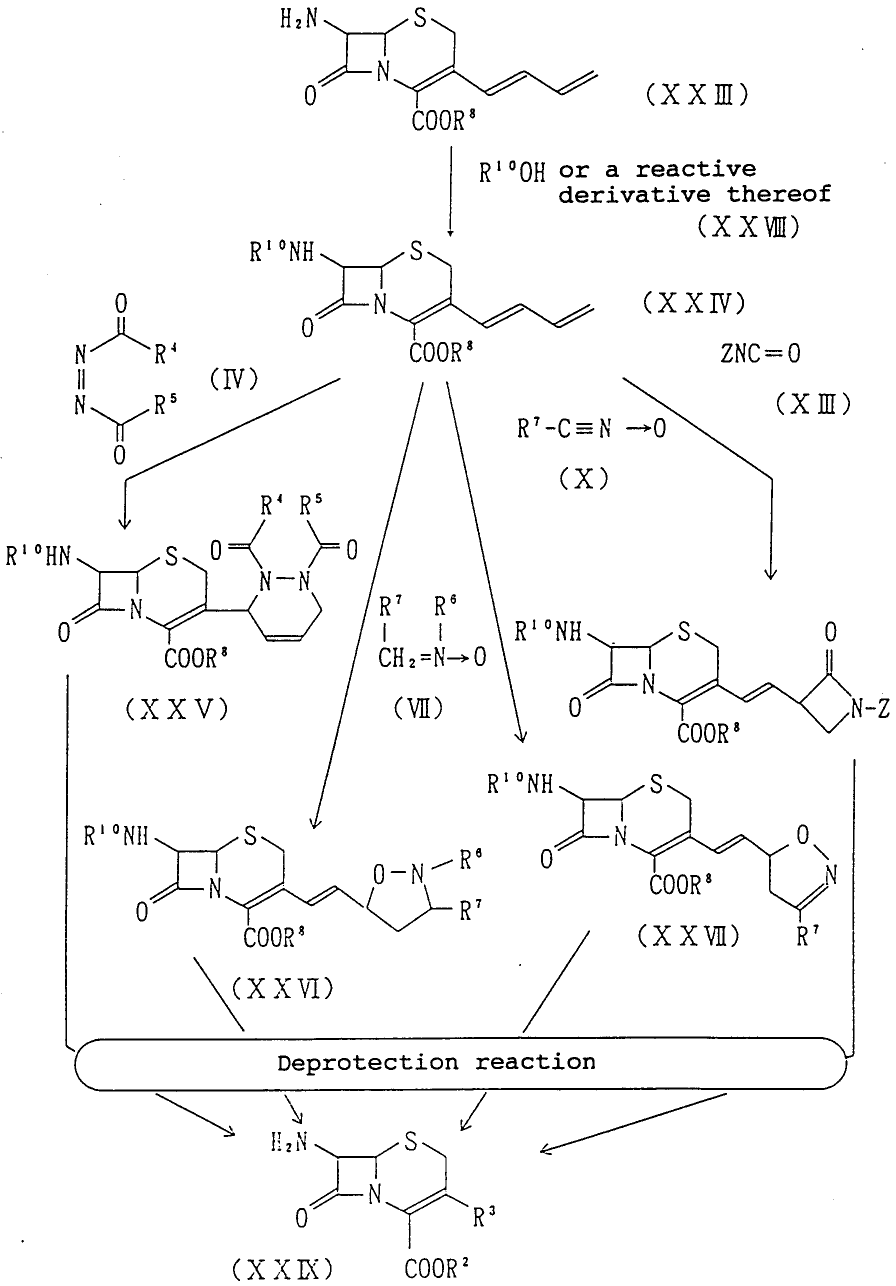

The compound (XXIX) as an intermediate of the compound of the present invention can be produced in the same manner as described in the production processes 1 to 3, except that a 7-acylamino-3-butadiene cephem compound (XXIV) is used instead of the butadiene compound (III) as the starting material.

Accordingly, the production process 7 will be described only about the acylation reaction of the 7-acylamino-3-butadiene cephem compound (XXIV). The acylation reaction can be carried out by stirring a 7-amino-3-butadiene cephem compound (XXIII) and a reaction equivalent amount of a carboxylic acid or a reactive derivative thereof (XXVIII) in an inert solvent at room temperature or with heating. The inert organic solvent may be selected from tetrahydrofuran, dioxane, ethyl ether, benzene, toluene, xylene, methylene chloride, dichloroethane, chloroform, dimethylformamide, ethyl acetate, acetonitrile and the like.

When the compound (XXVIII) is used in the form of a free carboxylic acid, it is advantageous to carry out the reaction using a condensing agent such as dicyclohexylcarbodiimide, 1,1'-carbonyldiimidazole or the like.

Examples of the reactive derivative of the compound (XXVIII) include an acid halide such as acid chloride, acid bromide or the like, an acid azide, an active ester with N-hydroxybenzotriazole, N-hydroxysuccinimide or the like, a symmetric acid anhydride and a mixed acid anhydride such as those with alkyl carbonic acid or p-toluenesulfonic acid.

Depending on the type of the reactive derivative, it is desirable in some cases to carry out the reaction in the presence of an organic base such as triethylamine, pyridine, picoline, lutidine, N,N'-dimethylaniline or the like or an inorganic base such as potassium carbonate, sodium hydroxide or the like, in order to effect smooth progress of the reaction. Pyridine can also serve as a solvent.

### Production process 9

This process is to produce a compound in which the 3-position substituent has a 1,3-butadienyl group. 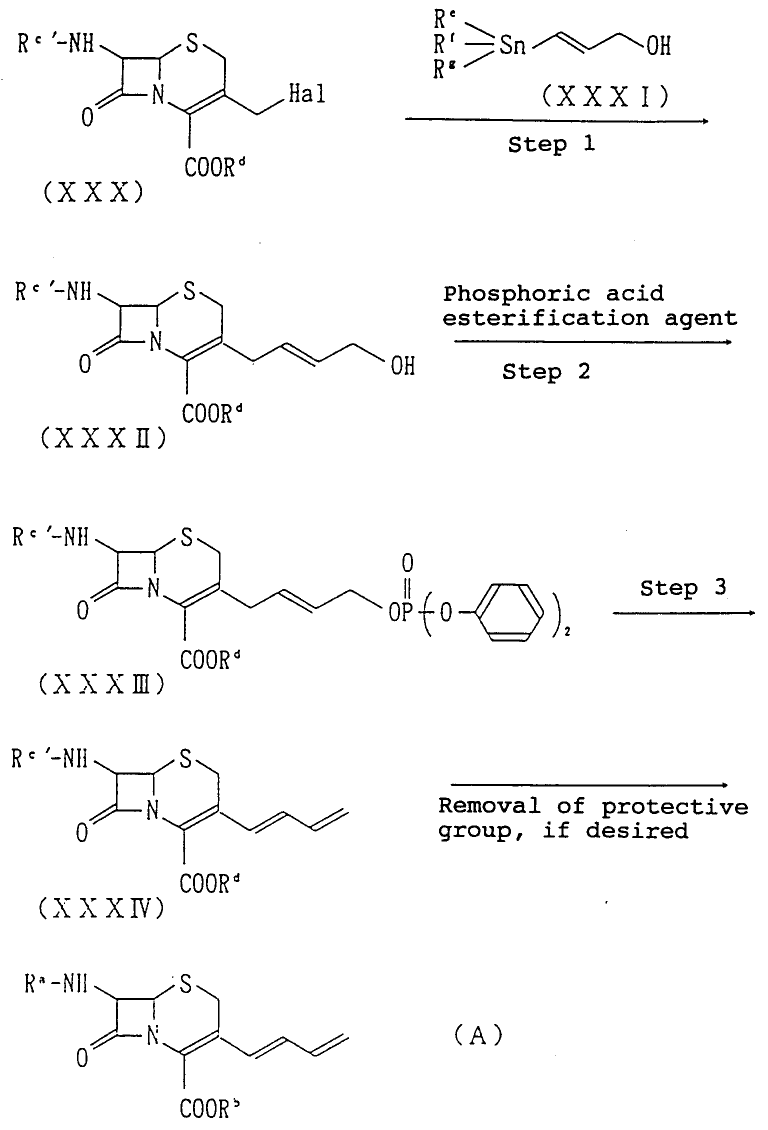
(In the above formulae, symbols other than those already described have the following meanings.
R^{c'}: all members of R^{a} except for the hydrogen atom of R¹ in R^{a},
R^{d}: a protective group for the carboxyl group in the meaning of R^{b}, and
R^{e}, R^{f}, R^{g}: the same or different from one another and each represents a hydrogen atom, a lower alkyl group, an aminocarbonyl group, a lower alkoxy group, a cyano group, a lower alkoxycarbonyl group or a nitro group.)

In this production process, the compound (A) is produced by a series of steps in which a compound represented by the general formula (XXX) is allowed to react with a compound (XXXI) to obtain a compound represented by the general formula (XXXII) which is subsequently allowed to react with a phosphoric acid esterification agent to obtain a compound (XXXIII), and the compound (XXXIII), preferably having E form (trans form) at the 3-position, is allowed to react with a base in the presence or absence of a halogen donor to obtain a compound (XXXIV) which, if desired, is further subjected to the elimination of 1 or 2 protective groups, esterification in the conventinal way and salt formation reaction.

Illustrative examples of the halogen atom include an iodine atom, a bromine atom, a chlorine atom and a fluorine atom.

In the first step of this production process, the compound represented by the general formula (XXX) is allowed to react with a reaction equivalent amount of the compound represented by the general formula (XXXI) in the presence of a palladium compound to obtain the compound represented by the general formula (XXXII).

Illustrative examples of the palladium compound include tetrakis(triphenylphosphine)palladium(0), bis(dibenzylideneacetone)palladium(0), tris(dibenzylideneacetone)palladium(0), dichlorobis(benzonitrile)palladium(II), carbonyltris(triphenylphosphine)palladium(0), trans-dimethylbis(triphenylphosphine)palladium(II), trans-(4-tert-butylcyclohexen-1-yl)chlorobis(triphenylphosphine)palladium(II) and the like, of which bis(dibenzylideneacetone)palladium(0) is preferred. In some cases, the reaction may be accelerated by the addition of a palladium ligand, and examples of the ligand include triphenylphosphine, tri(2-furyl)phosphine, tri(2-thienyl)phosphine and the like, of which tri(2-furyl)phosphine is preferred.

The solvent to be used in this reaction is an inert solvent which does not exert bad influence upon the reaction, such as tetrahydrofuran, dioxane, acetonitrile, 1,2-dimethoxyethane, diglyme, N,N-dimethylformamide, dimethyl sulfoxide, sulforane, N-methylpyrrolidinone, hexamethylphosphoramide, 1,3-dimethyl-3,4,5,6-tetrahydro-2(1H)-pyrimidinone and the like, of which tetrahydrofuran, dioxane, acetonitrile, N,N-dimethylformamide and the like are preferred, or in a mixture of such solvents.

The reaction temperature is in the range of from room temperature to reflux temperature, and the reaction time, though varies depending on the reaction conditions, is within the range of from several hours to several tens of hours.

In the second step, the compound represented by the general formula (XXXII) produced in the first step is allowed to react with a reaction equivalent amount of a phosphoric acid esterification agent to obtain the compound represented by the general formula (XXXIII). That is, this is a step in which the reaction with a phosphoric acid esterification agent (reactive derivative of phosphoric acid) is carried out in an inert solvent in the presence of an acid scavenger (dimethylaminopyridine or the like) in order to obtain a compound having a 4-phosphoryloxy-2-buten-1-yl group introduced into the 3-position of the cephalosporin nucleus.

As the reaction solvent to be used in this case, an aromatic hydrocarbon such as benzene, toluene, xylene or the like is desirable, and diethyl ether, dioxane, tetrahydrofuran, cyclohexane, chloroform, dichloromethane, acetonitrile, dimethyl sulfoxide, N,N-dimethylformamide, hexamethylphosphoramide, ethanol or the like may be used.

In the third step, the compound represented by the general formula (XXXIII) produced in the second step, preferably having E form (trans form) at the 3-position thereof, is allowed to react after its halogenation or directly with a base, preferably an aliphatic tertiary amine or the like, to obtain the compound represented by the general formula (XXXIV). That is, this is a step in which a 4-halogeno-2-buten-1-yl-cephalosporin compound obtained by allowing the compound represented by the general formula (XXXIII) to react with a halogen donor in an inert solvent with cooling or at room temperature or the compound represented by the general formula (XXXIII) itself is allowed to react with a reaction equivalent or excess amount of an aliphatic tertiary amine or the like in an inert solvent to obtain the 1,3-butadien-1-yl-cephalosporin compound (XXXIV).

Examples of the base include aromatic or aliphatic amines, but preferably aliphatic tertiary amines such as diisopropylethylamine, dipropylethylamine, diisopropylmethylamine, diethylmethylamine, dipropylmethylamine and the like.

Illustrative examples of the halogen donor include halides such as those with alkali metals, alkaline earth metals and the like, halogenated silyl compounds such as trialkylsilyl halide and the like and halogenated tin compounds such as trialkyltin halide, dialkyltin dihalide, monoalkyltin trihalide and the like.

The inert solvent to be used in this step can be selected from those described in the aforementioned second step.

Elimination of a protective group from the compound represented by the general formula (XXXIV) can easily be effected by, for example, treating the compound with water when the protective group is a tri-lower alkylsilyl group or the like. When the protective group is benzhydryl, p-methoxybenzyl, trityl, tert-butyl, formyl or the like group, it can be eliminated easily by treating it with an acid such as formic acid, trifluoroacetic acid, a trifluoroacetic acid-anisole mixture, a hydrobromic acid-acetic acid mixture, a hydrochloric acid-dioxane mixture or the like. Also, in the case of a protective group for the hydroxyl group, such as a lower acyl group or the like which can be eliminated easily under mild conditions, it can be eliminated by solvolysis using an aqueous solvent containing a base such as sodium bicarbonate, sodium carbonate, ammonium hydroxide, ammonium carbonate, ammonium carbamate or the like.

### Production process 10

This process is used for the production of a member of the compound (A) of the present invention in which the 7-position is an amino group. 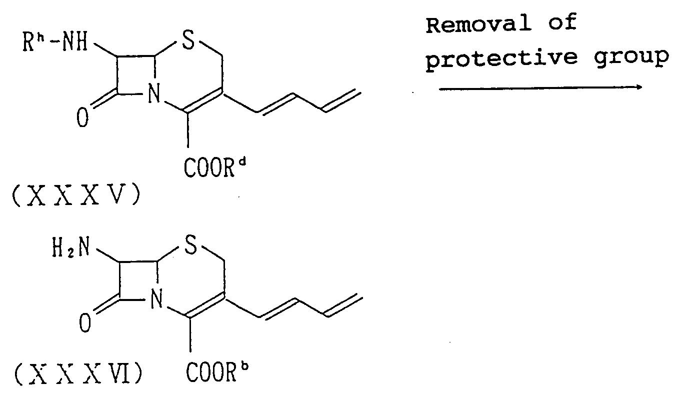
(R^{h}: a protective group for the phenylacetyl group and amino group in the meaning of R^{a})
This process is used for the production of a cephalosporin derivative represented by the general formula (XXXVI) which is a member of the compound (A) of the present invention. That is, this is a process in which the compound represented by the formula (XXXVI) is obtained by allowing phosphorus pentachloride to react with the compound represented by the general formula (XXXV) to effect formation of an iminochloro compound, adding a lower alcohol to the compound to effect iminoetherification and then adding water to effect hydrolysis.

Isolation and purification of the compound of interest from the reaction solution may be carried out in the usual way by optionally combining organic solvent extraction, chromatography, crystallization and the like techniques. When the compound (A) has an ester residue, it can be obtained in the usual way by the reaction of an esterification agent such as an alcohol or a halide thereof, a sulfonate, a sulfate, a diazo compound or the like, and a salt of the compound (A) can be obtained by applying usual salt formation reaction.

### Production process 11

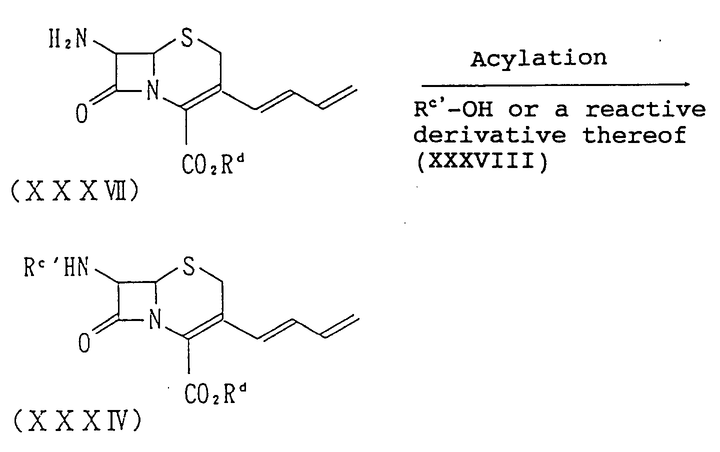

In this process, the compound (XXXIV) of the present invention is produced by allowing an amine compound represented by the general formula (XXXVII) to undergo acylation reaction with a carbonic acid represented by the general formula (XXXVIII) or a reactive derivative thereof.

The acylation reaction of the compound (XXXVII) with the compound (XXXVIII) or a reactive derivative thereof is generally carried out in a solvent with cooling or at room temperature. The solvent is not particularly limited, provided that it does not take part in the reaction. Examples of commonly used solvents include dioxane, ethyl ether, tetrahydrofuran, chloroform, dichloroethane, methylene chloride, ethyl acetate, ethyl formate, dimethylformamide, dimethyl sulfoxide, water and the like. These solvents may optionally be used as a mixture. The acylation reaction is carried out in the same manner as described in the production processes 4 and 8.

### Production process 12

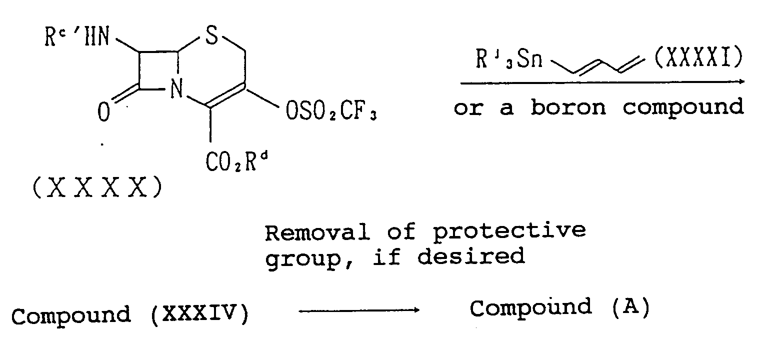

(In the above reaction formula, the R^{j} groups may be the same or different from one another and each represents a lower alkyl group.)

This production process is a reaction to introduce a butadienyl group. Other reactions than the butadienyl group-introducing reaction are omitted because they are the same as the case of the production process 9.

The aforementioned compound (XXXIV) is obtained by allowing a trifluoromethanesulfonyloxy compound (XXXX) to react with a reaction equivalent amount of an organic tin (XXXXI) or boron compound in an inert solvent at room temperature in the presence of a reaction enhancing agent such as a halogeno-zinc, a halogeno-lithium, trifluorophosphine, bis(dibenzylideneacetone)palladium or the like.

Examples of the inert solvent include methylpyrrolidinone, tetrahydrofuran, acetonitrile, dimethylformamide, dimethyl sulfoxide and the like.

The following tables show typical examples of the compounds represented by the general formula (I) and intermediates thereof which are produced by the production processes of the present invention described in the foregoing in detail.

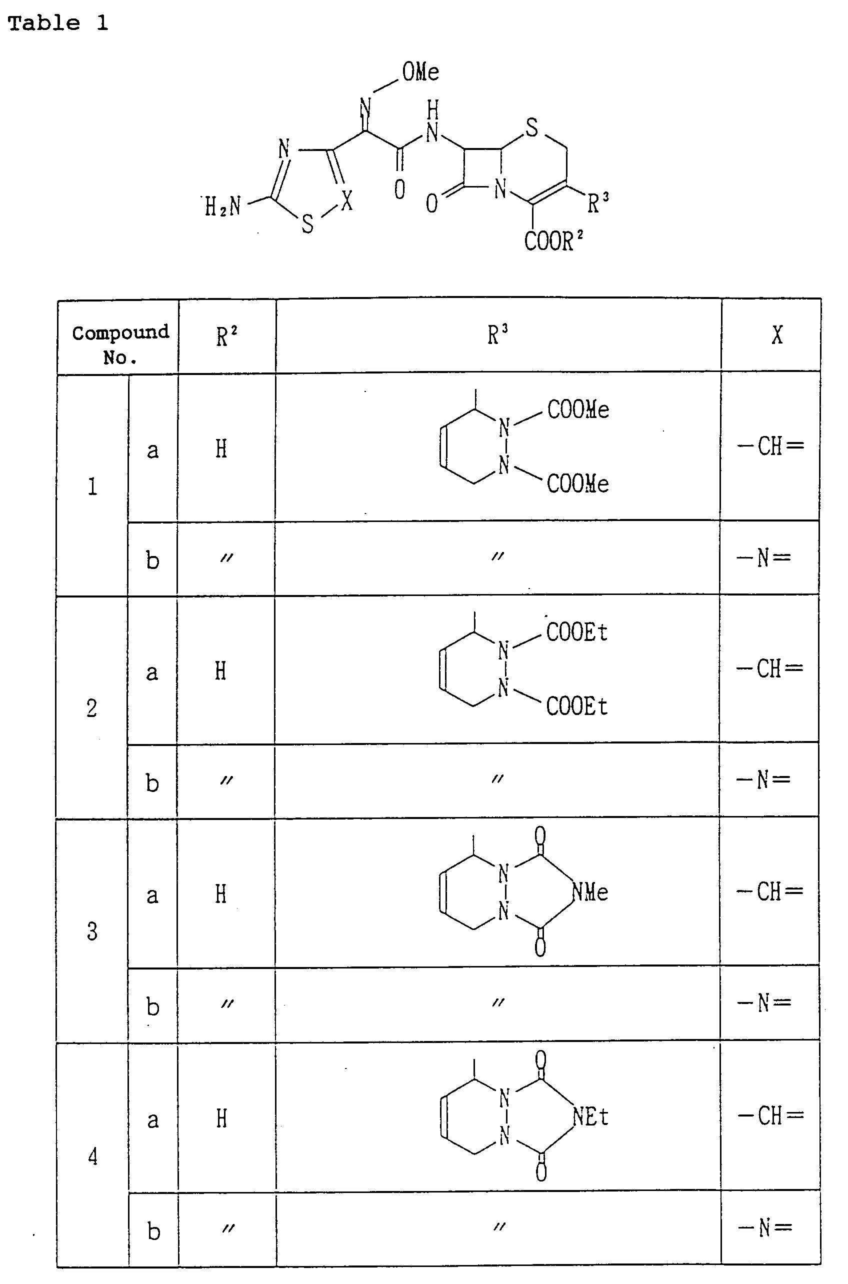

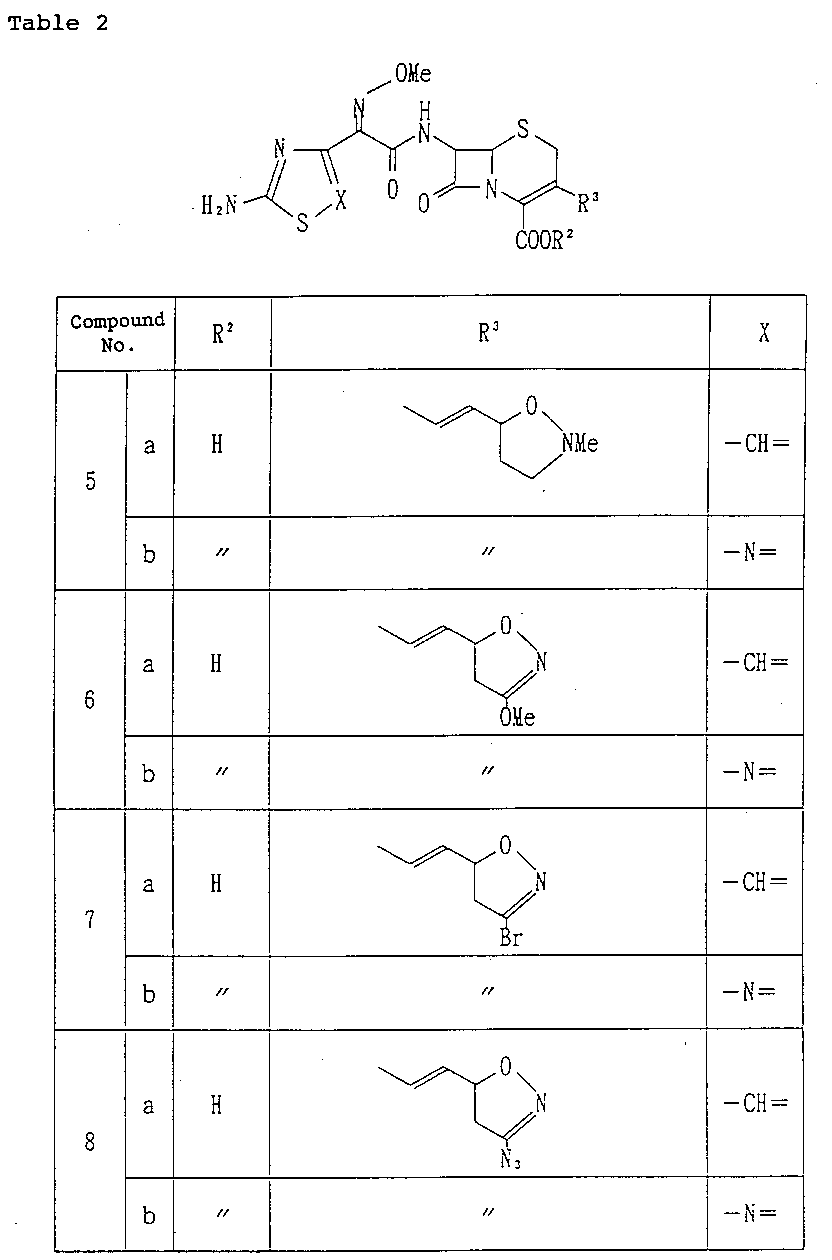

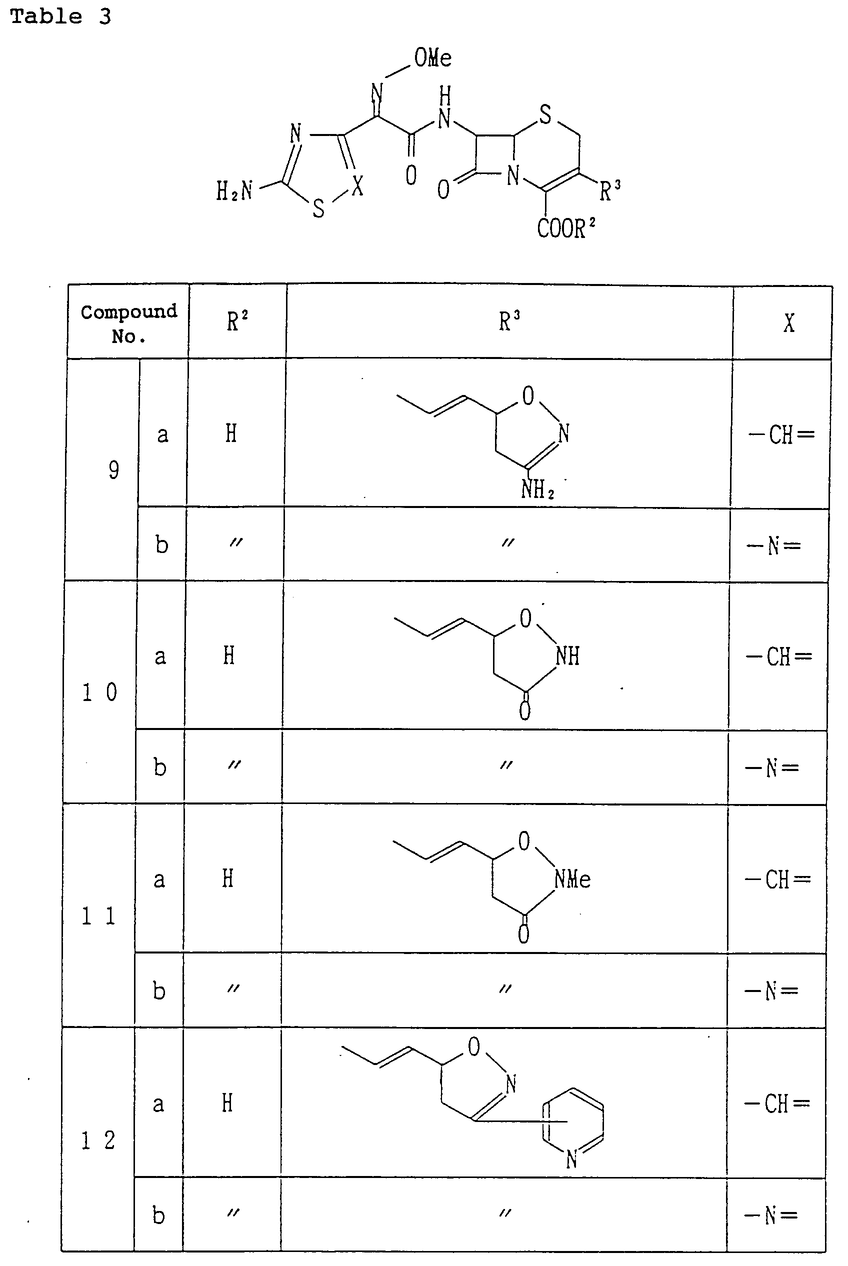

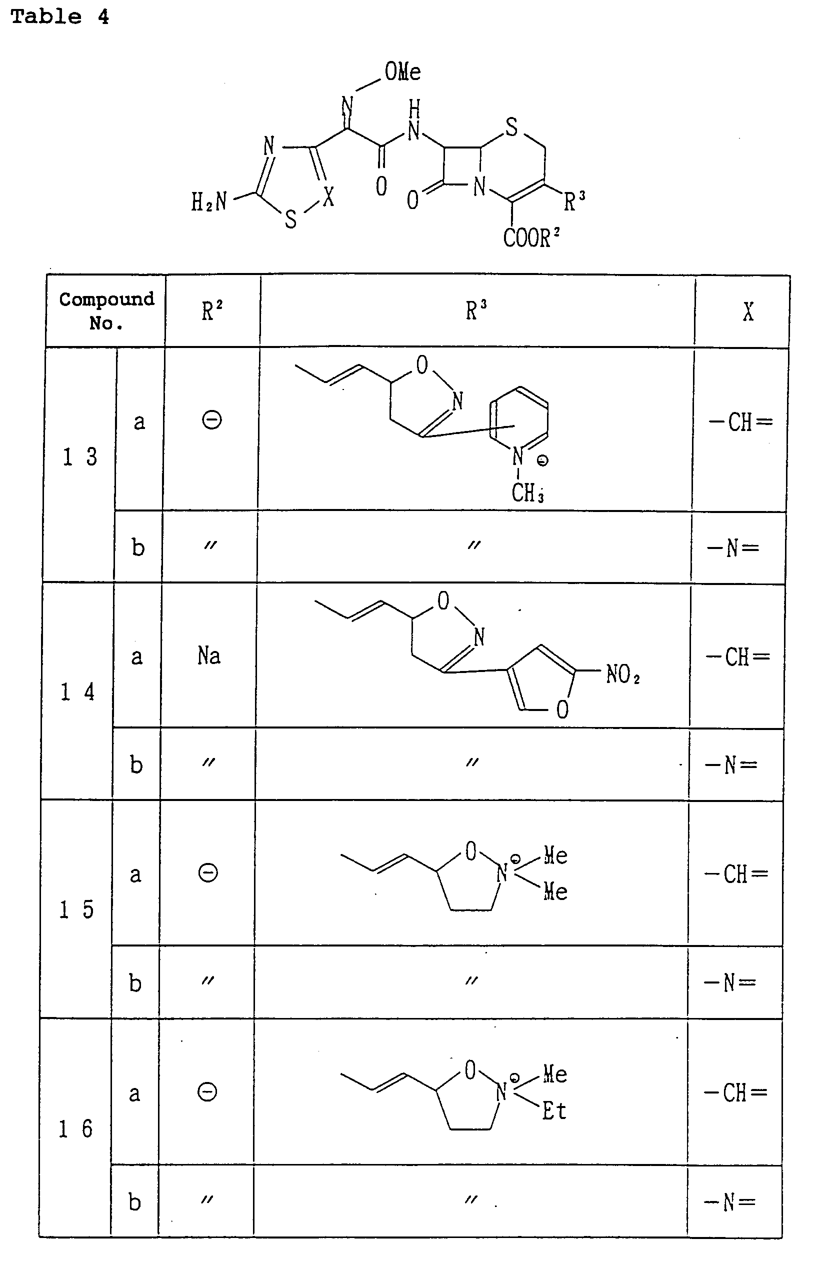

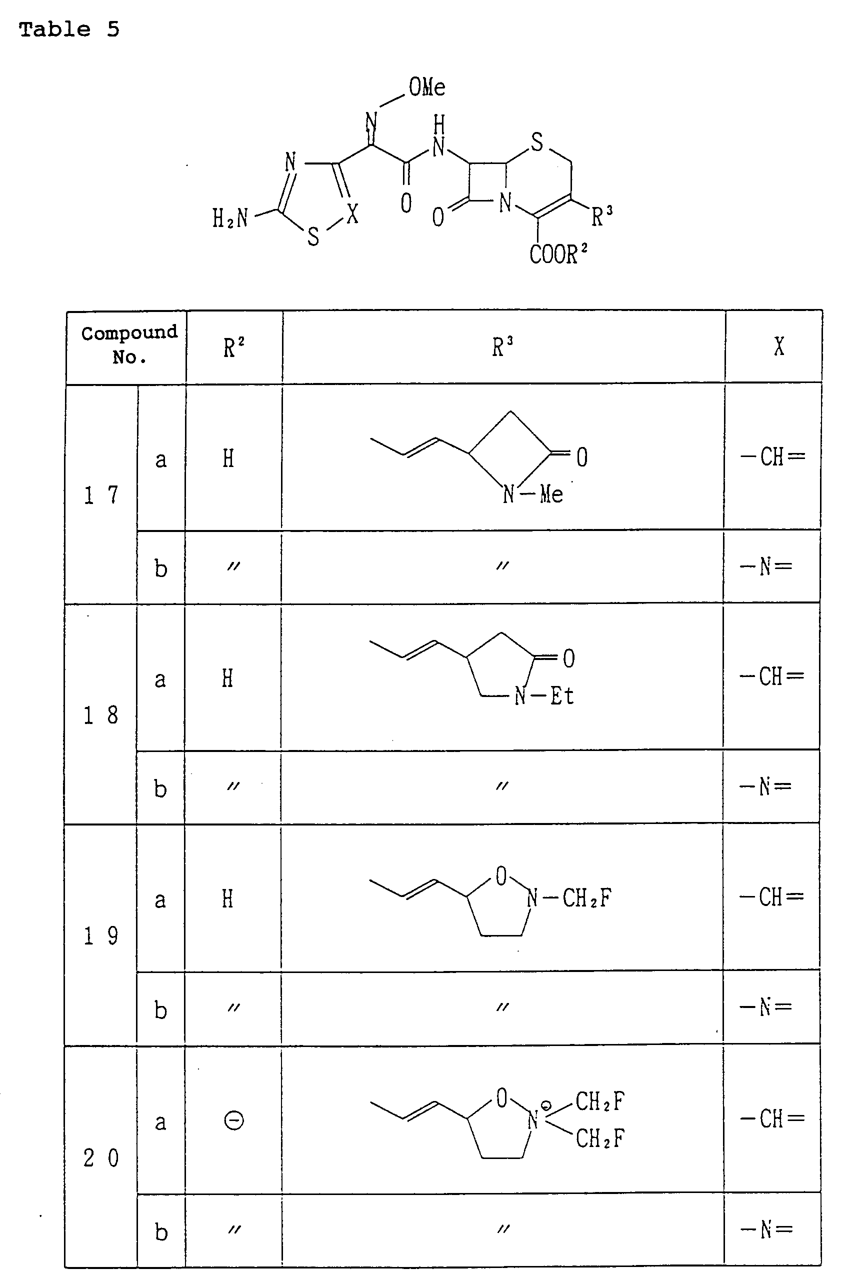

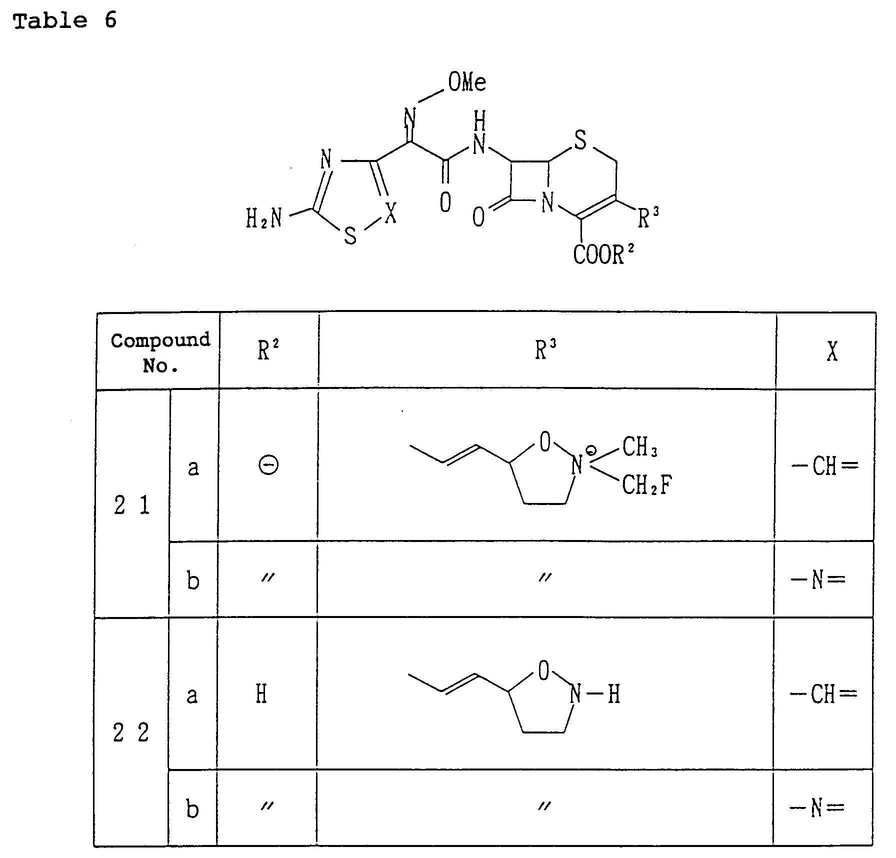

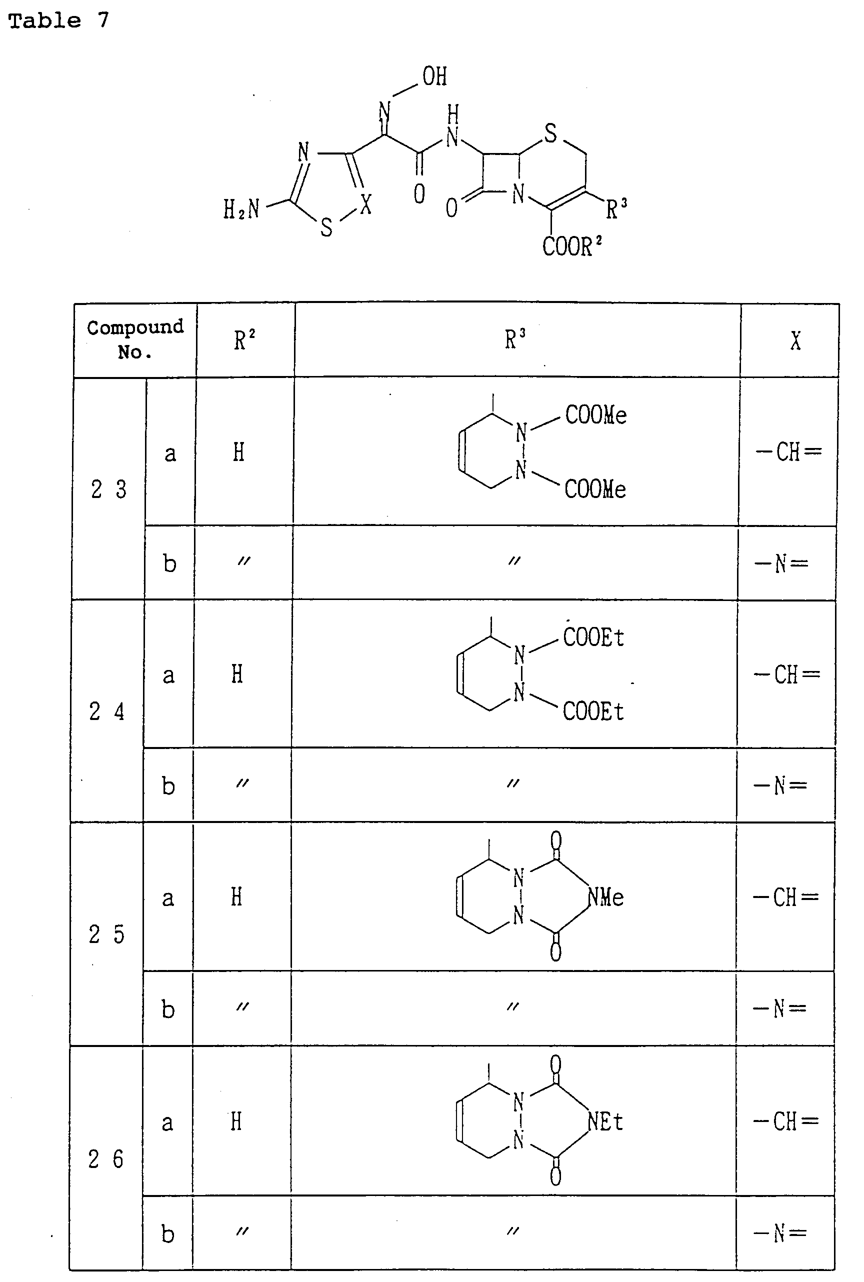

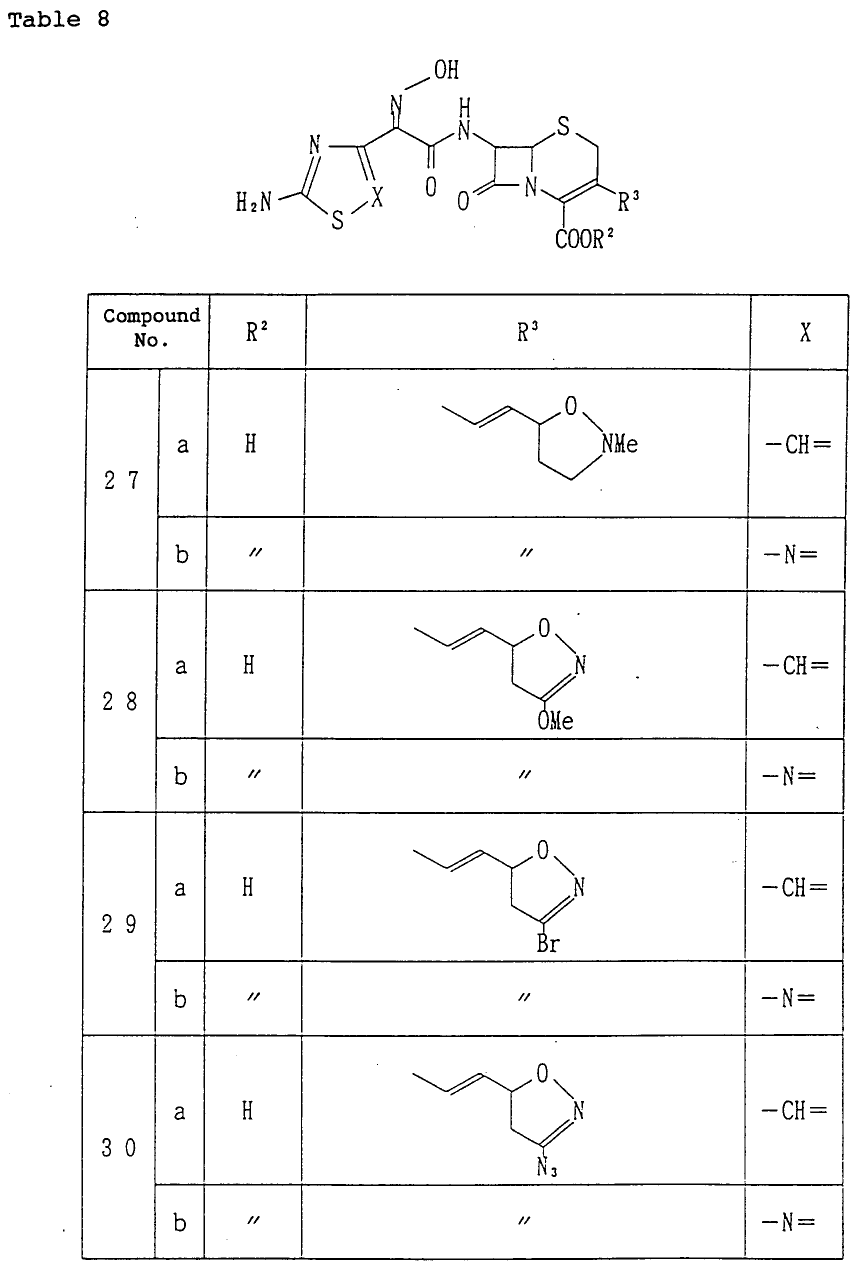

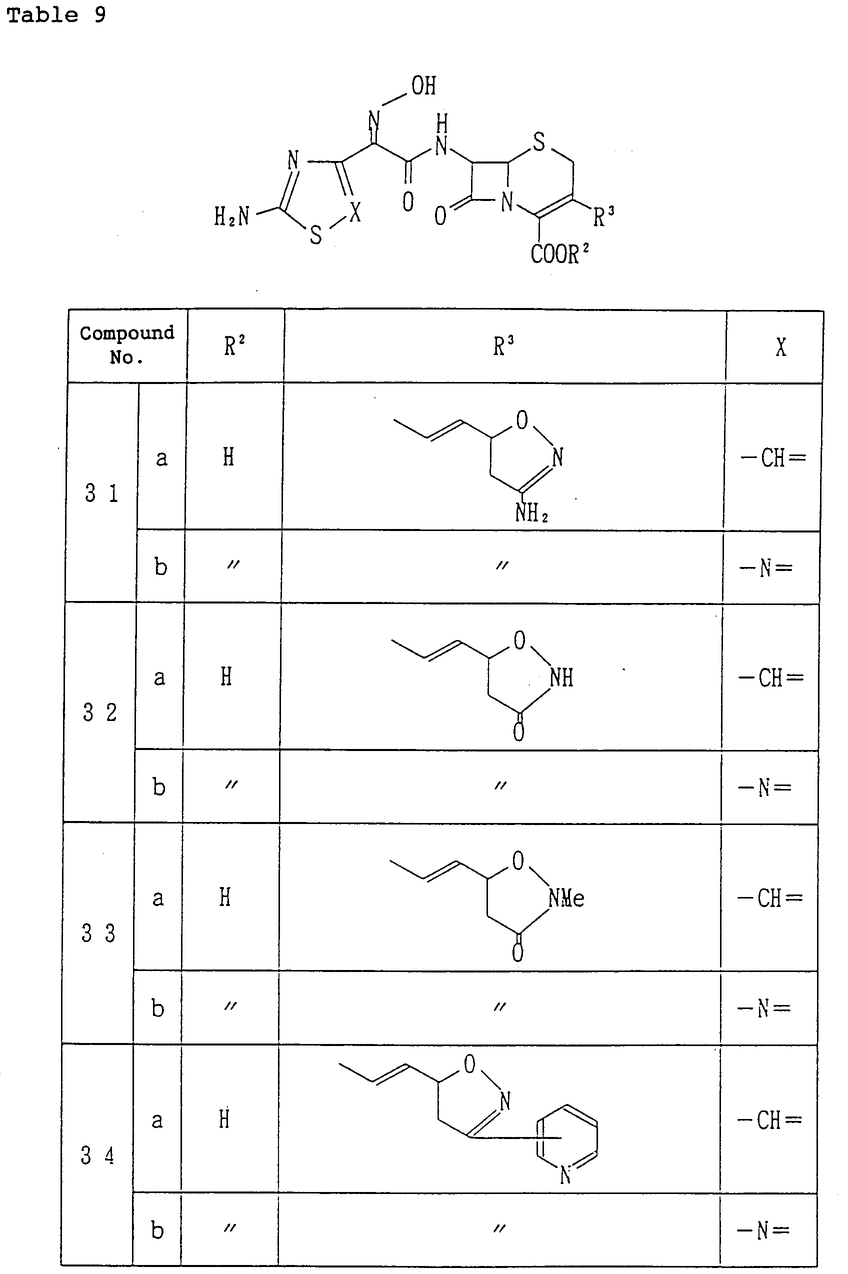

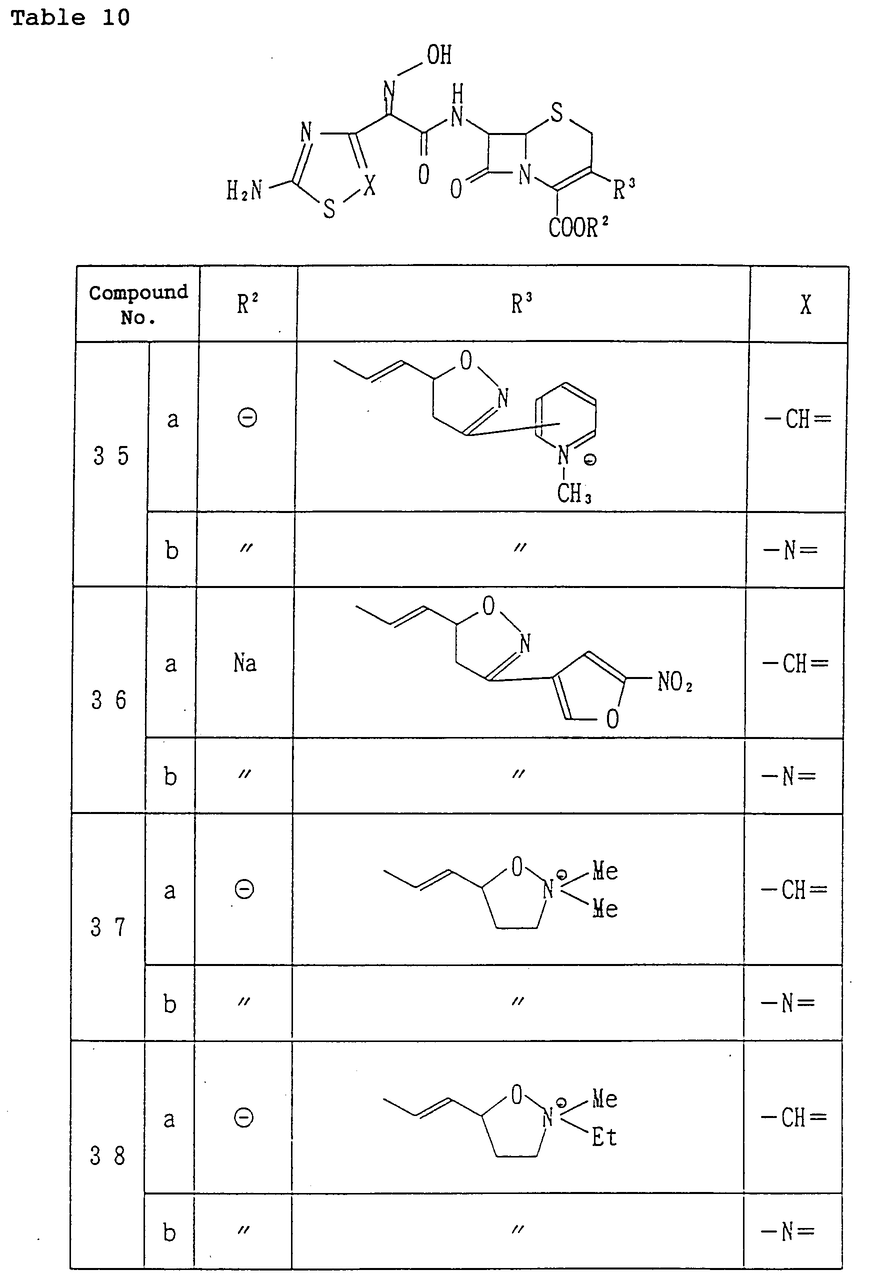

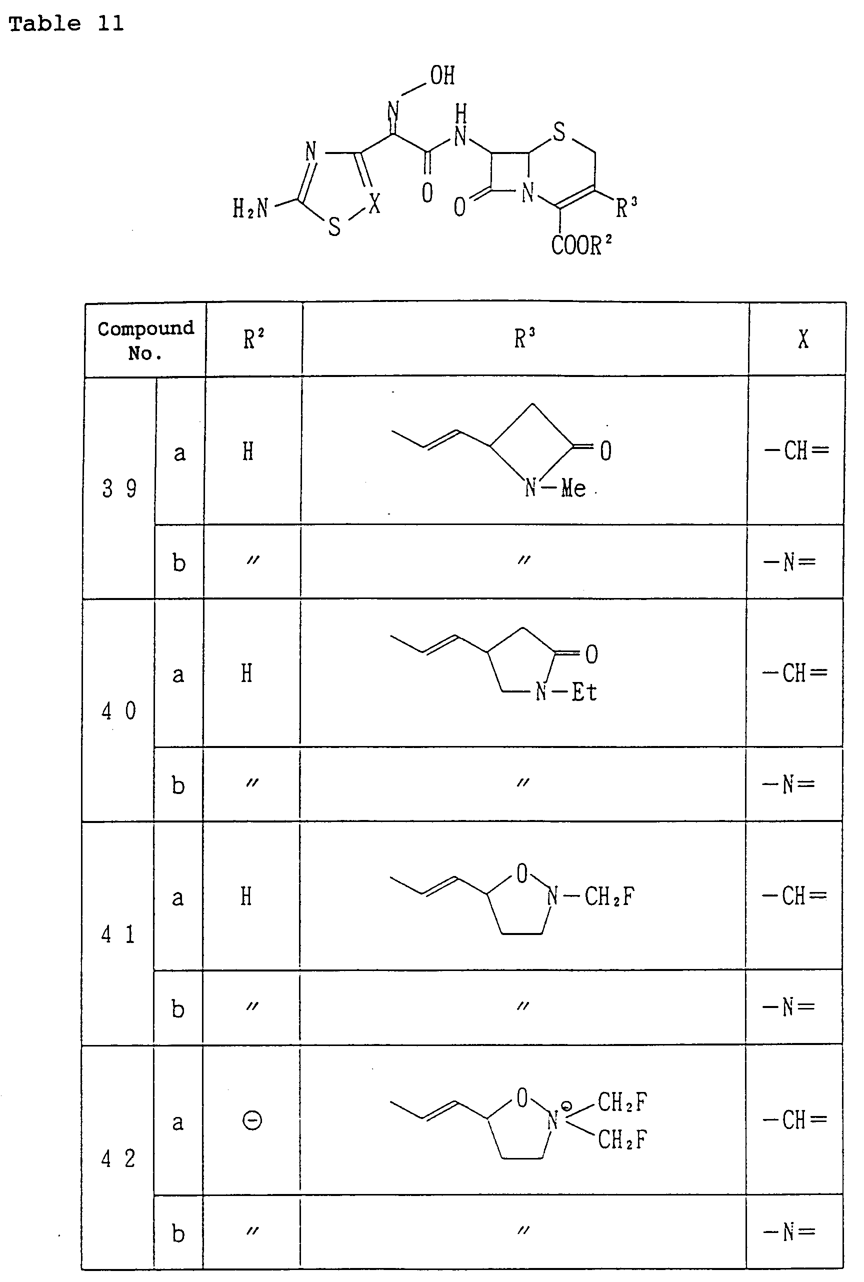

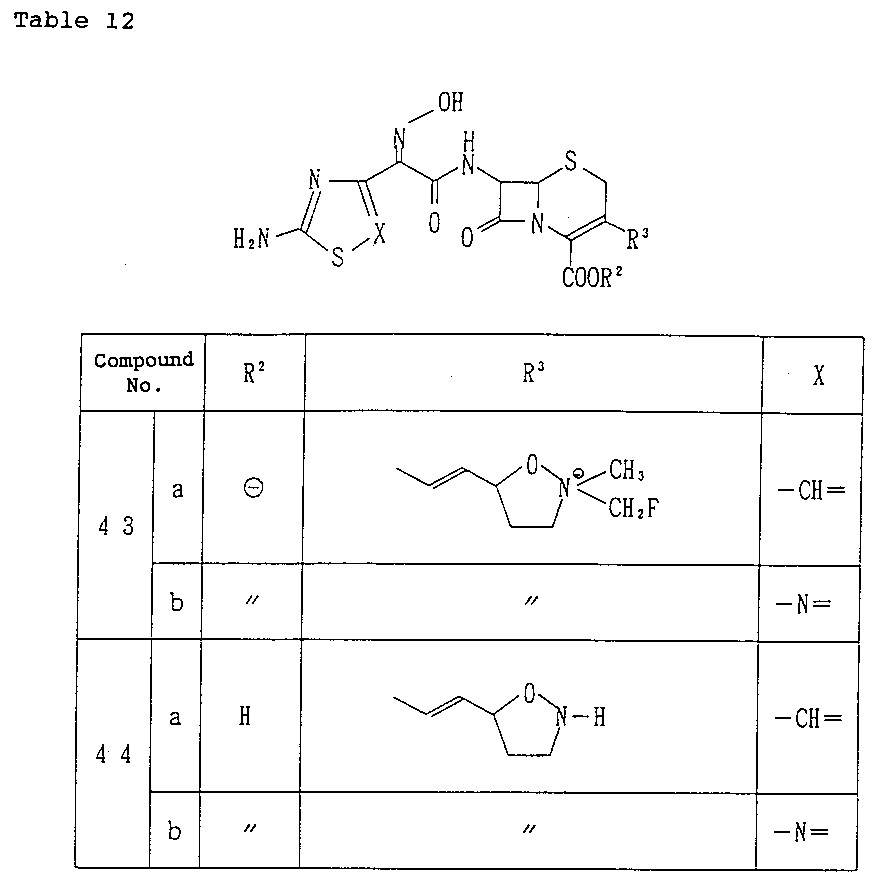

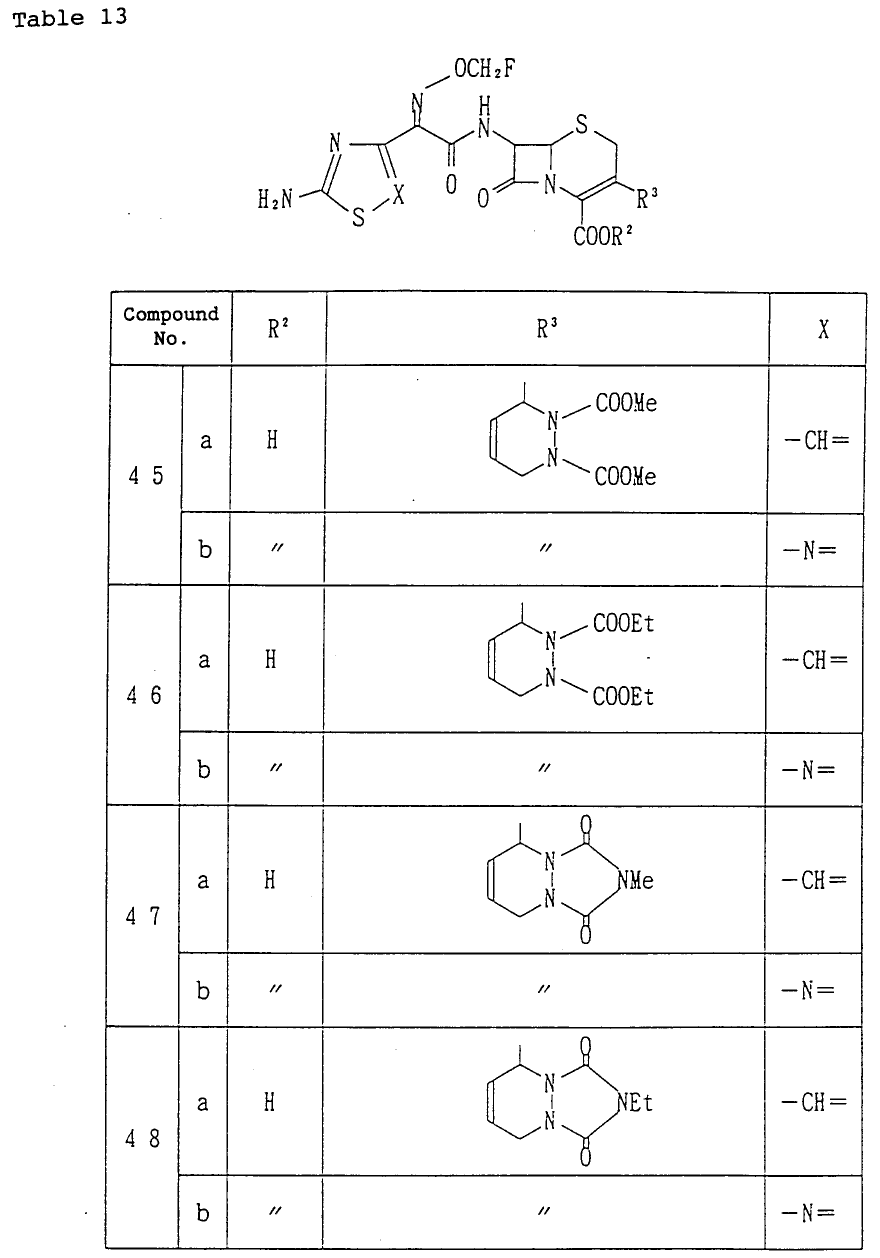

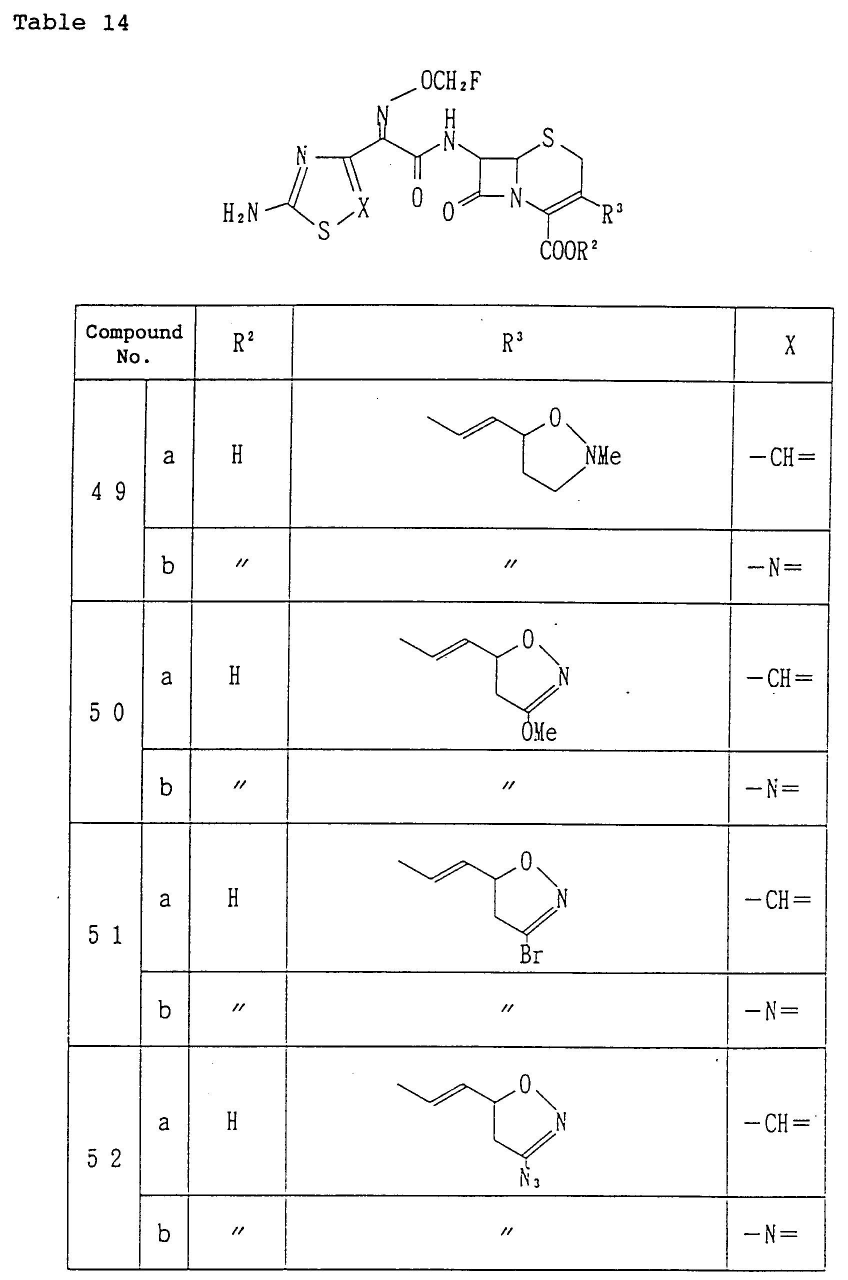

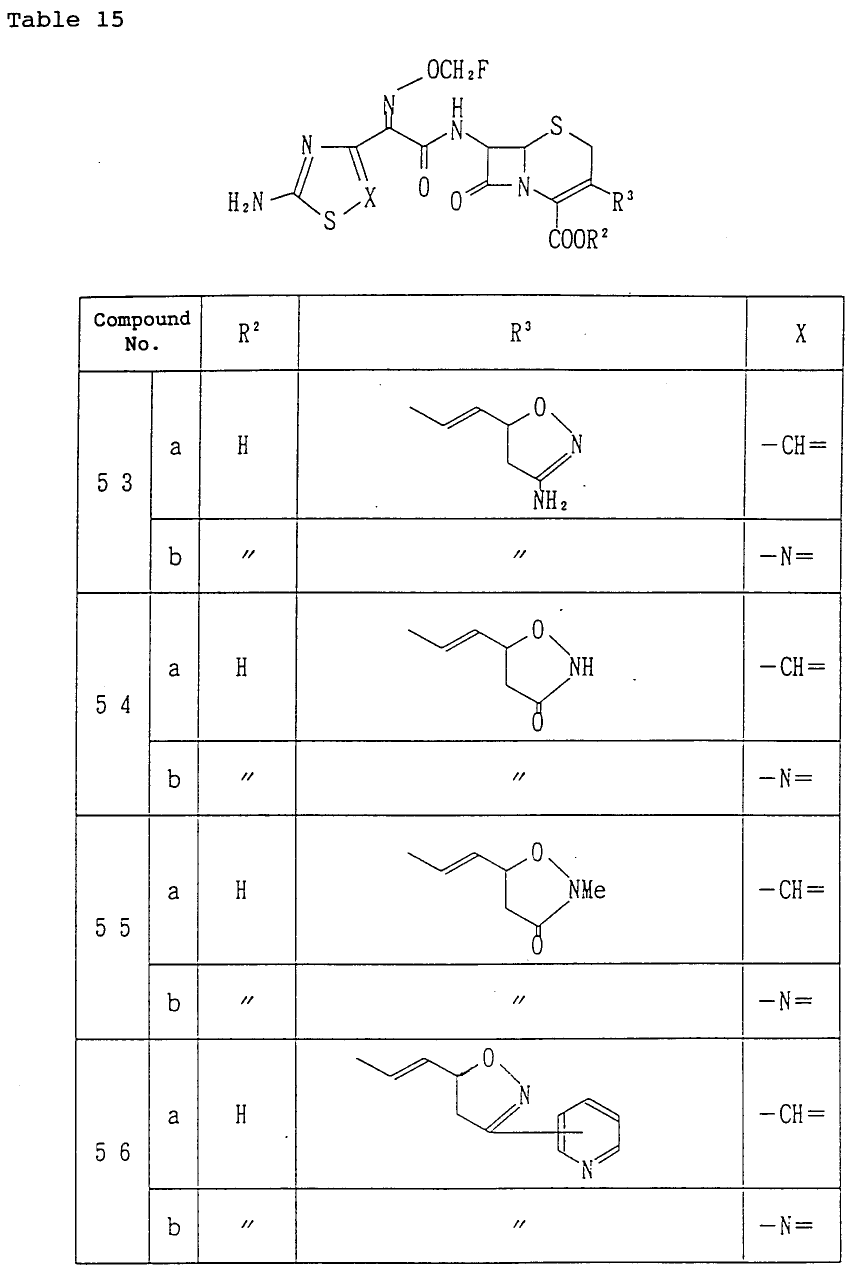

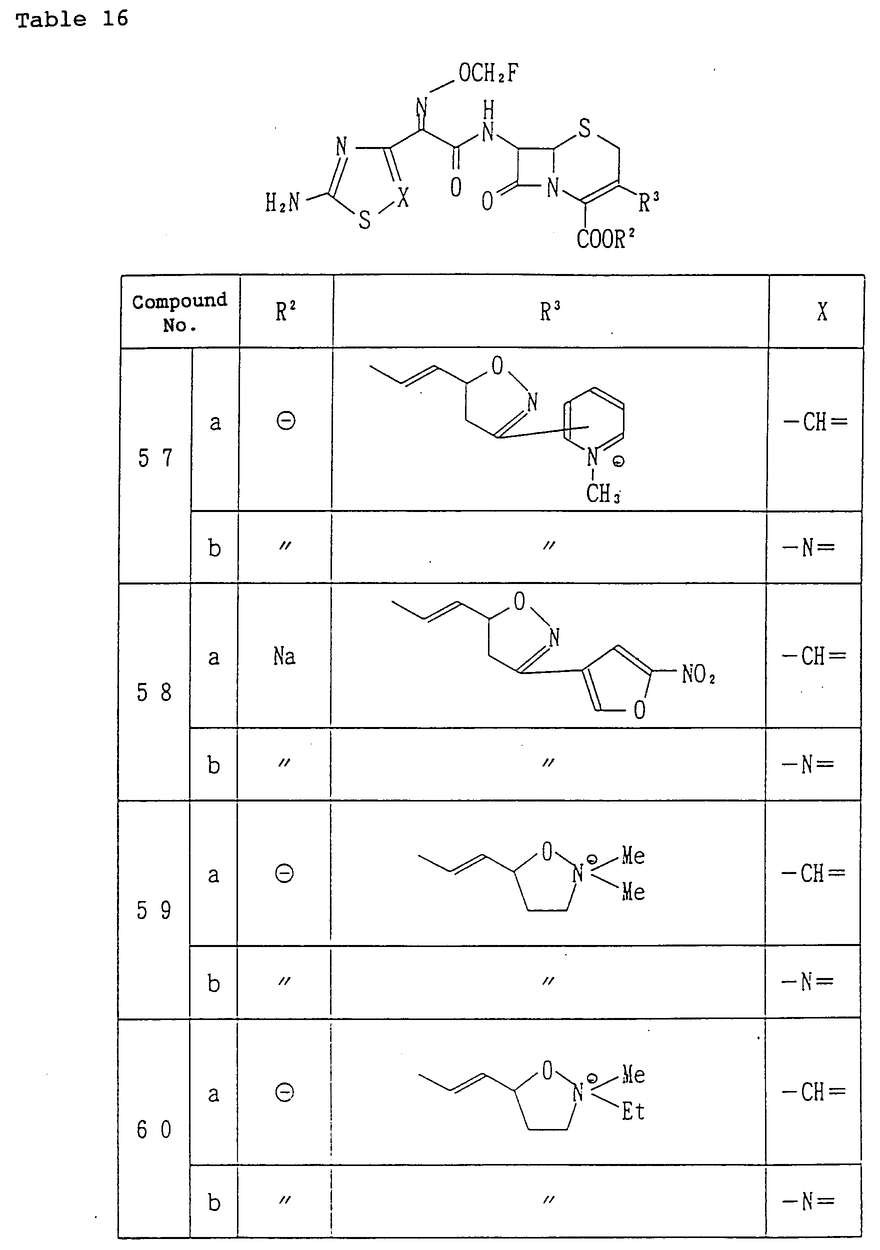

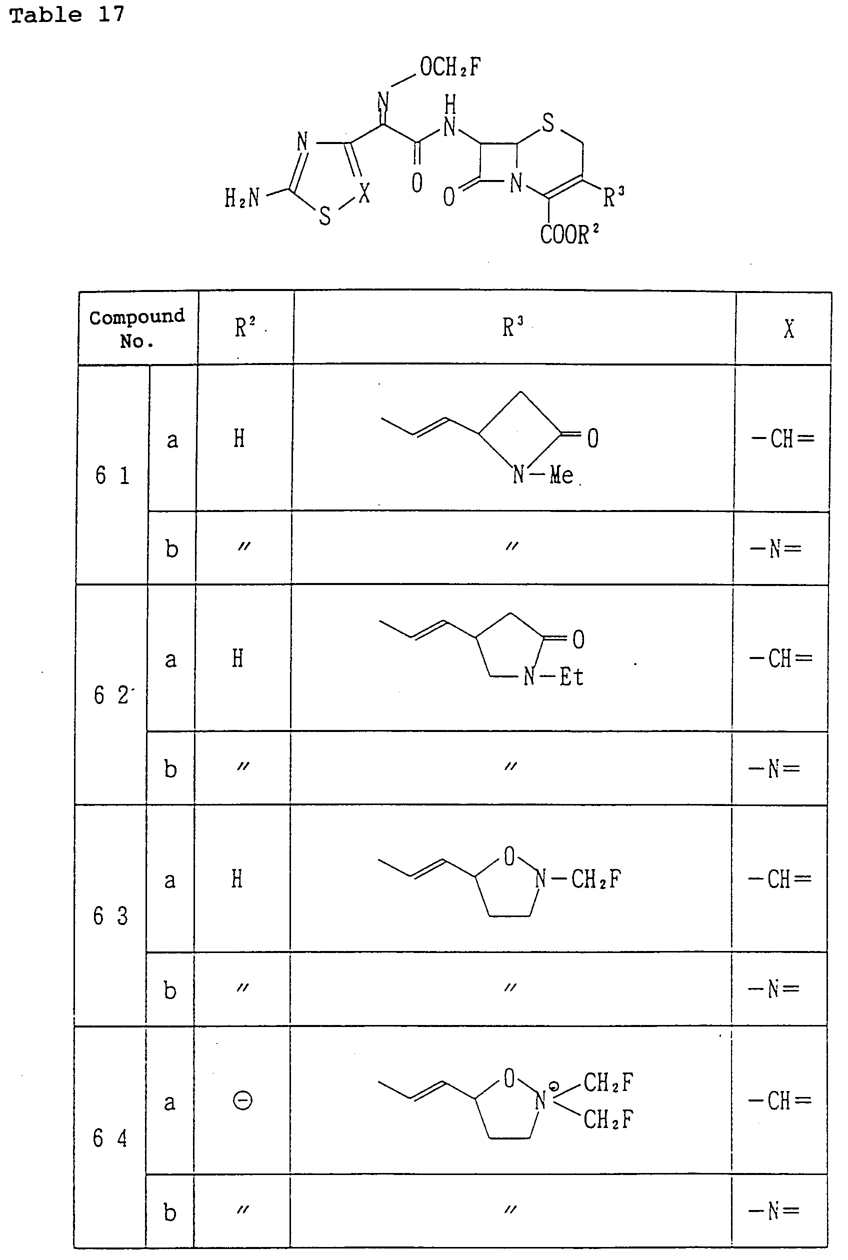

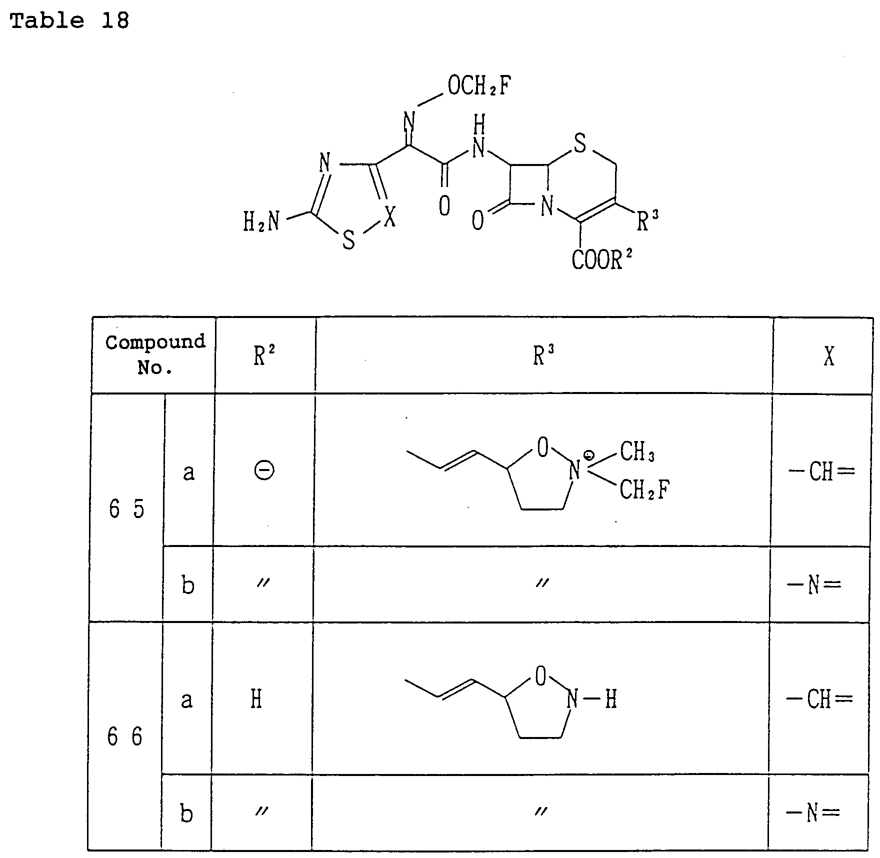

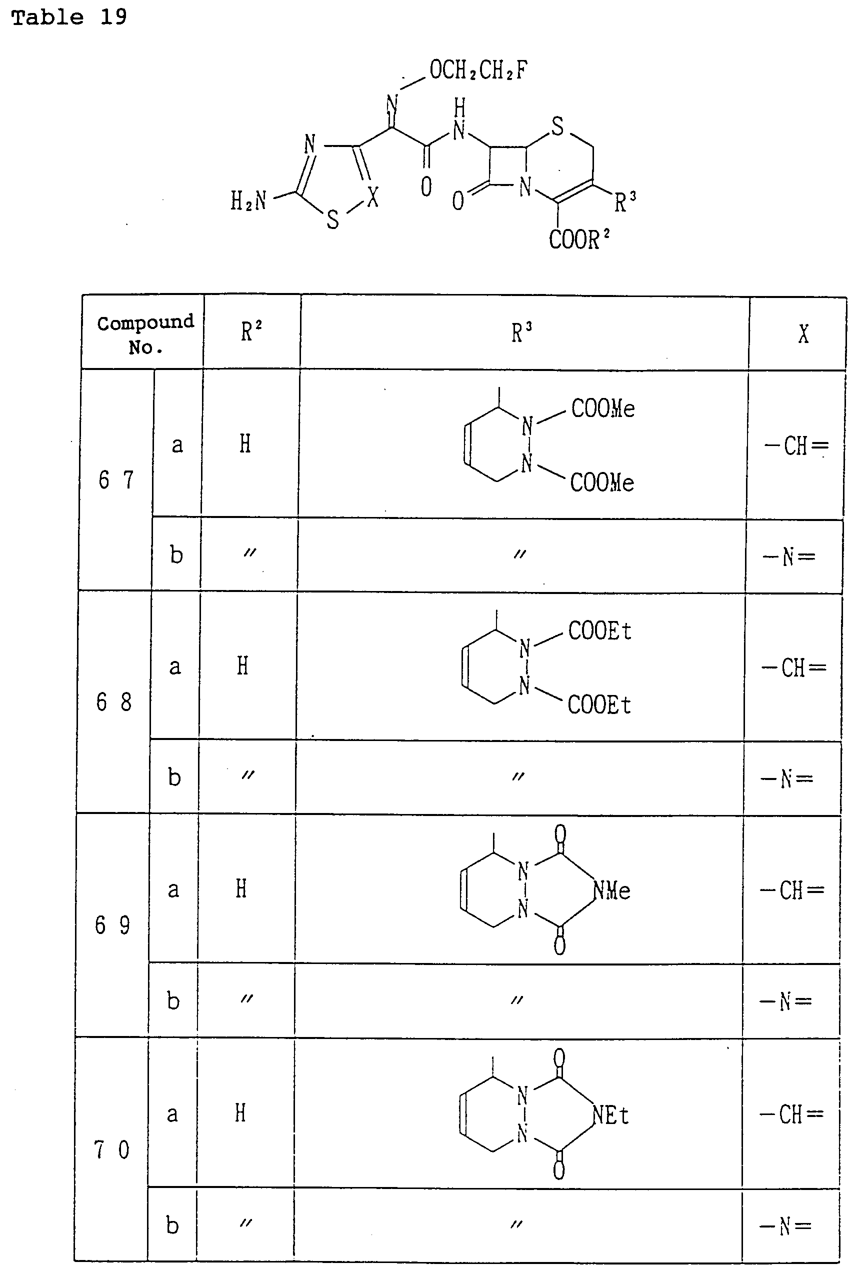

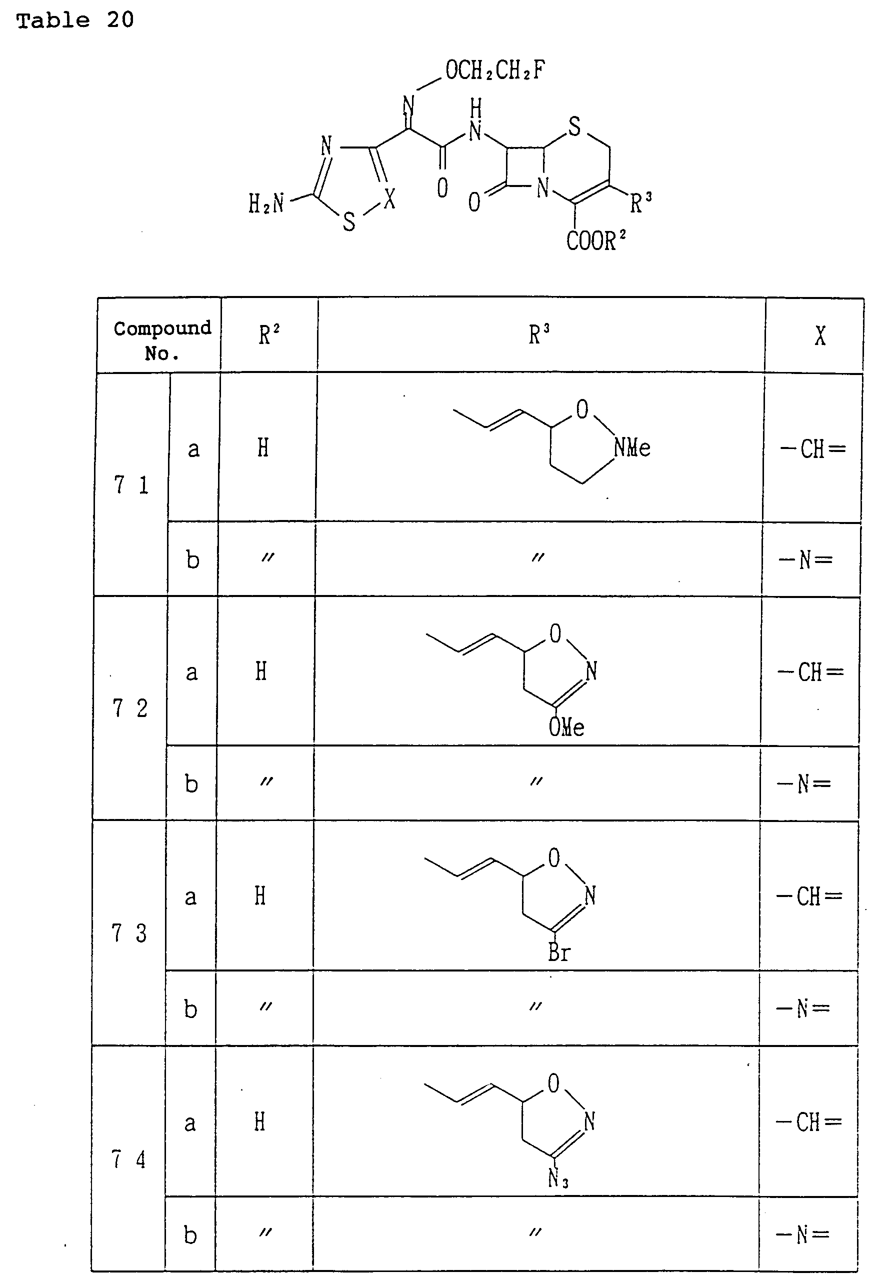

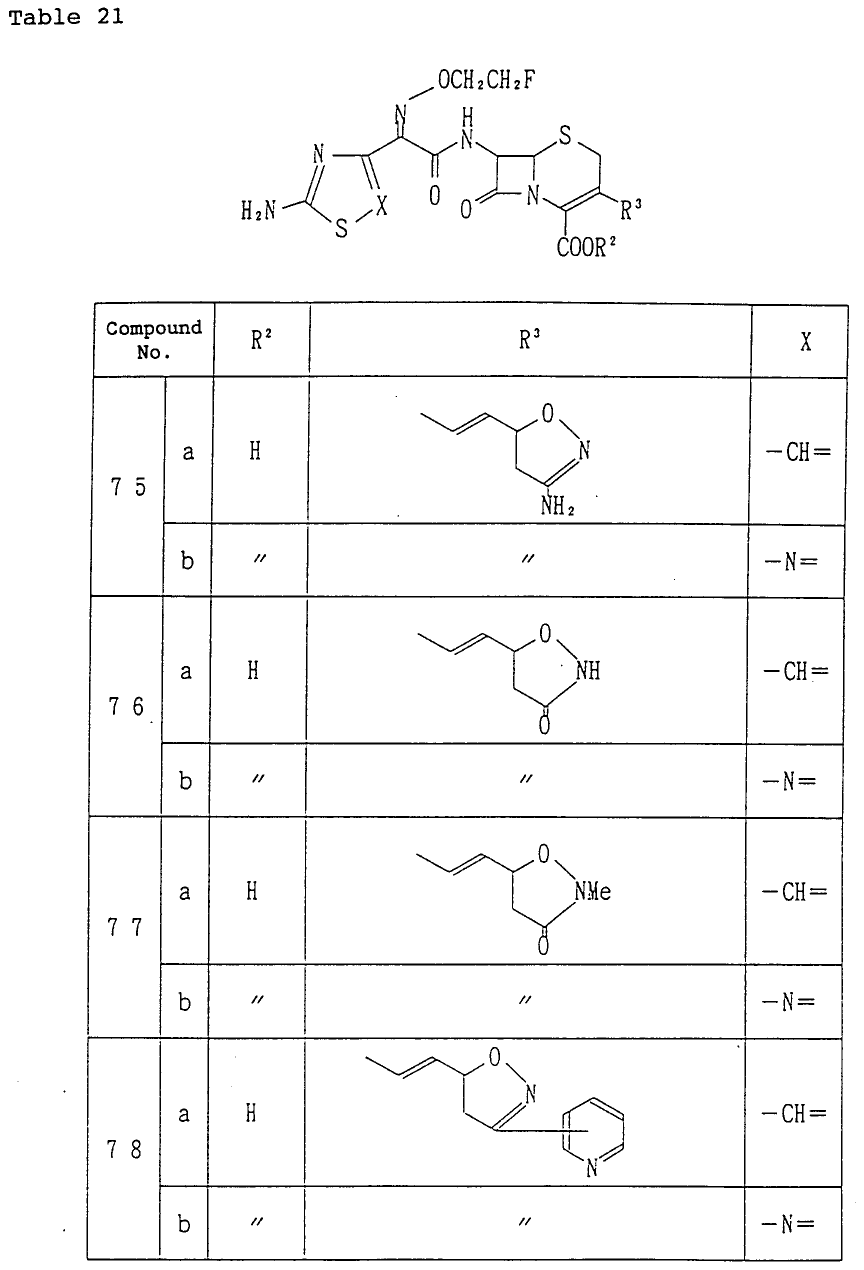

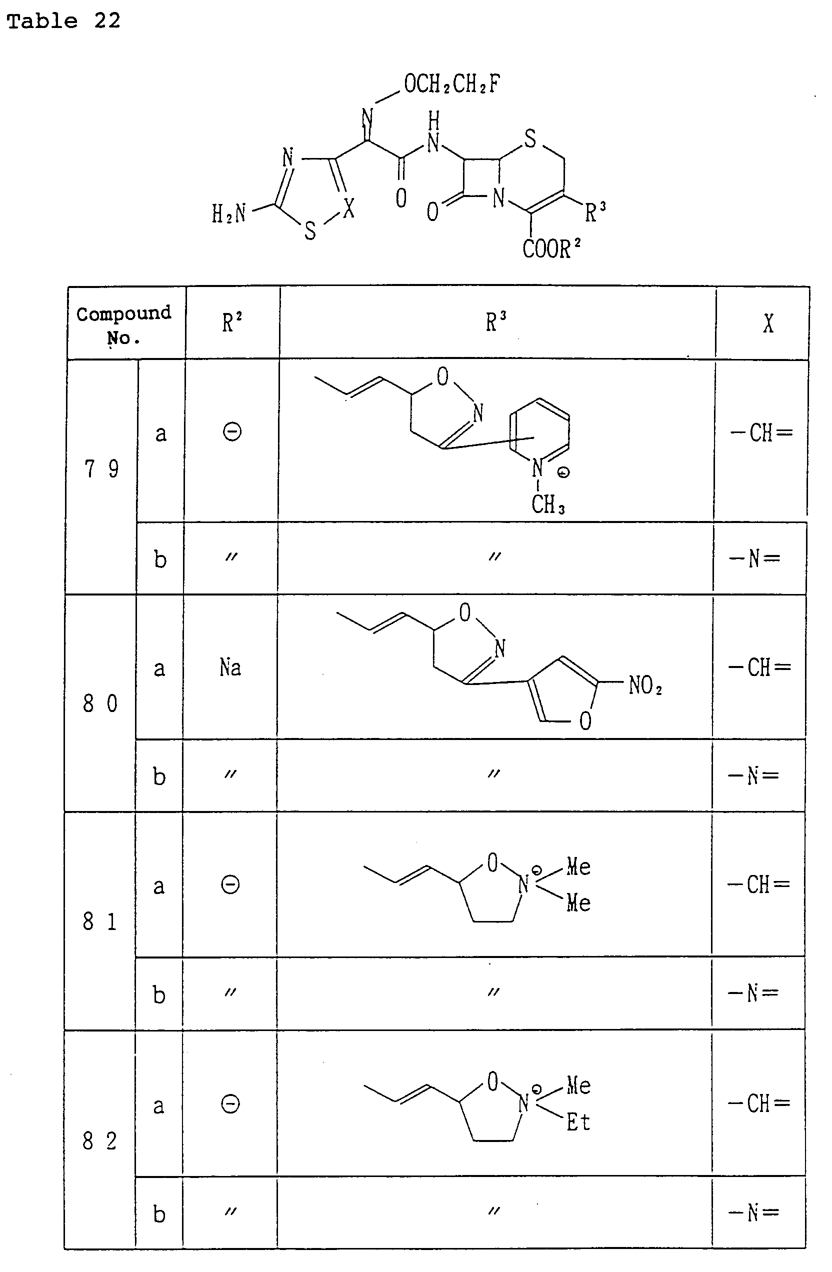

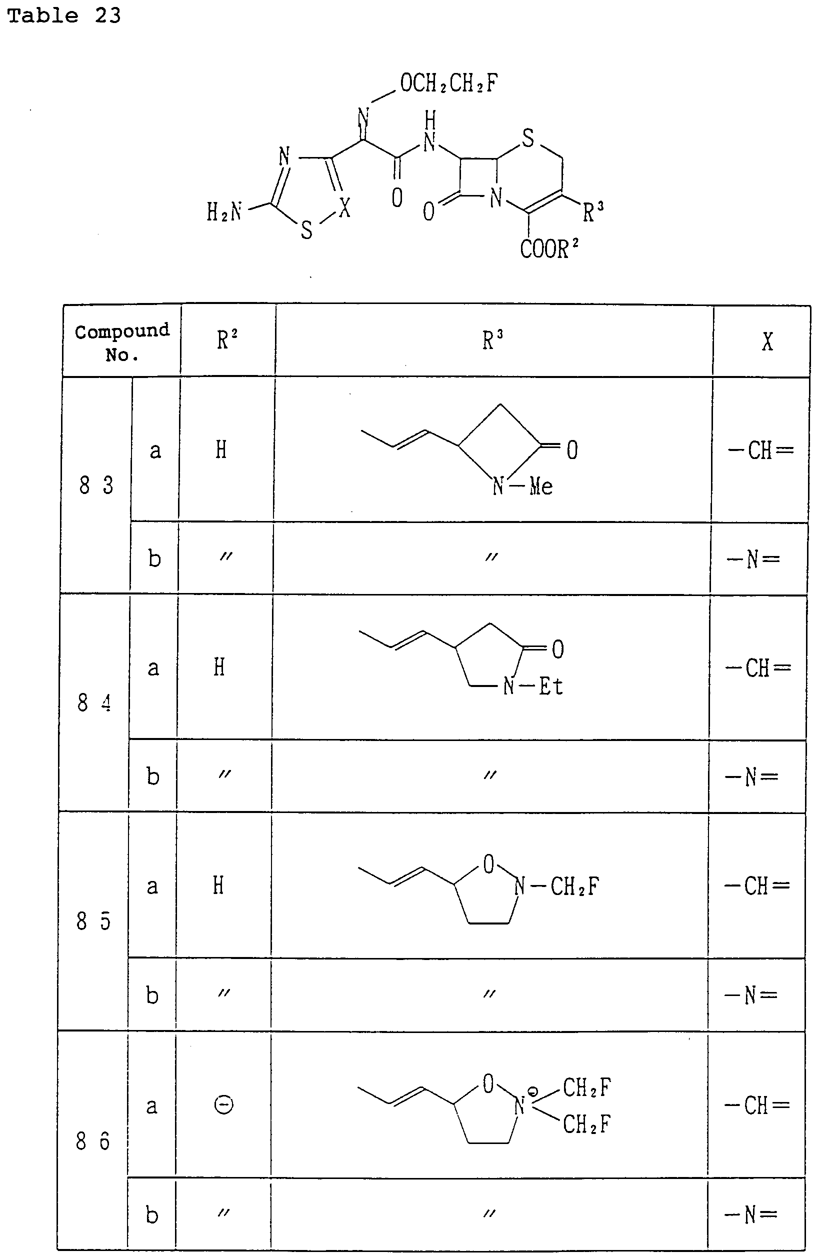

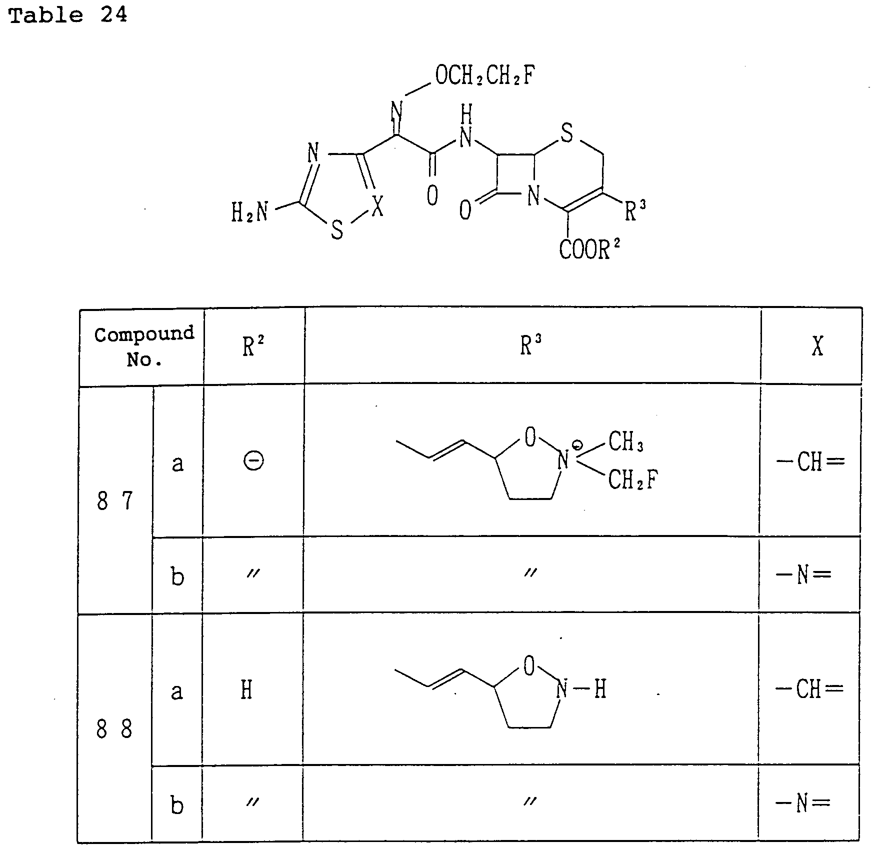

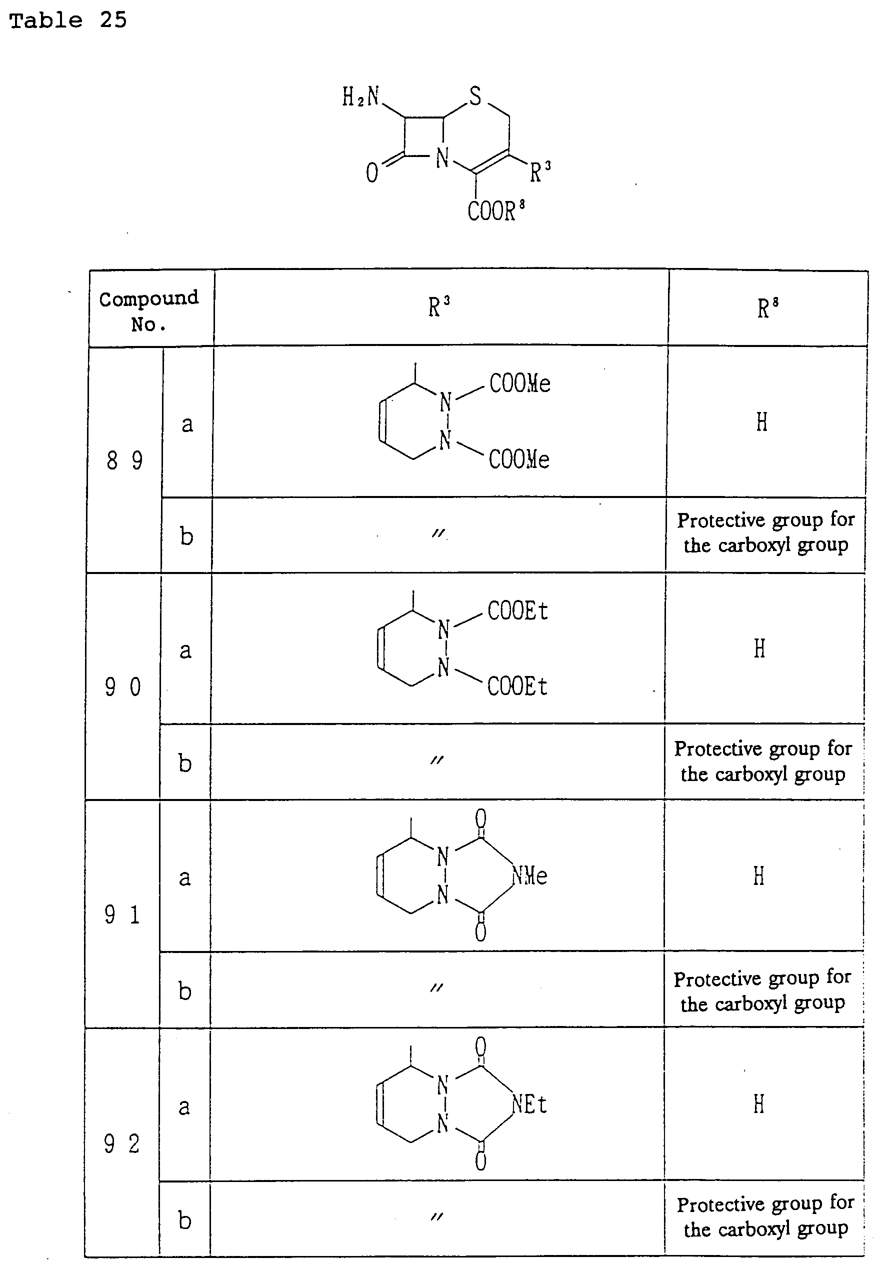

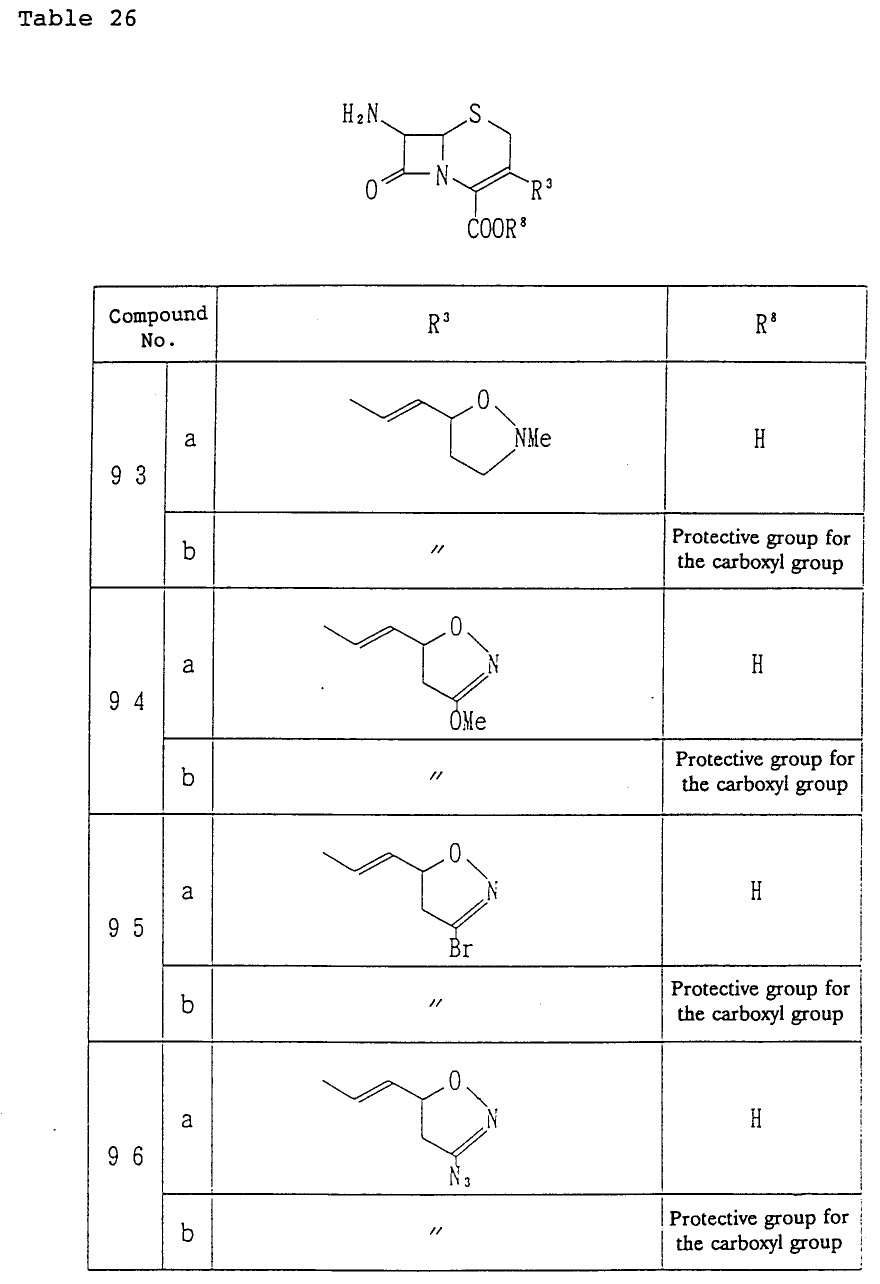

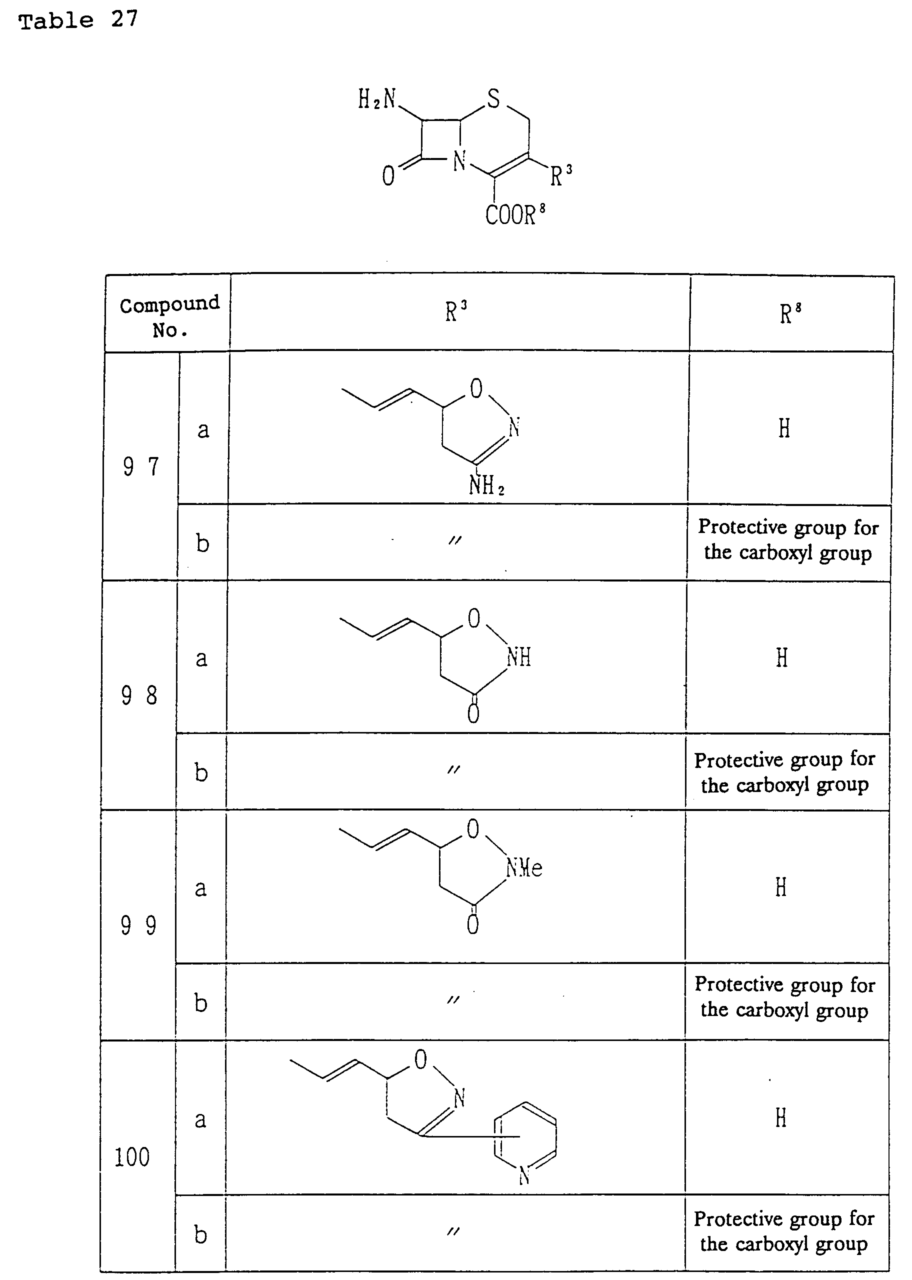

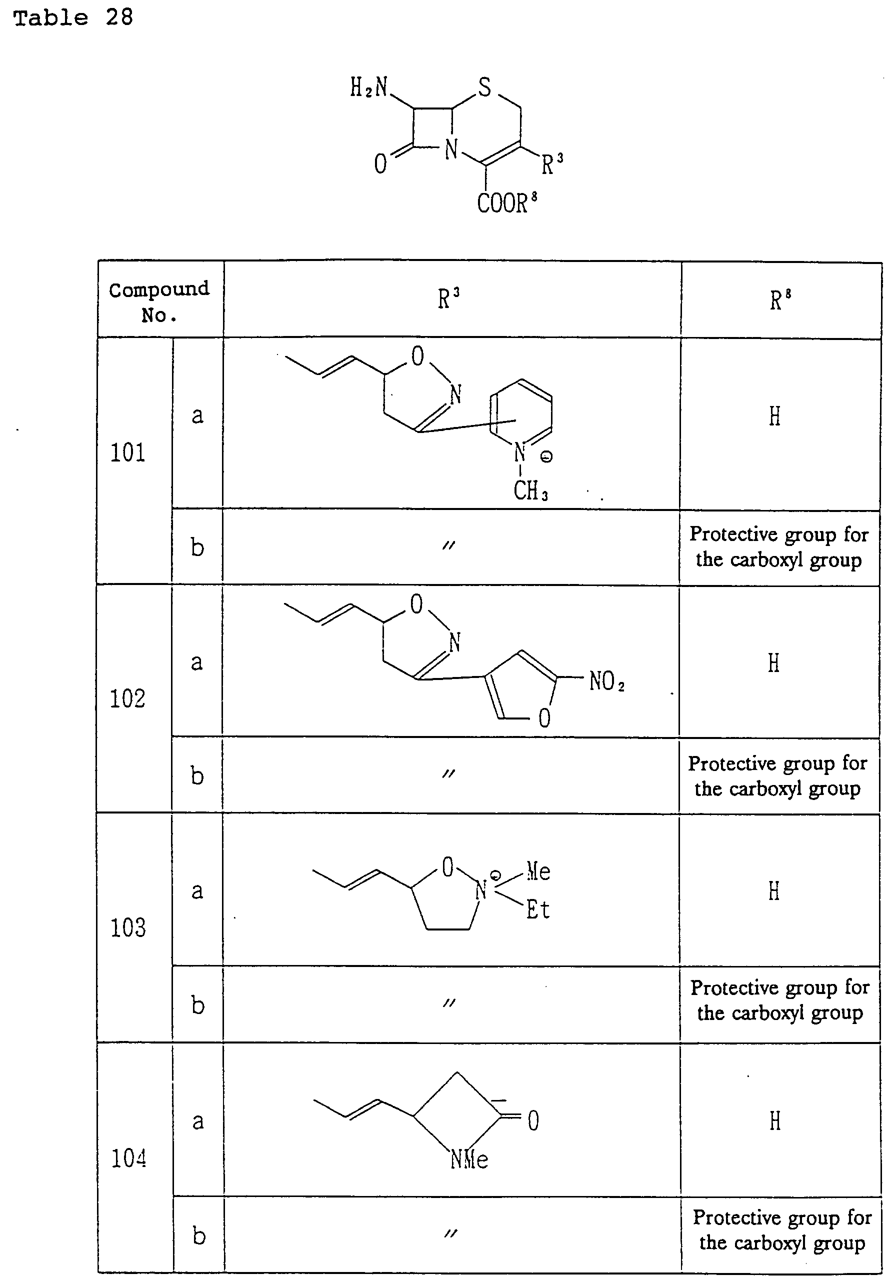

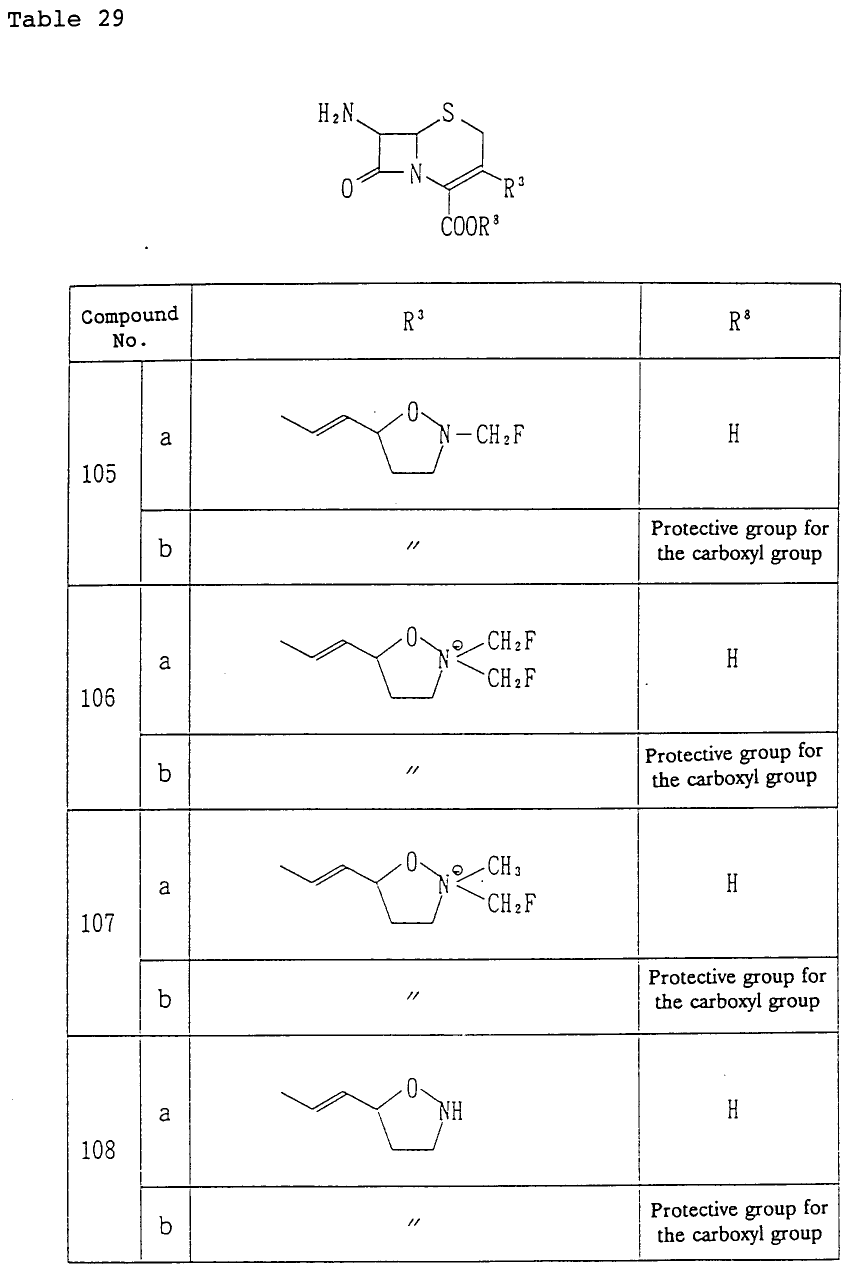

### INDUSTRIAL APPLICABILITY

The compound of the present invention shows excellent antibacterial activities against a broad range of strains belonging to Gram-positive bacteria and Gram-negative bacteria, especially against Gram-positive antibiotic-resistant bacteria such as MRSA and the like.

Antibacterial activities of the compound of the present invention are shown in the following table.

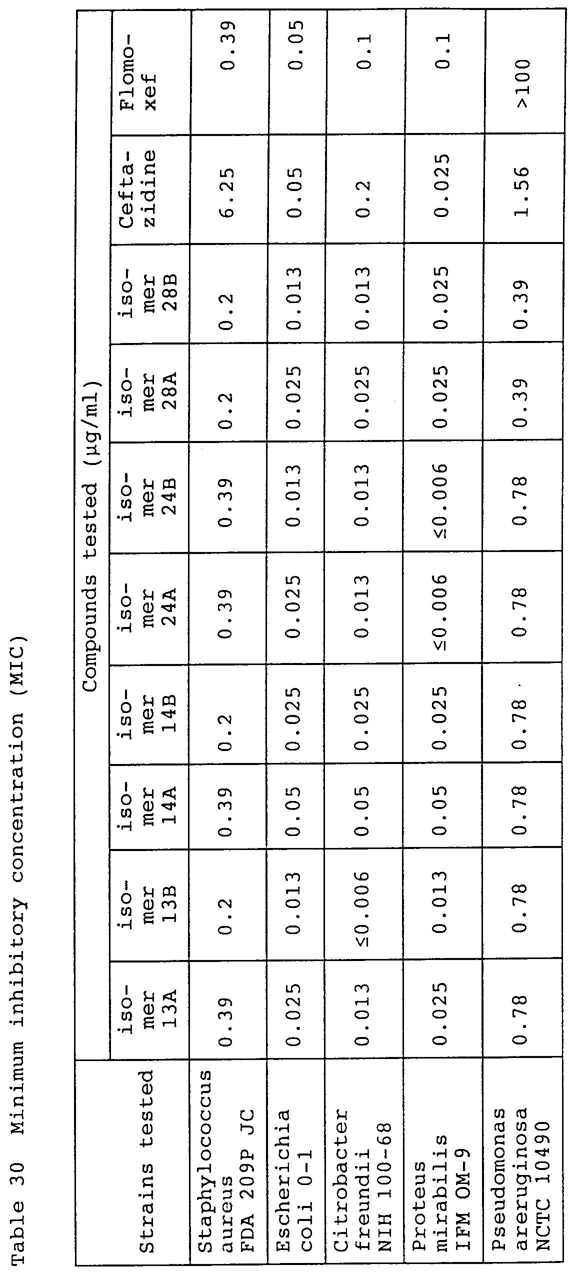

### Test on the curative effect against mouse abdominal infection:

Cells of each test strain cultured overnight at 37°C were suspended in mucin, and a 0.5 ml portion of the suspension was inoculated into a mouse (ICR or ddY male mouse, 4 to 5 weeks of age) in the usual way to effect infection with the strain. After 2 hours of the infection, 0.2 ml of each solution to be tested was administered by subcutaneous injection to observe mortality during 7 days thereafter and then ED₅₀ was calculated by the Probit or Weil method. Ten mice were used in one group, and sample to be tested was dissolved in physiological saline. The isomer B of the present invention obtained in Example 14 was used as the compound to be tested, the compound disclosed in Example 1 of unexamined published Japanese Patent Application (*Kokai*) No. 1-156984 (to be referred to as E-1077 hereinafter) and Flomoxef were used as control compounds of the curing effect against systemic infection with *Staphylococcus aureus* Smith and E-1077 and Ceftazidime were used as control compounds of the curing effect against systemic infection with *Pseudomonas aeruginosa*.

Results: The compound of the present invention showed infection-preventing effect about 10 times higher than those of E-1077 and Flomoxef against *S. aureus* Smith and about 2 times higher than E-1077 and about 8 times higher than Ceftazidime against *P. aeruginosa*.

Ceftazidime is a compound broadly used in the clinical field, because it is effective against Gram-negative bacteria, especially against *P. aeruginosa* which causes a problem as a causative strain of opportunistic infection, but the compound of the present invention has more superior effect.

Among compounds of the present invention, the compounds represented by the general formulae (II) and (A) are useful as intermediates in the aforementioned production process 4 for the production of the compound represented by the general formula (I) which is the objective compound of the present invention. Particularly, the butadiene compound represented by the general formula (A) has a completely new chemical structure due to its production process which is different from the prior art processes.

A pharmaceutical preparation which contains as the active ingredient 1 or more members of the compound (I) of the present invention is prepared making use of a carrier, a excipient and other additives commonly used for the preparation of medicines. The carrier and excipient may be in either solid or liquid form, which include, for example, lactose, magnesium stearate, starch, talc, gelatin, agar, pectin, gum arabic, olive oil, sesame oil, cacao butter, ethylene glycol and other commonly used substances.

The administration may be effected either by oral administration in the dosage form of tablets, pills, capsules, granules, powders, solutions and the like or by parenteral administration in the dosage form of injections for use in the intravenous injection, intramuscular injection and the like, suppositories, transdermal absorption preparations and the like. The dosage may optionally be decided depending on the symptoms, age and sex of each patient and the like, and it may generally be approximately from 200 to 4,000 mg per day.

### BEST MODE FOR CARRYING OUT THE INVENTION

Examples of the present invention are given below by way of illustration but the present invention should not be construed as being limited thereto.

In this connection, Examples of the production of starting compounds to be used in the Examples are described as the Reference Examples.

### Reference Example 1

(1) To dry tetrahydrofuran (64 ml) were added 6.36 g (8 mmol) of 7β-[(Z)-2-(2-tritylamino-4-thiazolyl-2-(methoxyimino)acetamido]-3-chloromethyl-3-cephem-4-carboxylate, 2.78 g (8.03 mmol) of (E)-3-hydroxy-1-tributylstannyl-1-propene, 92 mg (0.16 mmol) of bis(dibenzylideneacetone)palladium(0) and 74.4 mg (0.32 mmol) of tri(2-furyl)phosphine in an atmosphere of argon. After 2 hours of heating under reflux, the solvent was evaporated under a reduced pressure. The thus obtained residue was mixed with 50 ml of acetonitrile and washed twice with 50 ml of hexane and then the acetonitrile layer was evaporated under a reduced pressure.

The resulting residue was subjected to silica gel column chromatography and elution was carried out with hexane-ethyl acetate (2:3, v/v). Fractions containing the compound of interest were pooled, the solvent was evaporated under a reduced pressure, and then the resulting foamy product was solidified with ethyl ether-hexane to obtain 6 g (92%) of p-methoxybenzyl 7β-[(Z)-2-(2-tritylamino-4-thiazolyl)-2-(methoxyimino)acetamido]-3-[(E)-4-hydroxy-2-butenyl]-3-cephem-4-carboxylate.

### Physicochemical properties

Nuclear magnetic resonance spectrum (DMSO-d₆, TMS internal standard)
δ (ppm): 2.88 (1 H, dd, J = 14.2, 7.3 Hz), 3.17 (1 H, dd, J = 14.2, 4.9 Hz), 3.56 (1 H, d, J = 18.1 Hz), 3.74 (3 H, s), 3.80 (3 H, s), 3.86 (2 H, d, J = 4.9 Hz), 4.66 (1 H, t, J = 5.4 Hz), 5.1 - 5.63 (3 H, m), 6.71 (1 H, s), 6.92 (2 H, d, J = 8.3 Hz), 7.2 - 7.3 (17 H, m), 8.82 (1 H, s), 9.51 (1 H, d, J = 7.8 Hz)
Positive ion-FAB-mass spectrum: m/z; 816 (M + 1)⁺
Infrared absorption spectrum νₘₐₓ (KBr): 1784, 1522, 1250, 702 cm⁻¹
(2) In an atmosphere of argon, the thus obtained p-methoxybenzyl 7β-[(Z)-2-(2-tritylamino-4-thiazolyl)-2-(methoxyimino)acetamido]-3-[(E)-4-hydroxy-2-butenyl]-3- cephem-4-carboxylate (3.26 g) was dissolved in methylene chloride (25 ml), and the solution was cooled to -50°C, mixed with N,N-dimethylaminopyridine (733 mg) and diphenyl chlorophosphate (1.2 ml) and stirred at -50°C for 80 minutes. Thereafter, the reaction mixture was poured into methylene chloride (200 ml) and washed with 10% sodium dihydrogenphosphate aqueous solution (100 ml, twice), saturated sodium bicarbonate aqueous solution (100 ml), water (100 ml, twice) and saturated sodium chloride aqueous solution (100 ml) in that order, the resulting organic layer was dried over anhydrous magnesium sulfate and then the solvent was evaporated under a reduced pressure.

The resulting residue was subjected to silica gel column chromatography and elution was carried out with hexane-ethyl acetate (3:4, v/v). Fractions containing the compound of interest were pooled, the solvent was evaporated under a reduced pressure, and then the resulting foamy product was solidified with ethyl ether-hexane to obtain p-methoxybenzyl 7β-[(Z)-2-(2-tritylamino-4-thiazolyl)-2-(methoxyimino)acetamido]-3-[(E)-4-diphenylphosphoryloxy-2-butenyl]-3-cephem-4-carboxylate (3.30 g, 79%).

### Physicochemical properties

Nuclear magnetic resonance spectrum (DMSO-d₆, TMS internal standard)
δ (ppm): 2.94 (1 H, dd, J = 13.4, 6.7 Hz), 3.19 (1 H, dd, J = 13.4, 4.8 Hz), 3.26 (1 H, d, J = 18.3 Hz), 3.45 (1 H, d, J = 18.3 Hz), 3.73 (3 H, s), 3.80 (3 H, s), 4.71 (2 H, t, J = 6.7 Hz), 5.10 (1 H, d, J = 4.3 Hz), 5.16 (2 H, dd, J = 19.5, 12.2 Hz), 5.64 (1 H, dt, J = 10.4, 5.5 Hz), 5.69 (1 H, t, J = 6.1 Hz), 5.75 - 5.79 (1 H, m), 6.71 (1 H, s), 6.91 (2 H, d, J = 7.9 Hz), 7.22 - 7.43 (27 H, m), 8.83 (1 H, s), 9.53 (1 H, d, J = 7.9 Hz)
Positive ion-FAB-mass spectrum: m/z; 1048 (M)⁺
Infrared absorption spectrum νₘₐₓ (KBr): 1784, 1520, 1492, 1190, 1168, 956 cm⁻¹

### Reference Example 2

(1) To 400 ml of dry N,N-dimethylformamide were added 38.96 g (80 mmol) of p-methoxybenzyl 7β-(2-phenylacetamido)-3-chloromethyl-3-cephem-4-carboxylate, 27.69 g (80 mmol) of (E)-3-hydroxy-1-tributylstannyl-1,3-butadiene, 920 mg (1.6 mmol) of bis(dibenzylideneacetone)palladium(0) and 743 mg (3.2 mmol) of tri(2-furyl)phosphine in an atmosphere of argon.

After 3 hours of heating under reflux, the solvent was evaporated under a reduced pressure, the thus obtained residue was mixed with 400 ml of acetonitrile and washed three times with 400 ml of hexane. Then, the acetonitrile layer was evaporated under a reduced pressure. The thus obtained residue was mixed with 400 ml of ethyl acetate-benzene (1:1, v/v) and washed three times with 400 ml of water. The organic layer was dried over anhydrous magnesium sulfate and then evaporated under a reduced pressure. The resulting residue was subjected to silica gel column chromatography and elution was carried out with chloroform-methanol (100:1, v/v). Fractions containing the compound of interest were pooled and then the solvent was evaporated under a reduced pressure to obtain 38.5 g (94.6%) of p-methoxybenzyl 7β-(2-phenylacetamido)-3-[(E)-4-hydroxy-2-butenyl]-3-cephem-4-carboxylate.

### Physicochemical properties

Nuclear magnetic resonance spectrum (DMSO-d₆, TMS internal standard)
δ (ppm): 2.88 (1 H, dd, J = 14.2, 7.3 Hz), 3.18 (1 H, dd, J = 14.2, 4.9 Hz), 3.36 (1 H, d, J = 18.1 Hz), 3.52 (2 H, dd, J = 28.8, 14.2 Hz), 3.57 (1 H, d, J = 18.1 Hz), 3.75 (3 H, s), 3.88 (2 H, brs), 4.67 (1 H, t, J = 5.4 Hz), 5.08 (1 H, d, J = 4.4 Hz), 5.12 (1 H, d, J = 12.2 Hz), 5.22 (1 H, d, J = 12.2 Hz), 5.51 - 5.57 (1 H, m), 5.59 - 5.65 (2 H, m), 6.93 (2 H, d, J = 8.8 Hz), 7.2 - 7.3 (5 H, m), 7.34 (2 H, d, J = 8.8 Hz), 9.09 (1 H, d, J = 8.3 Hz)
Positive ion-FAB-mass spectrum: m/z; 509 (M + 1)⁺
Infrared absorption spectrum νₘₐₓ (KBr): 3292, 3048, 2972, 1784, 1718, 1654, 1618, 1540, 1520, 1366, 1252, 1174, 700 cm⁻¹
(2) In an atmosphere of argon, 33.3 g (66 mmol) of p-methoxybenzyl 7β-(2-phenylacetamido)-3-[(E)-4-hydroxy-2-butenyl]-3-cephem-4-carboxylate obtained in the above step (1) was dissolved in 350 ml of methylene chloride, and the solution was cooled to -70°C and mixed with 32.0 g of N,N-dimethylaminopyridine. Then, 54.6 ml (262 mmol) of diphenyl chlorophosphate was added dropwise at -70°C to 65°C spending 50 minutes, followed by 10 minutes of stirring at -65°C.

Thereafter, the reaction mixture was poured into 650 ml of methylene chloride and washed with 500 ml of saturated ammonium chloride aqueous solution (twice), 500 ml of saturated sodium bicarbonate aqueous solution, 500 ml of water and 500 ml of saturated sodium chloride aqueous solution in that order, the resulting organic layer was dried over anhydrous magnesium sulfate and then the solvent was evaporated under a reduced pressure. The resulting residue was subjected to silica gel column chromatography and elution was carried out with methylene chloride-ethyl acetate (10:1, v/v). Fractions containing the compound of interest were pooled and then the solvent was evaporated under a reduced pressure to obtain 32.9 g of p-methoxybenzyl 7β-(2-phenylacetamido)-3-[(E)-4-diphenylphosphoryloxy-2-butenyl]-3-cephem-4-carboxylate.

### Physicochemical properties

Nuclear magnetic resonance spectrum (DMSO-d₆, TMS internal standard)
δ (ppm): 2.93 (1 H, dd, J = 14.7, 7.3 Hz), 3.21 (1 H, dd, J = 14.0, 5.5 Hz), 3.29 (1 H, d, J = 18.3 Hz), 3.53 (2 H, dd, J = 36.0, 14.0 Hz), 3.54 (1H, d, J = 18.3 Hz), 3.73 (3 H, s), 4.73 (2 H, dd, J = 8.5, 6.1 Hz), 5.07 (1 H, d, J = 4.9 Hz), 5.18 (2 H, dd, J = 35.4, 12.2 Hz), 5.65 - 5.68 (1 H, m), 5.70 - 5.73 (1 H, m), 5.77 - 5.83 (1 H, m), 6.92 (2 H, d, J = 8.5 Hz), 7.1 - 7.4 (17 H, m), 9.10 (1 H, d, J = 8.5 Hz)
Positive ion-FAB-mass spectrum: m/z; 741 (M + 1)⁺
Infrared absorption spectrum νₘₐₓ (CHCl₃); 3440, 3012, 2976, 1788, 1728, 1688, 1616, 1594, 1494, 1286, 1168, 1014, 964 cm⁻¹

### Reference Example 3

(1) To 80 ml of dry tetrahydrofuran were added 7.95 g (10 mmol) of p-methoxybenzyl 7β-[(Z)-2-(5-tritylamino-1,2,4-thiadiazol-3-yl)-(2-methoxyiminoacetamido]-3-chloromethyl-3-cephem-4-carboxylate, 3.47 g (10 mmol) of (E)-3-hydroxy-1-tributylstannyl-1-propene, 115 mg (0.2 mmol) of bis(dibenzylideneacetone)palladium(0) and 93 mg (0.4 mmol) of tri(2-furyl)phosphine in an atmosphere of argon. After 3 hours of heating under reflux, the solvent was evaporated under a reduced pressure. The thus obtained residue was mixed with 200 ml of acetonitrile and washed with 200 ml and 100 ml of hexane and then the acetonitrile layer was separated and evaporated under a reduced pressure. The resulting residue was subjected to silica gel column chromatography and elution was carried out with hexane-ethyl acetate (gradient from 1:1 to 3:7, v/v). Fractions containing the compound of interest were pooled, the solvent was evaporated under a reduced pressure, and then the resulting foamy product was solidified with ethyl ether-hexane to obtain 5.94 g (72.7%) of p-methoxybenzyl 7β-[(Z)-2-(5-tritylamino-1,2,4-thiadiazol-3-yl)-2-(methoxyimino)acetamido]-3-[(E)-4-hydroxy-2-buten-1-yl]-3-cephem-4-carboxylate.

### Physicochemical properties

Nuclear magnetic resonance spectrum (DMSO-d₆, TMS internal standard)
δ (ppm): 2.87 (1 H, dd, J = 13.7, 6.8 Hz), 3.16 (1 H, dd, J = 13.7, 4.6 Hz), 3.54 (1 H, d, J = 18.6 Hz), 3.75 (3 H, s), 3.88 (5 H, m), 3.86 (2 H, d, J = 4.9 Hz), 4.66 (1 H, t, J = 5.5 Hz), 5.1 - 5.21 (3 H, m), 5.49 - 5.70 (2 H, m), 5.72 (1 H, m), 6.92 (2 H, d, J = 8.8 Hz), 7.2 - 7.4 (17 H, m), 9.50 (1 H, d, J = 8.8 Hz), 9.99 (1 H, s)
Positive ion-FAB-mass spectrum: m/z; 817 (M + H)⁺
Infrared absorption spectrum νₘₐₓ (KBr): 3416, 3316, 2952, 1778, 1726, 1690, 1520, 1248, 1042, 702 cm⁻¹
(2) In an atmosphere of argon, 5 g (6.1 mmol) of p-methoxybenzyl 7β-[(Z)-2-(5-tritylamino-1,2,4-thiadiazol-3-yl)-2-(methoxyimino)acetamido]-3-[(E)-4-hydroxy-2-butenyl]-3- cephem-4-carboxylate obtained in the above step (1) was dissolved in methylene chloride (40 ml). The solution was cooled to -50°C, mixed with N,N-dimethylaminopyridine (826 mg) and diphenylphosphoric acid chloride (1.55 ml), and stirred at -45°C for 60 minutes. Thereafter, the reaction mixture was poured into methylene chloride (200 ml) and washed with 10% sodium dihydrogenphosphate aqueous solution (100 ml, twice), saturated sodium bicarbonate aqueous solution (100 ml), water (100 ml, twice) and saturated sodium chloride aqueous solution (100 ml) in that order. The resulting organic layer was dried over anhydrous magnesium sulfate and then the solvent was evaporated under a reduced pressure. The resulting residue was subjected to silica gel column chromatography and elution was carried out with hexane-ethyl acetate (2:3 to 3:7, v/v). Fractions containing the compound of interest were pooled, the solvent was evaporated under a reduced pressure, and then the resulting foamy product was solidified by adding ethyl ether-hexane to obtain p-methoxybenzyl 7β-[(Z)-2-(5-tritylamino-1,2,4-thiadiazol-3-yl)-2-(methoxyimino)acetamido]-3-[(E)-4-diphenylphosphoryloxy-2-butenyl]-3-cephem-4-carboxylate (5.17 g, 80.5%).

### Physicochemical properties

Nuclear magnetic resonance spectrum (DMSO-d₆, TMS internal standard)
δ (ppm): 2.91 (1 H, dd, J = 14.2, 6.8 Hz), 3.18 (1 H, dd, J = 14.2, 5.1 Hz), 3.24 (1 H, d, J = 18.6 Hz), 3.51 (1 H, d, J = 18.6 Hz), 3.73 (3 H, s), 3.89 (3 H, s), 4.71 (2 H, t, J = 5.9 Hz), 5.07 - 5.2 (3 H, m), 5.65 - 5.8 (3 H, m), 6.91 (2 H, d, J = 8.3 Hz), 7.21 - 7.44 (27 H, m), 9.51 (1 H, d, J = 8.3 Hz), 9.99 (1 H, s)
Positive ion-FAB-mass spectrum: m/z; 1049 (M)⁺
Infrared absorption spectrum νₘₐₓ (KBr): 3300, 2952, 1778, 1726, 1686, 1520, 1492, 1394, 1248, 1188, 1168, 1094, 1040, 952, 754, 702, 690 cm⁻¹

### Reference Example 4

(1) To 80 ml of dry tetrahydrofuran were added 9.23 g (10 mmol) of p-methoxybenzyl 7β-[(Z)-2-(2-tritylamino-4-thiazolyl)-2-(1-tert-butoxycarbonyl-1-methylethoxyimino)acetamido]-3-chloromethyl-3-cephem-4-carboxylate, 3.47 g (10 mmol) of (E)-3-hydroxy-1-tributylstannyl-1-propene, 115 mg (0.2 mmol) of bis(dibenzylideneacetone)palladium(0) and 93 mg (0.4 mmol) of tri(2-furyl)phosphine in an atmosphere of argon. After 3 hours of heating under reflux, the solvent was evaporated under a reduced pressure, the thus obtained residue was mixed with 200 ml of acetonitrile and washed twice with 100 ml of hexane and then the acetonitrile layer was evaporated under a reduced pressure. The resulting residue was subjected to silica gel column chromatography and elution was carried out with hexane-ethyl acetate (1:1 to 2:3, v/v). Fractions containing the compound of interest were pooled, the solvent was evaporated under a reduced pressure, and then the resulting foamy product was solidified with ethyl ether-hexane to obtain 9.15 g (96.9%) of p-methoxybenzyl 7β-[(Z)-2-(2-tritylamino-4-thiazolyl)-2-(1-tert-butoxycarbonyl-1-methylethoxyimino)acetamido]-3-[ (E)-4-hydroxy-2-butenyl]-3-cephem-4-carboxylate.

### Physicochemical properties

Nuclear magnetic resonance spectrum (DMSO-d₆, TMS internal standard)
δ (ppm): 1.35 (15 H, s), 2.90 (1 H, dd, J = 14.2, 7.3 Hz), 3.18 (1 H, dd, J = 14.2, 4.9 Hz), 3.35 (1 H, d, J = 8.6 Hz), 3.55 (1 H, d, J = 18.6 Hz), 3.74 (3 H, s), 3.86 (2 H, m), 4.66 (1 H, t, J = 5.5 Hz), 5.1 - 5.21 (3 H, m), 5.49 - 5.67 (3 H, m), 6.69 (1 H, s), 6.92 (2 H, d, J = 8.8 Hz), 7.1 - 7.4 (17 H, m), 8.79 (1 H, s), 9.29 (1 H, d, J = 8.3)
Positive ion-FAB-mass spectrum: m/z; 944 (M)⁺
Infrared absorption spectrum νₘₐₓ (KBr): 3416, 3072, 2992, 1790, 1728, 1690, 1520, 1372, 1302, 1248, 1174, 1144, 702 cm⁻¹
(2) In an atmosphere of argon, 9.1 g (9.6 mmol) of p-methoxybenzyl 7β-[(Z)-2-(2-tritylamino-4-thiazolyl)-2-(1-tert-butoxycarbonyl-1-methylethoxyimino)acetamido]-3-[(E)-4- hydroxy-2-butenyl]-3-cephem-4-carboxylate obtained in the above step (1) was dissolved in 62 ml of methylene chloride. The solution was cooled to -50°C, mixed with 1.28 g of N,N-dimethylaminopyridine and 2.39 ml of diphenylphosphoric acid chloride, followed by stirring at -45°C for 2 hours. Thereafter, the reaction mixture was poured into 200 ml of methylene chloride and washed with 100 ml of 10% sodium dihydrogenphosphate aqueous solution (twice), 100 ml of saturated sodium bicarbonate aqueous solution, 100 ml of water (twice) and 100 ml of saturated sodium chloride aqueous solution in that order. The resulting organic layer was dried over anhydrous magnesium sulfate and then the solvent was evaporated under a reduced pressure. The resulting residue was subjected to silica gel column chromatography and elution was carried out with hexane-ethyl acetate (3:2 to 2:3, v/v). Fractions containing the compound of interest were pooled, the solvent was evaporated under a reduced pressure, and then the resulting foamy product was solidified by adding ethyl ether-hexane to obtain 10.21 g (90%) of p-methoxybenzyl 7β-[(Z)-2-(2-tritylamino-4-thiazolyl)-2-(1-tert-butoxycarbonyl-1-methylethoxyimino)acetamido]-3-[(E)-4-diphenylphosphoryloxy-2-butenyl]-3-cephem-4-carboxylate.

### Physicochemical properties

Nuclear magnetic resonance spectrum (DMSO-d₆, TMS internal standard)
δ (ppm): 1.34 (15 H, s), 2.95 (1 H, dd, J = 14.6, 7.4 Hz), 3.19 (1 H, m), 3.52 (1 H, d, J = 18.3 Hz), 3.72 (3 H, s), 4.71 (2 H, t, J = 6.1 Hz), 5.1 - 5.2 (3 H, m), 5.6 - 5.8 (3 H, m), 6.69 (1 H, s), 6.91 (2 H, d, J = 8.6 Hz), 7.18 - 7.43 (27 H, m), 8.79 (1 H, s), 9.28 (1 H, d, J = 8.5 Hz)
Positive ion-FAB-mass spectrum: m/z; 1176 (M)⁺
Infrared absorption spectrum νₘₐₓ (KBr): 3412, 3072, 2988, 1788, 1728, 1692, 1520, 1494, 1368, 1300, 1192, 1172, 1144, 1012, 954, 759, 702 cm⁻¹

### Reference Example 5

(1) A 5.15 g (12 mmol) portion of (Z)-2-hydroxyimino-2-(tritylamino-4-thiazolyl)acetic acid was suspended in 60 ml of methylene chloride and 5.2 ml of 2-methoxypropene was added thereto at 4°C. After 30 minutes of stirring at room temperature, the solvent was evaporated under a reduced pressure. The resulting residue was mixed with 50 ml of methylene chloride and then with 2.63 g of phosphorus pentachloride at -25°C. The mixture was stirred at -45 to -20°C for 60 minutes to obtain a solution of (Z)-2-(1-methoxy-1-methyl)ethoxyimino-2-(2-tritylamino-4-thiazolyl)acetic acid chloride. Separately, p-methoxybenzyl 7β-amino-3-chloromethyl-3-cephem-4-carboxylate hydrochloride (ACLE·HCl) was suspended in 50 ml of methylene chloride, and 2.8 ml of bis(trimethylsilyl)acetamide was added thereto at 10°C. After 30 minutes of stirring at room temperature, 4 ml of pyridine and the above-described acetic acid chloride solution were added in that order at -60°C. After 30 minutes of stirring at -35 to -20°C, this was poured into 150 ml of saturated potassium dihydrogenphosphate aqueous solution and extracted twice with 100 ml of methylene chloride. The extract was washed with saturated potassium dihydrogenphosphate aqueous solution and dried over anhydrous magnesium sulfate. The solvent was evaporated under a reduced pressure, the thus obtained residue was subjected to silica gel column chromatography, and elution was carried out with hexane-ethyl acetate (3:1 to 1:1, v/v). Fractions containing the compound of interest were pooled and then the solvent was evaporated under a reduced pressure to obtain 7.65 g (81.5%) of foamy p-methoxybenzyl 3-chloromethyl-7β-[(Z)-2-(2-tritylamino-4-thiazolyl)-2-(1-methoxy-1-methyl)ethoxyiminoacetamido]-3-cephem-4-carboxylate.

### Physicochemical properties

Nuclear magnetic resonance spectrum (DMSO-d₆, TMS internal standard)
δ (ppm): 3.11 (3 H, s), 3.53 (1 H, d, J = 18.1 Hz), 3.70 (11, d, J = 18.6 Hz), 3.74 (3 H, s), 4.50 (2 H, q, J = 23.4, J = 11.2 Hz), 5.1 - 5.3 (3 H, m), 5.73 (1 H, q, J = 4.9 Hz), 6.71 (1 H, s), 6.92 (2 H, d, J = 6.8 Hz), 7.2 - 7.4 (17 H, m), 8.86 (1 H, s), 9.53 (1 H, d, J = 8.3)
Positive ion-FAB-mass spectrum: m/z; 852 (M)⁺
Infrared absorption spectrum νₘₐₓ (KBr): 3416, 3072, 2948, 1794, 1730, 1692, 1630, 1618, 1522, 1386, 1374, 1250, 1178, 1164, 1068, 956, 924, 702 cm⁻¹
(2) To 80 ml of dry tetrahydrofuran were added 8.53 g (0.01 mol) of p-methoxybenzyl 3-chloromethyl-7β-[(Z)-2-(2-tritylamino-4-thiazolyl)-2-(1-methoxy-1-methyl)ethoxyiminoacetamido]-3-cephem-4-carboxylate, 3.47 g (0.01 mol) of 3-hydroxy-1-tributylstannyl-1-propene, 115 mg (0.2 mmol) of bis(dibenzylideneacetone)palladium(0) and 93 mg (0.4 mmol) of tri(2-furyl)phosphine in an atmosphere of argon. After 23 hours of heating under reflux, the solvent was evaporated under a reduced pressure. The thus obtained residue was mixed with 100 ml of acetonitrile and washed twice with 100 ml of hexane and then the acetonitrile layer was evaporated under a reduced pressure. The resulting residue was subjected to silica gel column chromatography and elution was carried out with hexane-ethyl acetate (3:2 to 2:3, v/v). Fractions containing the compound of interest were pooled, the solvent was evaporated under a reduced pressure, and then the resulting foamy product was solidified with ethyl ether-hexane to obtain 2.54 g (29.2%) of p-methoxybenzyl 7β-[(Z)-2-(2-tritylamino-4-thiazolyl)-2-(1-methoxy-1-methyl)ethoxyiminoacetamido]-3-[(E)-4-hydroxy-2-butenyl]-3-cephem-4-carboxylate.

### Physicochemical properties

Nuclear magnetic resonance spectrum (DMSO-d₆, TMS internal standard)
δ (ppm): 1.38 (6 H, s), 2.89 (1 H, dd, J = 14.2, 6.8 Hz), 3.17 (1 H, dd, J = 14.2, 5.4 Hz), 3.31 (3 H, s), 3.35 (d, J = 18.6 Hz), 3.55 (1 H, d, J = 18.6 Hz), 3.74 (3 H, s), 3.86 (2 H, m), 4.65 (1 H, t, J = 5.4 Hz), 5.1 - 5.2 (3 H, m), 5.5 - 5.7 (4 H, m), 6.71 (1 H, s), 6.92 (2 H, d, J = 6.8 Hz), 7.1 - 7.4 (17 H, m), 8.85 (1 H, s), 9.45 (1 H, d, J = 7.8)
Positive ion-FAB-mass spectrum: m/z; 874 (M)⁺, 802 (M-C(CH₃)₂OCH₃ + 1)⁺
Infrared absorption spectrum νₘₐₓ (KBr): 3416, 3072, 3008, 2952, 2848, 1788, 1728, 1686, 1618, 1520, 1376, 1300, 1248, 1176, 1072, 1034, 978, 902, 848, 824, 754, 702 cm⁻¹
(3) In an atmosphere of argon, 1.6 g (1.83 mmol) of p-methoxybenzyl 7β-[(Z)-2-(2-tritylamino-4-thiazolyl)-2-(1-methoxy-1-methyl)ethoxyiminoacetamido]-3-[(E)-4-hydroxy-2-butenyl]-3-cephem-4-carboxylate obtained in the above step (2) was dissolved in 12 ml of methylene chloride, and the solution was cooled to -50°C, mixed with 0.27 g (2 mmol) of N,N-dimethylaminopyridine and 0.455 ml of diphenylphosphoric acid chloride, followed by stirring -45 to -20°C for 2 hours. Thereafter, the reaction mixture was poured into 100 ml of 10% sodium dihydrogenphosphate aqueous solution and extracted twice with 50 ml of methylene chloride. The extract was washed with 20 ml of saturated sodium bicarbonate aqueous solution, 20 ml of water (twice) and 20 ml of saturated sodium chloride aqueous solution in that order and dried over anhydrous magnesium sulfate. Then, the solvent was evaporated under a reduced pressure. The resulting residue was subjected to silica gel column chromatography and elution was carried out with hexane-ethyl acetate (3:2 to 1:1 to 2:3, v/v). Fractions containing the compound of interest were pooled, the solvent was evaporated under a reduced pressure, and then the resulting foamy product was solidified with ethyl ether-hexane to obtain 1.46 g (71.9%) of p-methoxybenzyl 7β-[(Z)-2-(2-tritylamino-4-thiazolyl)-2-(1-methoxy-1-methyl)ethoxyiminoacetamido]-3-[(E)-4-diphenylphosphoryloxy-2-butenyl]-3-cephem-4-carboxylate.

### Physicochemical properties

Nuclear magnetic resonance spectrum (DMSO-d₆, TMS internal standard)
δ (ppm): 1.37 (6 H, s), 2.95 (1 H, dd, J = 14.7, 6.8 Hz), 3.10 (3 H, s), 3.18 (1 H, dd, J = 14.7, 9.3 Hz), 3.29 (1 H, d, J = 18.6 Hz), 3.54 (1 H, d, J = 18.6 Hz), 3.72 (3 H, s), 4.70 (2 H, dd, J = 9.3, 8.8 Hz), 5.1 - 5.2 (3 H, m), 5.6 - 5.8 (3 H, m), 6.71 (1 H, s), 6.90 (2 H, d, J = 8.8 Hz), 7.19 - 7.44 (27 H, m), 8.86 (1 H, s), 9.47 (1 H, d, J = 7.8 Hz)
Positive ion-FAB-mass spectrum: m/z; 1106 (M)⁺, 1034 (M-C(CH₃)₂OCH₃ + 1)⁺
Infrared absorption spectrum νₘₐₓ (KBr): 3420, 3072, 2954, 2884, 1788, 1728, 1686, 1592, 1520, 1492, 1376, 1286, 1248, 1220, 1190, 1166, 1072, 1012, 954, 772, 754, 702, 692 cm⁻¹

### Reference Example 6

(1) A 1.38 g (3.0 mmol) portion of (Z)-2-(2-tritylamino-4-thiazolyl)-2-fluoromethoxyiminoacetic acid was suspended in 30 ml of methylene chloride, and 621 mg (3.0 mmol) of phosphorus pentachloride was added thereto at -10°C. The mixture was stirred for 30 minutes at the same temperature to obtain an acid chloride solution. Separately, 1.22 g (3.0 mmol) of ACLE hydrochloride was suspended in 30 ml of methylene chloride, and 1.5 ml of N,O-bis(trimethylsilyl)acetamide and 1.5 ml of pyridine were added thereto. After 10 minutes of stirring, this solution was cooled to -50°C, mixed with the acid chloride solution prepared as above and stirred for 30 minutes at the same temperature. The reaction solution was poured into 160 ml of saturated sodium dihydrogenphosphate aqueous solution. The resulting organic layer was separated, washed with water and saturated sodium chloride aqueous solution and dried over anhydrous magnesium sulfate, and then the solvent was evaporated under a reduced pressure. The thus obtained residue was subjected to silica gel column chromatography and elution was carried out with chloroform-methanol (20:1). Fractions containing the compound of interest were pooled, the solvent was evaporated under a reduced pressure, and then the resulting foamy product was solidified with ethyl ether to obtain 1.87 g (76,8%) of p-methoxybenzyl 3-chloromethyl-7β-[(Z)-2-(2-tritylamino-4-thiazolyl)-2-fluoromethoxyimino)acetamido]-3-cephem-4-carboxylate.

### Physicochemical properties

Nuclear magnetic resonance spectrum (DMSO-d₆, TMS internal standard)
δ (ppm): 3.53 (1 H, d, J = 17.6 Hz), 3.70 (1 H, d, J = 17.6 Hz), 3.76 (3 H, s), 4.46 (1 H, d, J = 11.2 Hz), 4.53 (1 H, d, J = 11.2 Hz), 5.10 - 5.25 (3 H, m), 5.70 (2 H, d, J = 55.2 Hz), 5.72 (1 H, m), 6.88 (1 H, s), 6.93 (1 H, d), 7.20 - 7.40 (17H, m), 8.92 (1 H, s), 9.77 (1 H, m)
Positive ion-FAB-mass spectrum: m/z; 812 (M + 1)⁺
Infrared absorption spectrum νₘₐₓ (KBr): 1792, 1730, 1692, 1522, 1496, 1366, 1250, 1176, 1098, 1068, 702 cm⁻¹
(2) To dry tetrahydrofuran (80 ml) were added 1.7 g (2.1 mmol) of p-methoxybenzyl 7β-[(Z)-2-(2-tritylamino-4-thiazolyl)-2-fluoromethoxyimino)acetamido]-3-chloromethyl-3-cephem-4-carboxylate, 724 mg (2.1 mmol) of 3-hydroxy-1-tributylstannyl-1-propene, 24 mg (0.042 mmol) of bis(dibenzylideneacetone)palladium(0) and 20 mg (0.086 mmol) of tri(2-furyl)phosphine in an atmosphere of argon. After 4 hours of heating under reflux, the solvent was evaporated under a reduced pressure. The thus obtained residue was mixed with 25 ml of acetonitrile and washed twice with 20 ml of hexane and then the acetonitrile layer was evaporated under a reduced pressure. The resulting residue was subjected to silica gel column chromatography and elution was carried out with hexane-ethyl acetate (3:2 to 2:3, v/v). Fractions containing the compound of interest were pooled, the solvent was evaporated under a reduced pressure and then the resulting foamy product was solidified with ethyl ether-hexane to obtain 1.48 g (84.8%) of p-methoxybenzyl 7β-[(Z)-2-(2-tritylamino-4-thiazolyl)-2-fluoromethoxyimino)acetamido]-3-[(E)-4-hydroxy-2-butenyl]-3-cephem-4-carboxylate.

### Physicochemical properties

Nuclear magnetic resonance spectrum (DMSO-d₆, TMS internal standard)
δ (ppm): 2.89 (1 H, dd, J = 14.2, 6.8 Hz), 3.17 (1 H, dd, J = 14.2, 5.4 Hz), 3.34 (1 H, d, J = 18.6 Hz), 3.55 (1 H, d, J = 18, 6 Hz), 3.74 (3 H, s), 3.86 (2 H, m), 4.65 (1 H, t, J = 5.5 Hz), 5.1 - 5.2 (3 H, m), 5.51 - 5.65 (4 H, m), 5.76 (1 H, s), 6.88 (1 H, s), 6.92 (2 H, d, J = 8.8 Hz), 7.1 - 7.4 (17 H, m), 8.91 (1 H, s), 9.71 (1 H, d, J = 7.8)
Positive ion-FAB-mass spectrum: m/z; 834 (M + 1)⁺
Infrared absorption spectrum νₘₐₓ (KBr): 3416, 3320, 3068, 3040, 2972, 1784, 1728, 1686, 1520, 1368, 1302, 1248, 1174, 1096, 1034, 966, 754, 702 cm⁻¹
(3) In an atmosphere of argon, 1.4 g (1.68 mmol) of p-methoxybenzyl 7β-[(Z)-2-(2-tritylamino-4-thiazolyl)-2-fluoromethoxyimino)acetamido]-3-[(E)-4-hydroxy-2-butenyl]-3-cephem-4-carboxylate obtained in the above step (2) was dissolved in methylene chloride (11 ml), and the solution was cooled to 50°C, mixed with 0.25 g (2 mmol) of N,N-dimethylaminopyridine and 0.42 ml of diphenylphosphoric acid chloride, followed by stirring at -45 to -35°C for 5 hours. Thereafter, the reaction mixture was poured into 100 ml of 10% sodium dihydrogenphosphate aqueous solution and extracted twice with 50 ml of methylene chloride. The extract was washed with 20 ml of saturated sodium bicarbonate aqueous solution, 20 ml of water (twice) and 20 ml of saturated sodium chloride aqueous solution in that order and dried over anhydrous magnesium sulfate. Then, the solvent was evaporated under a reduced pressure. The resulting residue was subjected to silica gel column chromatography and elution was carried out with hexane-ethyl acetate (3:2 to 1:1 to 2:3, v/v). Fractions containing the compound of interest were pooled, the solvent was evaporated under a reduced pressure, and then the resulting foamy product was solidified by adding ethyl ether-hexane to obtain 1.34 g (74.8%) of p-methoxybenzyl 7β-[(Z)-2-(2-tritylamino-4-thiazolyl)-2-fluoromethoxyimino)acetamido]-3-[(E)-4-diphenylphosphoryloxy-2-butenyl]-3-cephem-4-carboxylate.

### Physicochemical properties

Nuclear magnetic resonance spectrum (DMSO-d₆, TMS internal standard)
δ (ppm): 2.95 (1 H, dd, J = 14.7, 7.3 Hz), 3.20 (1 H, dd, J = 14.7, 5.4 Hz), 3.25 (1 H, d, J = 18.1 Hz), 3.51 (1 H, d, J = 18.6 Hz), 3.73 (3 H, s), 4.70 (2 H, dd, J = 8.8, 5.9 Hz), 5.1 - 5.2 (3 H, m), 5.6 - 5.8 (6 H, m), 6.80 - 6.94 (3 H, m), 7.2 - 7.44 (27 H, m), 8.91 (1 H, s), 9.72 (1 H, d, J = 8.3 Hz)
Positive ion-FAB-mass spectrum: m/z; 1066 (M)⁺
Infrared absorption spectrum νₘₐₓ (KBr): 3420, 3300, 3072, 2976, 1786, 1728, 1686, 1592, 1520, 1492, 1370, 1286, 1248, 1224, 1190, 1168, 1096, 1070, 1012, 952, 754, 702, 690 cm⁻¹

### Reference Example 7

(1) Using 1.34 g (1.65 mmol) of p-methoxybenzyl 7β-[(Z)-2-(5-tritylamino-1,2,4-thiadiazol-3-yl)-2-fluoromethoxyimino)acetamido]-3-chloromethyl-3-cephem-4-carboxylate as the starting material, the procedure of Reference Example 3 (1) was repeated to obtain 1.25 g (91.0%) of p-methoxybenzyl 7β-[(Z)-2-(5-tritylamino-1,2,4-thiadiazol-3-yl)-2-fluoromethoxyimino)acetamido)-3-[(E)-4-hydroxy-2-butenyl]-3-cephem-4-carboxylate.

### Physicochemical properties

Nuclear magnetic resonance spectrum (DMSO-d₆, TMS internal standard)
δ (ppm): 2.87 (1 H, dd, J = 7.3, 14.2 Hz), 3.16 (1 H, dd, J = 4.9, 13.7 Hz), 3.32 (1 H, d, J = 18.6 Hz), 3.55 (1 H, d, J = 18.6 Hz), 3.75 (3 H, s), 3.86 (2 H, m), 4.65 (1 H, t, J = 5.5 Hz), 5.1 - 5.21 (3 H, m), 5.49 - 5.75 (4 H, m), 5.83 (1 H, s), 6.92 (2 H, d, J = 8.3 Hz), 7.2 - 7.5 (17 H, m), 9.68 (1 H, d, J = 8.3 Hz), 10.08 (1 H, s)
Positive ion-FAB-mass spectrum: m/z; 835 (M + 1)⁻
Infrared absorption spectrum νₘₐₓ (KBr): 3416, 3044, 2972, 1780, 1724, 1692, 1618, 1532, 1520, 1450, 1394, 1368, 1304, 1248, 1174, 1124, 1076, 1004, 754, 702 cm⁻¹
(2) Using 1.25 g (1.5 mmol) of p-methoxybenzyl 7β-[(Z)-2-(5-tritylamino-1,2,4-thiadiazol-3-yl)-2-fluoromethoxyimino)acetamido]-3-[(E)-4-hydroxy-2-butenyl]-3-cephem-4-carboxylate obtained in the above step (1) as the starting material, the procedure of Reference Example 3 (2) was repeated to obtain 1.4 g (87.5%) of p-methoxybenzyl 7β-[(Z)-2-(5-tritylamino-1,2,4-thiadiazol-3-yl)-2-fluoromethoxyimino)acetamido]-3-[(E)-4-diphenylphosphoryloxy-2-butenyl]-3-cephem-4-carboxylate.

### Physicochemical properties

Nuclear magnetic resonance spectrum (DMSO-d₆, TMS internal standard)
δ (ppm): 2.92 (1 H, dd, J = 7.3, 14.7 Hz), 3.19 (1 H, m), 3.24 (1 H, d, J = 18.6 Hz), 3.52 (1 H, d, J = 18.6 Hz), 3.73 (3 H, s), 4.70 (2 H, dd, J = 5.9, 8.8 Hz), 5.1 - 5.2 (3 H, m), 5.6 - 5.9 (5 H, m), 6.91 (2 H, d, J = 8.5 Hz), 7.1 - 7.5 (27 H, m), 9.68 (1 H, d, J = 8.3 Hz), 10.08 (1 H, s)
Positive ion-FAB-mass spectrum: m/z; 1067 (M + 1)⁺
Infrared absorption spectrum νₘₐₓ (KBr): 3416, 3280, 3072, 2976, 1784, 1726, 1692, 1616, 1592, 1522, 1492, 1452, 1392, 1368, 1286, 1250, 1222, 1188, 1164, 1124, 1076, 1012, 954, 756, 702, 690, 522 cm⁻¹

### Example 1

A 3.03 g (2.89 mmol) portion of p-methoxybenzyl 7β-[(Z)-2-(2-tritylamino-4-thiazolyl)-2-(methoxyimino)acetamido]-3-[(E)-4-diphenylphosphoryloxy-2-butenyl]-3-cephem-4-carboxylate obtained in Reference Example 1 (2) was dissolved in 60 ml of acetonitrile. To the resulting solution cooled in an ice bath was added dropwise diisopropylethylamine (1.5 ml). After stirring for a whole day and night at the same temperature, the solvent was evaporated under a reduced pressure. The resulting residue was subjected to silica gel column chromatography and elution was carried out with methylene chloride-ethyl acetate (15:1, v/v). Fractions containing the compound of interest were pooled, the solvent was evaporated under a reduced pressure, and then the resulting foamy product was solidified with ethyl ether-hexane to obtain 2.19 g (94%) of p-methoxybenzyl 7β-[(Z)-2-(2-tritylamino-4-thiazolyl)-2-(methoxyimino)acetamido]-3-[(E)-1,3-butadienyl]-3-cephem-4-carboxylate.

### Physicochemical properties

Nuclear magnetic resonance spectrum (DMSO-d₆, TMS internal standard)
δ (ppm): 3.61 (1 H, d, J = 17.6 Hz), 3.75 (3 H, s), 3.81 (3 H, s), 3.89 (1 H, d, J = 17.6 Hz), 5.18 (1 H, d, J = 4.9 Hz), 5.20 (2 H, s), 5.26 (1 H, d, J = 11.5 Hz), 5.39 (1 H, d, J = 17.1 Hz), 5.69 (1 H, dd, J = 8.0, 4.9 Hz), 6.3 - 6.4 (1 H, m), 6.7 - 6.8 (3 H, m), 6.94 (2 H, d, J = 9.2 Hz), 7.2 - 7.4 (17 H, m), 8.85 (1 H, s), 9.59 (1 H, d, J = 7.9 Hz)
Positive ion-FAB-mass spectrum: m/z; 799 (M + 2)⁻
Infrared absorption spectrum νₘₐₓ (KBr): 3304, 1780, 1724, 1686, 1520, 1306, 1248, 1168, 1038, 702 cm⁻¹

### Example 2

A 32.1 g (43 mmol) portion of p-methoxybenzyl 7β-(2-phenylacetamido)-3-[(E)-4-diphenylphosphoryloxy-2-butenyl]-3-cephem-4-carboxylate was dissolved in 480 ml of acetonitrile. To the resulting solution cooled in an ice bath was added dropwise 14.6 ml (86 mmol) of diisopropylethylamine. After stirring for a whole day and night at the same temperature, the precipitate was collected by filtration, washed with diisopropyl ether and dried under a reduced pressure to obtain 5.1 g of p-methoxybenzyl 7β-(2-phenylacetamido)-3-[(E)-1,3-butadienyl]-3-cephem-4-carboxylate.

The filtrate obtained above was evaporated under a reduced pressure, the resulting residue was subjected to silica gel column chromatography and elution was carried out with methylene chloride-ethyl acetate (10:1, v/v). Fractions containing the compound of interest were pooled and then the solvent was evaporated under a reduced pressure to obtain 4.9 g of p-methoxybenzyl 7β-(2-phenylacetamido)-3-[(E)-1,3-butadienyl]-3-cephem-4-carboxylate.

### Physicochemical properties

Nuclear magnetic resonance spectrum (DMSO-d₆, TMS internal standard)
δ (ppm): 3.53 (2 H, dd, J = 25.9, 14.2 Hz), 3.62 (1 H, d, J = 17.6 Hz), 3.75 (3 H, s), 3.94 (1 H, d, J = 17.6 Hz), 5.15 (1 H, d, J = 4.9 Hz), 5.17 - 5.27 (3 H, m), 5.39 (1 H, d, J = 17.1 Hz), 5.70 (1 H, dd, J = 8.3, 4.9 Hz), 6.66 - 6.77 (2 H, m), 6.94 (2 H, d, J = 8.8 Hz), 7.21 - 7.32 (5 H, m), 7.36 (2 H, d, J = 8.8 Hz), 9.16 (1 H, d, J = 8.3 Hz)
Positive ion-FAB-mass spectrum: m/z; 491 (M + 1)⁺
Infrared absorption spectrum νₘₐₓ (KBr): 3284, 3048, 2976, 1780, 1718, 1660, 1538, 1520, 1388, 1360, 1234, 1170, 698 cm⁻¹

### Alternative method

In an atmosphere of argon, 586 mg (1 mmol) of p-methoxybenzyl 7β-(2-phenylacetamido)-3-trifluoromethanesulfonyloxy-3-cephem-4-carboxylate was dissolved in 2 ml of 1-methyl-2-pyrrolidinone. Then, 273 mg (2 mmol) of zinc chloride, 12 mg (0.05 mmol) of tri(2-furyl)phosphine and 15 mg (0.025 mmol) of bis(dibenzylideneacetone)palladium(0) were added thereto. After 10 minutes of stirring, 377 mg (1.1 mmol) of (E)-1-tributylstannyl-1,3-butadiene and 2 ml of 1-methyl-2-pyrrolidine were added, followed by 4 hours of reaction at room temperature. The reaction-solution was diluted with 30 ml of ethyl acetate, washed three times with water and once with saturated sodium chloride aqueous solution and dried over anhydrous magnesium sulfate. The solvent was then evaporated under a reduced pressure. The thus obtained residue was dissolved in 80 ml of acetonitrile and washed three times with hexane. The acetonitrile layer was concentrated, and the resulting crude product (608 mg) was purified by silica gel column chromatography, thereby obtaining 385 mg (79%) of the compound of interest. Physicochemical properties of this compound were the same as those of p-methoxybenzyl 7β-(2-phenylacetamido)-3-[(E)-1,3-butadienyl]-3-cephem-4-carboxylate obtained in Example 2 (3).

### Example 3

(1) In an atmosphere of argon, 312 mg (1.5 mmol) of phosphorus pentachloride was dissolved in 5 ml of methylene chloride, and the solution was mixed with 122 µl (1.5 mmol) of pyridine at 5°C, followed by stirring for 1 hour. To the thus obtained reaction solution was added 245 mg (0.5 mmol) of p-methoxybenzyl 7β-(2-phenylacetamido)-3-[(E)-1,3-butadienyl]-3-cephem-4-carboxylate obtained in Example 2, followed by 1.5 hours of stirring at 8°C. The reaction solution was cooled to -30°C and mixed with 2 ml of methanol, and the mixture was stirred at -15°C for 1.5 hours. Thereafter, 5 ml of methylene chloride and 10 ml of water were added. The water layer was separated from the reaction solution, the remaining organic layer was extracted with water, and the water layers were combined, neutralized with saturated sodium bicarbonate aqueous solution while cooling in an ice bath and then extracted with chloroform. After drying the chloroform extract over anhydrous magnesium sulfate, the solvent was evaporated under a reduced pressure to obtain 175 mg of p-methoxybenzyl 7β-amino-3-[(E)-1,3-butadienyl]-3-cephem-4-carboxylate as a partially purified product.

### Physicochemical properties

Nuclear magnetic resonance spectrum (DMSO-d₆, TMS internal standard)
δ (ppm): 3.32 (2 H, brs), 4.82 (1 H, d, J = 4.9 Hz), 5.04 (1 H, d, J = 5.4 Hz), 5.19 (2 H, d, J = 2.4 Hz), 5.23 (1 H, d, J = 11.7 Hz), 5.97 (1 H, d, J = 16.6 Hz), 6.38 (1 H, dt, J = 17.1, 9.8 Hz), 6.63 - 6.75 (2 H, m)
Positive ion-FAB-mass spectrum: m/z; 372 (M + 1)⁺

### Example 4

A 5 g (4.76 mmol) portion of p-methoxybenzyl 7β-[(Z)-2-(5-tritylamino-1,2,4-thiadiazol-3-yl)-2-(methoxyimino)acetamido]-3-[(E)-4-diphenylphosphoryloxy-2-butenyl]-3-cephem-4-carboxylate obtained in Reference Example 3 (2) was dissolved in 100 ml of acetonitrile, and diisopropylethylamine (2.6 ml) was added dropwise to the resulting solution at room temperature. After stirring for a whole day and night at room temperature, the solvent was evaporated under a reduced pressure, the resulting residue was subjected to silica gel column chromatography and elution was carried out with methylene chloride-ethyl acetate (15:1, v/v). Fractions containing the compound of interest were pooled, the solvent was evaporated under a reduced pressure, and then the resulting foamy product was solidified with ethyl ether-hexane to obtain 3.4 g (89.5%) of p-methoxybenzyl 7β-[(Z)-2-(5-tritylamino-1,2,4-thiadiazol-3-yl)-2-(methoxyimino)acetamido]-3-[(E)-1,3-butadienyl]-3-cephem-4-carboxylate.

### Physicochemical properties

Nuclear magnetic resonance spectrum (DMSO-d₆, TMS internal standard)
δ (ppm): 3.58 (1 H, d, J = 17.6 Hz), 3.74 (3 H, s), 3.85 - 3.93 (5 H, s, d), 5.17 (2 H, m), 5.21 (1 H, d, J = 11.7 Hz), 5.38 (1 H, d, J = 17.1 Hz), 5.77 (1 H, m), 6.3 - 6.5 (1 H, m), 6.6 - 6.8 (2 H, m), 6.94 (2 H, d, J = 8.7 Hz), 7.2 - 7.4 (17 H, m), 9.57 (1 H, d, J = 8.3 Hz), 9.99 (1 H, s)
Positive ion-FAB-mass spectrum: m/z; 799 (M + 1)⁺
Infrared absorption spectrum νₘₐₓ (KBr): 3296, 2952, 1778, 1724, 1688, 1520, 1388, 1364, 1248, 1220, 1160, 1042, 702 cm⁻¹

### Example 5

A 9.95 g (8.46 mmol) portion of p-methoxybenzyl 7β-[(Z)-2-(2-tritylamino-4-thiazolyl)-2-(1-tert-butoxycarbonyl-1-methylethoxyimino)acetamido]-3-[(E)-4-diphenylphosphoryloxy-2-butenyl]-3-cephem-4-carboxylate obtained in Reference Example 4 (2) was dissolved in 177 ml of acetonitrile. To the resulting solution cooled in an ice bath was added dropwise diisopropylethylamine (4.6 ml). After stirring for a whole day and night at the same temperature, the solvent was evaporated under a reduced pressure, the resulting residue was subjected to silica gel column chromatography and elution was carried out with methylene chloride-ethyl acetate (15:1, v/v). Fractions containing the compound of interest were pooled, the solvent was evaporated under a reduced pressure, and then the resulting foamy product was solidified with ethyl ether-hexane to obtain 7.53 g (96.3%) of p-methoxybenzyl 7β-[(Z)-2-(2-tritylamino-4-thiazolyl)-2-(1-tert-butoxycarbonyl-1-methylethoxyimino)acetamido]-3-[(E)-1,3-butadienyl]-3-cephem-4-carboxylate.

### Physicochemical properties

Nuclear magnetic resonance spectrum (DMSO-d₆, TMS internal standard)
δ (ppm): 1.35 (15 H, s), 3.60 (1 H, d, J = 17.6 Hz), 3.91 (1 H, d, J = 15.8 Hz), 5.09 - 5.20 (1 H, m), 5.20 (2 H, s), 5.25 (1 H, d, J = 11.2 Hz), 5.38 (1 H, d, J = 16.6 Hz), 5.70 (1 H, m), 6.3 - 6.45 (1 H, m), 6.69 - 6.78 (3 H, m), 6.93 (2 H, d, J = 8.8 Hz), 7.2 - 7.4 (17 H, m), 8.80 (1 H, s), 9.36 (1 H, d, J = 8.3 Hz)
Positive ion-FAB-mass spectrum: m/z; 926 (M + 1)⁺
Infrared absorption spectrum νₘₐₓ (KBr): 3408, 2922, 2952, 1788, 1726, 1696, 1520, 1386, 1368, 1304, 1252, 1220, 1168, 1146, 1006, 702 cm⁻¹

### Example 6

A 1.4 g (1.27 mmol) portion of p-methoxybenzyl 7β-[(Z)-2-(2-tritylamino-4-thiazolyl)-2-(1-methoxy-1-methyl)ethoxyiminoacetamido]-3-[(E)-4-diphenylphosphoryloxy-2-butenyl)-3-cephem-4-carboxylate obtained in Reference Example 5 (3) was dissolved in 20 ml of acetonitrile. To the resulting solution cooled in an ice bath was added 0.69 ml of diisopropylethylamine. After stirring for 3 days with cooling in an ice bath, the solvent was evaporated under a reduced pressure. The resulting residue was subjected to silica gel column chromatography, elution was carried out with methylene chloride-ethyl acetate (15:1, v/v), and fractions containing the compound of interest were pooled. Thereafter, the solvent was evaporated under a reduced pressure and the resulting foamy product was solidified with ethyl ether-hexane to obtain 896 mg (82.7%) of p-methoxybenzyl 7β-[(Z)-2-(2-tritylamino-4-thiazolyl)-2-(1-methoxy-1-methyl)ethoxyiminoacetamido]-3-[(E)-1,3-butadienyl]-3-cephem-4-carboxylate.

### Physicochemical properties

Nuclear magnetic resonance spectrum (DMSO-d₆, TMS internal standard)
δ (ppm): 1.39 (6 H, s), 3.11 (3 H, s), 3.60 (1 H, d, J = 17.6 Hz), 3.74 (3 H, s), 3.91 (1 H, d, J = 17.6 Hz), 5.06 - 5.49 (5 H, m), 5.69 (1 H, d, J = 7.8 Hz), 6.39 (1 H, m), 6.66 - 6.78 (3 H, m), 6.93 (2 H, d, J = 8.79 Hz), 7.20 - 7.4 (17 H, m), 8.87 (1 H, s), 9.77 (1 H, d, J = 8.3 Hz)
Positive ion-FAB-mass spectrum: m/z; 856 (M)⁺, 784 (M-C(CH₃)₂OCH₃ + 1)⁺
Infrared absorption spectrum νₘₐₓ (KBr): 3336, 3072, 3004, 2952, 2844, 1790, 1722, 1692, 1618, 1516, 1450, 1384, 1306, 1248, 1220, 1170, 1106, 1070, 1034, 1004, 978, 892, 848, 824, 808, 754, 702 cm⁻¹

### Alternative method

A 2.1 g (4.9 mmol) portion of (Z)-2-hydroxyimino-(2-tritylamino-4-thiazolyl)acetic acid was suspended in 25 ml of methylene chloride, and 2.1 ml of 2-methoxypropene was added thereto at 4°C. After 30 minutes of stirring at room temperature, the solvent was evaporated under a reduced pressure. The resulting residue was mixed with 20 ml of methylene chloride and then with 1.05 g of phosphorus pentachloride at -21°C, and the mixture was stirred for 50 minutes at -45 to -20°C to obtain (Z)-2-(1-methoxy-1-methyl)ethoxyimino-2-(2-tritylamino-4-thiazolyl)acetic acid chloride solution. Separately, 1.82 g (4.45 mmol) of p-methoxybenzyl 7β-amino-3-[(E)-1,3-butadienyl]-3-cephem-4-carboxylate hydrochloride was suspended in 20 ml of methylene chloride, and 1.12 ml of bis(trimethylsilyl)acetamide was added thereto at 4°C. After 30 minutes of stirring at room temperature, 1.6 ml of pyridine and the above-described acetic acid chloride solution were added thereto in that order at -60°C. After 30 minutes of stirring at -35 to -20°C, this was poured into 100 ml of saturated potassium dihydrogenphosphate aqueous solution and the mixture was extracted twice with 100 ml of methylene chloride. The extract was washed with saturated potassium dihydrogenphosphate aqueous solution and dried over anhydrous magnesium sulfate. The solvent was evaporated under a reduced pressure, the thus obtained residue was subjected to silica gel column chromatography, and elution was carried out with hexane-ethyl acetate (3:2 to 1:1, v/v). Fractions containing the compound of interest were pooled and then the solvent was evaporated under a reduced pressure to obtain 625 mg (16.4%) of p-methoxybenzyl 7β-[(Z)-2-(2-tritylamino-4-thiazolyl)-2-(1-methoxy-1-methyl)ethoxyiminoacetamido]-3-[(E)-1,3-butadienyl]-3-cephem-4-carboxylate as a foamy compound.

### Example 7

A 1.32 g (1.24 mmol) portion of p-methoxybenzyl 7β-[(Z)-2-(2-tritylamino-4-thiazolyl)-2-fluoromethoxyimino)acetamido)-3-[(E)-4-diphenylphosphoryloxy-2-butenyl]-3-cephem-4-carboxylate obtained in Reference Example 6 (3) was dissolved in 20 ml of acetonitrile. To the resulting solution cooled in an ice bath was added 0.67 ml of diisopropylethylamine. After stirring for two days and nights in an ice bath, the solvent was evaporated under a reduced pressure. The resulting residue was subjected to silica gel column chromatography and elution was carried out with methylene chloride-ethyl acetate (15:1, v/v). Fractions containing the compound of interest were pooled, the solvent was evaporated under a reduced pressure, and then the resulting foamy product was solidified with ethyl ether-hexane to obtain 966 mg (95.6%) of p-methoxybenzyl 7β-[(Z)-2-(2-tritylamino-4-thiazolyl)-2-fluoromethoxyimino)acetamido]-3-[(E)-1,3-butadienyl]-3-cephem-4-carboxylate.

### Physicochemical properties

Nuclear magnetic resonance spectrum (DMSO-d₆, TMS internal standard)
δ (ppm): 3.62 (1 H, d, J = 17.6 Hz), 3.88 (1 H, d, J = 17.6 Hz), 5.09 - 5.30 (4 H, m), 5.39 (1 H, d, J = 17.1 Hz), 5.63 (1 H, s), 5.70 (1 H, dd, J = 7.8, 4.9 Hz), 5.77 (1 H, s), 6.3 - 6.5 (1 H, m), 6.65 - 6.8 (2 H, m), 6.89 (1 H, s), 6.93 (2 H, d, J = 8.8 Hz), 7.2 - 7.4 (17 H, m), 8.92 (1 H, s), 9.77 (1 H, d, J = 7.8 Hz)
Positive ion-FAB-mass spectrum: m/z; 816 (M + 1)⁺
Infrared absorption spectrum νₘₐₓ (KBr): 3312, 3068, 3040, 2980, 2844, 1788, 1726, 1692, 1616, 1520, 1466, 1386, 1364, 1306, 1248, 1220, 1168, 1098, 1068, 1034, 1004, 770, 702 cm⁻¹

### Example 8

A 5.69 g portion of (Z)-2-(5-tert-butoxycarbonylamino-1,2,4-thiadiazol-3-yl)-2-hydroxyiminoacetic acid was mixed with 60 ml of methylene chloride and 8.5 ml of 2-methoxypropene and the mixture was stirred for 30 minutes at room temperature. Then, the solvent was evaporated under a reduced pressure to obtain 7.22 g of (Z)-2-(5-tert-butoxycarbonylamino-1,2,4-thiadiazol-3-yl)-2-(1-methoxy-1-methyl)ethoxyiminoacetic acid as a foamy compound. A 1.72 g (4.77 mmol) portion of the thus obtained compound was dissolved in 20 ml of methylene chloride. The solution was mixed with 1.03 g (4.95 mmol) of phosphorus pentachloride at -20°C and stirred for 1 hour to prepare an acid chloride. Separately, 1.601 g (4.30 mmol) of 4-methoxybenzyl 7-amino-3-(1,3-butadienyl)-3-cephem-4-carboxylate hydrochloride was mixed with 20 ml of methylene chloride and 1.12 ml of bistrimethylsilylacetamide (BSA) and the mixture was stirred at room temperature for 85 minutes to prepare a uniform solution. This solution was cooled to -40°C and mixed with 1.6 ml of pyridine and the aforementioned acid chloride in that order to carry out 1.5 hours of reaction. The reaction solution was poured into 100 ml of saturated sodium dihydrogenphosphate solution and extracted twice with 100 ml of chloroform. The organic layers were combined, washed three times with saturated sodium dihydrogenphosphate solution and once with saturated sodium chloride aqueous solution, and then dried over anhydrous magnesium sulfate. Thereafter, the solvent was evaporated under a reduced pressure. The resulting crude product was subjected to silica gel column chromatography and elution was carried out with hexane-ethyl acetate (1:1, v/v) to obtain 368 mg of p-methoxybenzyl 7β-[(Z)-2-(5-tert-butoxycarbonylamino-1,2,4-thiadiazol-3-yl)-2-(1-methoxy-1-methyl)ethoxyiminoacetamido]-3-[(E)-1,3-butadienyl]-3-cephem-4-carboxylate.

### Physicochemical properties

Nuclear magnetic resonance spectrum (DMSO-d₆, TMS internal standard)
δ (ppm): 1.46 (6 H, s), 1.50 (9 H, s), 3.17 (3 H, s), 3.63 and 3.95 (2 H, ABq), 3.76 (3 H, s), 5.21 (1 H, d, J = 6 Hz), 5.23 - 5.27 (2 H, m), 5.38 (1 H, d, J = 17 Hz), 5.88 (1 H, q, J = 6.8 Hz), 6.38 - 6.42 (1 H, m), 6.7 (1 H, m), 6.94 and 7.36 (4 H, ABq), 9.69 (1 H, d)
Positive ion-FAB-mass spectrum: m/z;
643 (M-CH(CH₃)₂OCH₃ + H)⁺
Infrared absorption spectrum νₘₐₓ (KBr): 2996, 1788, 1722, 1548, 1386, 1374, 1250, 1154 cm⁻¹

### Example 9

(1) A 4.9 g (10 mmol) portion of p-methoxybenzyl 7β-(2-phenylacetamido)-3-[(E)-1,3-butadienyl]-3-cephem-4-carboxylate, 1.23 g (15 mmol) of sodium acetate and 1.58 mg (20 mmol) of 38% aqueous formalin were dissolved in 150 ml of tetrahydrofuran, and 1.25 g (15 mmol) of N-methylhydroxyamine hydrochloride that has been dissolved in 20 ml of 80% aqueous ethanol was added thereto. After 4.5 hours of heating under reflux, the solvent was evaporated under a reduced pressure. The resulting residue was dissolved in methylene chloride, washed with saturated sodium bicarbonate aqueous solution, saturated ammonium chloride aqueous solution, water and saturated sodium chloride aqueous solution and dried over anhydrous magnesium sulfate. Then, the solvent was evaporated under a reduced pressure.

The resulting residue was subjected to silica gel column chromatography and elution was carried out with methylene chloride-methanol (50:1, v/v). Fractions containing the compound of interest were pooled and then the solvent was evaporated under a reduced pressure to obtain 5.35 g of p-methoxybenzyl 7β-(2-phenylacetamido)-3-[(E)-2-(2-methyl-5-isooxazolidinyl)vinyl]-3-cephem-4-carboxylate as a mixture of isomers.

### Physicochemical properties

Nuclear magnetic resonance spectrum (DMSO-d₆, TMS internal standard)
δ (ppm): 1.9 (1 H, m), 2.3 (1 H, m), 2.54 (3 H, brs), 2.67 (1 H, m), 3.1 (1 H, m), 3.5 - 3.6 (3 H, m), 3.75 (3 H, s), 3.88 (1 H, brd), 4.34 and 4.59 (1 H), 5.1 - 5.3 (3 H, m), 5.7 (1 H, m), 6.1 (1 H, m), 6.6 - 6.7 (1 H, m), 6.94 (2 H, m), 7.2 - 7.4 (7 H, m), 9.15 (1 H, m)
Positive ion-FAB-mass spectrum: m/z; 550 (M + 1)⁺
Infrared absorption spectrum νₘₐₓ (KBr): 3296, 1780, 1720, 1670, 1618, 1590, 1536, 1520, 1390, 828, 698 cm⁻¹
(2) In an atmosphere of argon, 625 mg (3 mmol) of phosphorus pentachloride was dissolved in 10 ml of chloroform, and 243 µl (3 mmol) of pyridine was added thereto at 5°C, followed by 1 hour of stirring. The thus obtained reaction solution was mixed with 550 mg (1 mmol) of p-methoxybenzyl 7β-(2-phenylacetamido)-3-[(E)-2-(2-methyl-5-isooxazolidinyl)vinyl]-3-cephem-4-carboxylate prepared in the above step (1) and the mixture was stirred at 8°C for 1.5 hours. The reaction solution was cooled to -30°C and mixed with 4.1 ml of methanol, and the mixture was stirred at -15°C for 1.5 hours.

After the reaction, 10 ml of chloroform and 20 ml of saturated sodium chloride aqueous solution were added thereto. The water layer was separated from the reaction solution, the remaining organic layer was extracted with water, the resulting water layers were combined and neutralized with saturated sodium bicarbonate aqueous solution while cooling in an ice bath and then the thus obtained aqueous solution was extracted with chloroform. The chloroform extract was dried over anhydrous magnesium sulfate and then the solvent was evaporated under a reduced pressure to obtain p-methoxybenzyl 7β-amino-3-[(E)-2-(2-methyl-5-isooxazolidinyl)vinyl]-3-cephem-4-carboxylate.

### Physicochemical properties

Nuclear magnetic resonance spectrum (DMSO-d₆, TMS internal standard)
δ (ppm): 3.75 (3 H, s), 4.79 (1 H, d, J = 4.9 Hz), 5.01 (1 H, m), 6.04 (1 H, brs), 6.63 (1 H, m), 6.93 (2 H, m), 7.37 (2 H, m)
Positive ion-FAB-mass spectrum: m/z; 432 (M + 1)⁺
Infrared absorption spectrum νₘₐₓ (KBr): 2848, 1780, 1726, 1618, 1394, 1358, 1304, 1248, 1176 cm⁻¹

### Example 10

(1) In an atmosphere of argon, 208 mg (0.26 mmol) of p-methoxybenzyl 7β-[(Z)-2-(2-tritylamino-4-thiazolyl)-2-(methoxyimino)acetamido]-3-[(E)-1,3-butadienyl]-3-cephem-4-carboxylate obtained in Example 1 was dissolved in 2.6 ml of benzene, and the resulting solution was mixed with diethylazodicarboxylate (32 µl) and the mixture was heated for 10.5 hours under reflux.

After completion of the reaction, the solvent was evaporated under a reduced pressure. The resulting residue was subjected to silica gel column chromatography and elution was carried out with methylene chloride-ethyl acetate (500:40, v/v). Fractions containing the compound of interest were pooled, the solvent was evaporated under a reduced pressure, and then the resulting foamy product was solidified with ethyl ether-hexane to obtain 87 mg of p-methoxybenzyl 7β-[(Z)-2-(2-tritylamino-4-thiazolyl)-2-(methoxyimino)acetamido]-3-[1,2-bis(ethoxycarbonyl)-1,2,3,6-tetrahydro-3-pyridazinyl]-3-cephem-4-carboxylate.

### Physicochemical properties

Nuclear magnetic resonance spectrum (DMSO-d₆, TMS internal standard)
δ (ppm): 3.75 (3 H, s), 3.79 (3 H, s)
Positive ion-FAB-mass spectrum: m/z; 972 (M + 1)⁺
Infrared absorption spectrum νₘₐₓ (KBr): 3320, 1792, 1728, 1618, 1520, 1218, 702 cm⁻¹
(2) In an atmosphere of argon, 160 mg of p-methoxybenzyl 7β-[(Z)-2-(2-tritylamino-4-thiazolyl)-2-(methoxyimino)acetamido]-3-[1,2-bis(ethoxycarbonyl)-1,2,3,6-tetrahydro-3-pyridazinyl]-3-cephem-4-carboxylate obtained in the above step (1) was dissolved in methylene chloride (3 ml) and anisole (2 ml). After cooling to 10°C, trifluoroacetic acid (5 ml) was added dropwise thereto. After 60 minutes of stirring at 10°C to room temperature, the solvent and trifluoroacetic acid were evaporated under a reduced pressure, and the resulting residue was solidified by adding ethyl ether-hexane and collected by filtration. This was added to trifluoroacetic acid (10 ml) which has been cooled to 10°C, and water (5 ml) was added dropwise thereto.

After 60 minutes of stirring at room temperature, trifluoroacetic acid was evaporated under a reduced pressure, and the resulting residue was mixed with ethyl alcohol and subjected to azeotropic distillation to remove water. The thus obtained residue was solidified by adding ethyl ether and collected by filtration. The thus collected powder was dissolved in saturated sodium bicarbonate aqueous solution and purified by HP-20 column chromatography to obtain sodium 7β-[(Z)-2-(2-amino-4-thiazolyl)-2-(methoxyimino)acetamido]-3-[1,2-bis(ethoxycarbonyl)-1,2,3,6-tetrahydro-3-pyridazinyl]-3-cephem-4-carboxylate (71 mg).

### Physicochemical properties

Nuclear magnetic resonance spectrum (DMSO-d₆, TMS internal standard)
δ (ppm): 3.82 (3 H, s), 7.20 (2 H, brs)
Positive ion-FAB-mass spectrum: m/z; 608 (M-Na)⁺
Infrared absorption spectrum νₘₐₓ (KBr): 1772, 1694, 1618, 1540, 1386, 1344, 1220, 1076, 1040 cm⁻¹

### Example 11

(1) In an atmosphere of argon, 160 mg (0.2 mmol) of p-methoxybenzyl 7β-[(Z)-2-(2-tritylamino-4-thiazolyl)-2-(methoxyimino)acetamido]-3-[(E)-1,3-butadienyl]-3-cephem-4- carboxylate obtained in Example 1 was dissolved in methylene chloride (1 ml). To the resulting solution cooled in an ice bath was added 4-methyl-1,2,4-triazol-3,5-dione (15 mg, 0.13 mmol).

After 30 minutes of stirring in an ice bath, the reaction solution was subjected to thin layer separation chromatography and elution was carried out with methylene chloride-ethyl acetate (100:8, v/v). Fractions containing the compound of interest were pooled, the solvent was evaporated under a reduced pressure, and then the resulting foamy product was solidified by adding diisopropyl ether to obtain 90 mg of p-methoxybenzyl 7β-[(Z)-2-(2-tritylamino-4-thiazolyl)-2-(methoxyimino)acetamido]-3-[1,3-dioxo-2-methyl-2,3,5,8-tetrahydro-1H-[1,2,4]triazolo[1,2-a]pyridazin-5-yl]-3-cephem-4-carboxylate.

### Physicochemical properties

Nuclear magnetic resonance spectrum (DMSO-d₆, TMS internal standard)
δ (ppm): 2.90 (3 H, s), 3.3 - 3.4 (2 H, m), 3.75 (3 H, s), 3.79 (3 H, s), 4.01 (1 H, brd, J = 14.6 Hz), 4.20 (1 H, brd, J = 15.9 Hz), 5.14 (1 H, d, J = 4.9 Hz), 5.21 (2 H, dd, J = 26.9, 12.2 Hz), 5.58 (1 H, brs), 5.7 (2 H, m), 6.19 (1 H, brd, J = 10.4 Hz), 6.70 (1 H, s), 6.93 (2 H, d, J = 8.5 Hz), 7.2 - 7.4 (17 H, m), 8.82 (1 H, s), 9.51 (1 H, d, J = 7.9 Hz)
Positive ion-FAB-mass spectrum: m/z; 911 (M + 1)⁺
Infrared absorption spectrum νₘₐₓ (KBr): 1780, 1712, 1520, 1472, 1250, 1220, 1178, 1036, 702 cm⁻¹
(2) In an atmosphere of argon, 160 mg (0.18 mmol) of p-methoxybenzyl 7β-[(Z)-2-(2-tritylamino-4-thiazolyl)-2-(methoxyimino)acetamido]-3-[1,3-dioxo-2-methyl-2,3,5,8-tetrahydro-1H-[1,2,4]triazolo[1,2-a]pyridazin-5-yl]-3-cephem-4-carboxylate obtained in the above step (1) was dissolved in methylene chloride (3 ml) and anisole (2 ml). To the resulting solution cooled at 10°C was added dropwise trifluoroacetic acid (5 ml). After 60 minutes of stirring at 10°C to room temperature, the solvent and trifluoroacetic acid were evaporated under a reduced pressure, and the thus obtained residue was solidified by adding ethyl ether-hexane and collected by filtration.

This was added to trifluoroacetic acid (10 ml) cooled at 10°C, and water (5 ml) was added dropwise to the resulting mixture. After 60 minutes of stirring at room temperature, trifluoroacetic acid was evaporated under a reduced pressure, and the resulting residue was mixed with ethyl alcohol and subjected to azeotropic distillation to remove water. The resulting residue was solidified by adding ethyl ether and collected by filtration. The thus obtained powder was dissolved in saturated sodium bicarbonate aqueous solution and purified using an HP-20 column to obtain sodium 7β-[(Z)-2-(2-amino-4-thiazolyl)-2-(methoxyimino)acetamido]-3-[1,3-dioxo-2-methyl-2,3,5,8-tetrahydro-1H-[1,2,4]triazolo[1.2-a)pyridazin-5-yl]-3-cephem-4-carboxylate (74 mg).

### Physicochemical properties

Nuclear magnetic resonance spectrum (DMSO-d₆, TMS internal standard)
δ (ppm): 2.92 (3 H, s), 3.08 (1 H, d, J = 17.1 Hz), 3.17 (1 H, d, J = 17.1 Hz), 3.82 (3 H, s), 3.94 (1 H, d, J = 14 Hz), 4.18 (1 H, d, J = 14 Hz), 4.95 (1 H, d, J = 4.9 Hz), 5.57 (1 H, m), 5.89 (1 H, m), 6.0 (2 H, m), 6.72 (1 H, s), 7.20 (2 H, s), 9.45 (1 H, d, J = 7.9 Hz)
Positive ion-FAB-mass spectrum: m/z; 547 (M-Na)⁺
Infrared absorption spectrum νₘₐₓ (KBr): 1764, 1698, 1614, 1540, 1488, 1398, 1362, 1284, 1038 cm⁻¹

### Example 12

(1) A 5.59 g (7 mmol) portion of p-methoxybenzyl 7β-[(Z)-2-(2-tritylamino-4-thiazolyl)-2-(2-methoxyimino)acetamido]-3-[(E)-1,3-butadienyl]-3-cephem-4-carboxylate, 861 mg (10.5 mmol) of sodium acetate and 1.1 ml (14 mmol) of 38% aqueous formalin were dissolved in 112 ml of tetrahydrofuran. Then, 878 mg (10.5 mmol) of N-methylhydroxyamine hydrochloride that has been dissolved in 18 ml of 80% aqueous ethanol was added dropwise thereto, followed by 2.5 hours of heating under reflux.

Thereafter, the solvent was evaporated under a reduced pressure, the resulting residue was dissolved in methylene chloride, and the solution was washed with saturated sodium bicarbonate aqueous solution, saturated ammonium chloride aqueous solution, water and saturated sodium chloride aqueous solution and dried on anhydrous magnesium sulfate. Then, the solvent was evaporated under a reduced pressure. The resulting residue was subjected to silica gel column chromatography and elution was carried out with chloroform-methanol (100:1, v/v). Fractions containing the compound of interest were pooled and then the solvent was evaporated under a reduced pressure to obtain 5.97 g of p-methoxybenzyl 7β-[(Z)-2-(2-tritylamino-4-thiazolyl)-2-(methoxyimino)acetamido]-3-[(E)-2-(2-methyl-5-isooxazolidinyl)vinyl]-3-cephem-4-carboxylate as a mixture of isomers.

### Physicochemical properties

Nuclear magnetic resonance spectrum (DMSO-d₆, TMS internal standard)
δ (ppm): 1.8 - 2.0 (1 H, m), 2.3 - 2.8 (5 H, m), 3.14 (1 H, m), 3.56 (1 H, m), 3.75 (3 H, s), 3.81 (3 H, s), 3.87 (1 H, m), 4.2 - 4.7 (1 H, m), 5.1 - 5.2 (3 H, m), 5.7 (1 H, m), 6.1 (1 H, m), 6.7 (1 H, m), 6.72 (1 H, s), 6.9 (2 H, m), 7.2 - 7.4 (17 H, m), 8.84 (1 H, s), 9.59 (1 H, m)
Positive ion-FAB-mass spectrum: m/z; 857 (M + 1)⁺
Infrared absorption spectrum νₘₐₓ (KBr): 3352, 2944, 2860, 1784, 1724, 1632, 1580, 1518, 1246 cm⁻¹

### Alternative method

A 310 mg (0.7 mmol) portion of (Z)-2-(2-tritylamino-4-thiazolyl)-2-(methoxyimino)acetic acid was dissolved in 9 ml of methylene chloride-N,N-dimethylformamide mixed solution (2:1, v/v). Then, 95 mg (0.7 mmol) of 1-hydroxybenzotriazole and 144 mg (0.7 mmol) of 1,3-dicyclohexylcarbodiimide were added thereto, and the mixture was stirred for 1.5 hours at room temperature to obtain an ester compound. With cooling in an ice bath, this was added to 9 ml of methylene chloride-N,N-dimethylformamide mixed solution (2:1, v/v) in which 280 mg of p-methoxybenzyl 7β-amino-3-[(E)-2-(2-methyl-5-isooxazolidinyl)vinyl]-3-cephem-4-carboxylate obtained in Example 9 has been dissolved, and the resulting mixture was stirred for 15 hours at ice cold temperature to room temperature. After completion of the reaction, the solvent was evaporated under a reduced pressure, and the thus obtained residue was mixed with 50 ml of chloroform and washed with water and saturated sodium chloride aqueous solution in that order.

The resulting organic layer was dried over anhydrous magnesium sulfate, the solvent was evaporated under a reduced pressure, the thus obtained residue was subjected to silica gel column chromatography and elution was carried out with chloroform-methanol (100:0 to 100:3, v/v). Fractions containing the compound of interest were pooled and then the solvent was evaporated under a reduced pressure to obtain p-methoxybenzyl 7β-[(Z)-2-(2-tritylamino-4-thiazolyl)-2-(methoxyimino)acetamido]-3-[(E)-2-(2-methyl-5-isooxazolidinyl)vinyl]-3-cephem-4-carboxylate.
(2) In an atmosphere of argon, 170 mg of p-methoxybenzyl 7β-[(Z)-2-(2-tritylamino-4-thiazolyl)-2-(methoxyimino)acetamido]-3-[(E)-2-(2-methyl-5-isooxazolidinyl)vinyl]-3-cephem-4-carboxylate obtained in the above step (1) was dissolved in 3 ml of methylene chloride and 2 ml of anisole, the solution was cooled to 10°C and mixed with 5 ml of trifluoroacetic acid. Then, the mixture was stirred for 60 minutes at 10°C to room temperature. This was again cooled to 10°C, mixed with 5 ml of water and the mixture was stirred for 60 minutes.

Thereafter, the solvent was evaporated under a reduced pressure, the resulting residue was mixed with ethanol and subjected to azeotropic distillation to remove water, and then the thus obtained ethyl ether solution was mixed with diisopropyl ether to collect the precipitated insoluble substance by filtration. The thus obtained powder was purified by high performance liquid separation chromatography to obtain two isomers (isomer 12A and isomer 12B) of 7β-[(Z)-2-(2-amino-4-thiazolyl)-2-(methoxyimino)acetamido]-3-[(E)-2-(2-methyl-5-isooxazolidinyl)vinyl]-3-cephem-4-carboxylic acid.

### Physicochemical properties (isomer 12A)

Nuclear magnetic resonance spectrum (DMSO-d₆, TMS internal standard)
δ (ppm): 1.9 (1 H, m), 2.3 - 2.8 (4 H, m), 3.84 (3 H, s), 4.33 and 4.62 (1 H), 5.09 (1 H, d, J = 4.6 Hz), 5.6 (1 H, m), 5.75 (1 H, m), 6.74 (1 H, s), 6.84 (1 H, d, J = 15.9 Hz), 7.19 (2 H, brd), 9.56 (1 H, d, J = 8.2 Hz)
Positive ion-FAB-mass spectrum: m/z; 495 (M + 1)⁺
Infrared absorption spectrum νₘₐₓ (KBr): 1770, 1676, 1608, 1538, 1388, 1038 cm⁻¹

### Physicochemical properties (isomer 12B)

Nuclear magnetic resonance spectrum (DMSO-d₆, TMS internal standard)
δ (ppm): 1.9 (1 H, m), 2.3 - 2.8 (4 H, m), 3.84 (3 H, s), 4.32 and 4.59 (1 H), 5.03 (1 H, brd), 5.6 (3 H, m), 6.74 (1 H, s), 6.87 (1 H, d, J = 15.9 Hz), 7.19 (2 H, brs), 9.55 (1 H, d, J = 7.9 Hz)
Positive ion-FAB-mass spectrum: m/z; 495 (M + 1)⁺
Infrared absorption spectrum νₘₐₓ (KBr): 1768, 1674, 1612, 1542, 1388, 1038 cm⁻¹

### Example 13

A 5.84 g portion of p-methoxybenzyl 7β-[(Z)-2-(2-tritylamino-4-thiazolyl)-2-(methoxyimino)acetamido]-3-[(E)-2-(2-methyl-5-isooxazolidinyl)vinyl]-3-cephem-4-carboxylate obtained in Example 12 (1) was dissolved in 117 ml of N,N-dimethylformamide, and the resulting solution was mixed with 2.12 ml of methyl iodide, and the mixture was stirred for a whole day and night at room temperature.

Thereafter, the solvent and excess methyl iodide were evaporated under a reduced pressure and the resulting foamy product was solidified with diisopropyl ether to obtain 5.83 of p-methoxybenzyl 7β-[(Z)-2-(2-tritylamino-4-thiazolyl)-2-(methoxyimino)acetamido]-3-[(E)-2-(2,2-dimethyl-5-isooxazolidinio)vinyl]-3-cephem-4-carboxylate·iodide as a mixture of isomers.

### Physicochemical properties

Nuclear magnetic resonance spectrum (DMSO-d₆, TMS internal standard)
δ (ppm): 2.56 (1 H, m), 2.82 (1 H, m), 4.1 (1 H, m), 4.2 (1 H, m), 5.1 - 5.3 (3 H, m), 5.7 (1 H, m), 6.2 (1 H, m), 6.7 (1 H, s), 6.85 (1 H, d, J = 16.1 Hz), 6.94 (2 H, m), 7.2 - 7.4 (17 H, m), 8.85 (1 H, brs), 9.59 (1 H, d, J = 7.8 Hz)
Positive ion-FAB-mass spectrum: m/z; 871 (M - 1)⁺
Infrared absorption spectrum νₘₐₓ (KBr): 1786, 1724, 1670, 1520, 1390, 1248, 1222, 1036, 704 cm⁻¹
(2) In an atmosphere of argon, 5.72 g of p-methoxybenzyl 7β-[(Z)-2-(2-tritylamino-4-thiazolyl)-2-(methoxyimino)acetamido]-3-[(E)-2-(2,2-dimethyl-5-isooxazolidinio)vinyl]-3-cephem-4-carboxylate·iodide obtained in the above step (1) was dissolved in 30 ml of methylene chloride and 20 ml of anisole, the solution was cooled to 4°C and mixed with 50 ml of trifluoroacetic acid, and then the mixture was stirred for 60 minutes at 4°C to room temperature. This was again cooled to 4°C, mixed with 50 ml of trifluoroacetic acid and 50 ml of water, and the mixture was stirred for 60 minutes.

Thereafter, the solvent was evaporated under a reduced pressure, the resulting residue was mixed with ethanol and subjected to azeotropic distillation to remove water. Then, the thus obtained ethanol solution was mixed with diisopropyl ether to collect the precipitated insoluble substance by filtration. The thus obtained powder was purified by HP-20 column chromatography and subsequent high performance liquid separation chromatography to obtain two isomers (isomer 13A and isomer 13B) of 7β-[(Z)-2-(2-amino-4-thiazolyl)-2-(methoxyimino)acetamido]-3-[(E)-2-(2,2-dimethyl-5-isooxazolidinio)vinyl]-3-cephem-4-carboxylate.

### Physicochemical properties (isomer 13A)

Nuclear magnetic resonance spectrum (DMSO-d₆, TMS internal standard)
δ (ppm): 2.5 (1 H, m), 2.7 (1 H, m), 3.4 (2 H, brs), 3.52 (3 H, s), 3.55 (3 H, s), 3.84 (3 H, s), 4.0 - 4.1 (1 H, m), 4.1 - 4.2 (1 H, m), 5.02 (1 H, d, J = 4.9 Hz), 5.19 (1 H, m), 5.5 - 5.6 (2 H, m), 6.74 (1 H, s), 7.10 (1 H, d, J = 15.6 Hz), 7.21 (2 H, brs), 9.55 (1 H, d, J = 7.8 Hz)
Positive ion-FAB-mass spectrum: m/z; 509 (M + 1)⁺
Infrared absorption spectrum νₘₐₓ (KBr): 1770, 1668, 1610, 1540, 1390, 1356, 1038 cm⁻¹

### Physicochemical properties (isomer 13B)

Nuclear magnetic resonance spectrum (DMSO-d₆, TMS internal standard)
δ (ppm): 2.5 (1 H, m), 2.7 (1 H, m), 3.51 (3 H, s), 3.54 (3 H, s), 3.84 (3 H, s), 4.0 - 4.1 (1 H, m), 4.1 - 4.2 (1 H, m), 5.03 (1 H, d, J = 4.9 Hz), 5.18 (1 H, m), 5.5 - 5.6 (2 H, m), 6.74 (1 H, s)

### Example 14

(1) A 800 mg (1 mmol) portion of the compound obtained in Example 3 was dissolved in 16 ml of tetrahydrofuran, and the resulting solution was mixed with 123 mg (1.5 mmol) of sodium acetate and 0.16 ml (2 mmol) of 38% aqueous formalin. To this solution, cooled in an ice bath, was added dropwise 125 mg (1.5 mmol) of N-methylhydroxylamine hydrochloride which has been dissolved in 80% aqueous ethanol. After 3.25 hours of heating under reflux, the solvent was evaporated under a reduced pressure, the resulting residue was subjected to silica gel column chromatography and elution was carried out with chloroform-methanol (100:1, v/v). Fractions containing the compound of interest were pooled and then the solvent was evaporated under a reduced pressure to obtain 774 mg of p-methoxybenzyl 7β-[(Z)-2-(5-tritylamino-1,2,4-thiadiazol-3-yl)-2-methoxyiminoacetamido]-3-[(E)-2-(2-methyl-5-isooxazolidinyl)vinyl]-3-cephem-4-carboxylate.

### Physicochemical properties

Nuclear magnetic resonance spectrum (DMSO-d₆, TMS internal standard)
δ (ppm): 1.8 - 2.0 (1 H, m), 2.3 - 2.7 (2 H, m), 2.5 (3 H, m), 3.14 (2 H, m), 3.31 (3 H, s), 3.52 (1 H, ABq), 3.86 (1 H, ABq), 3.75 (3 H, s), 3.90 (3 H, s), 4.2 - 4.6 (1 H, m), 5.1 - 5.2 (3 H, m), 5.7 (1 H, m), 6.1 (1 H, m), 6.64 (1 H, d, J = 15 Hz), 6.93 (2 H, ABq, J = 8 Hz), 7.2 - 7.4 (17 H, m), 9.57 (1 H, m), 9.90 (1 H, brs)
Positive ion-FAB-mass spectrum: m/z; 858 (M + H)⁺
Infrared absorption spectrum νₘₐₓ (KBr): 2952, 1784, 1724, 1688, 1520, 1390, 1220, 1042, 752, 702 cm⁻¹
(2) A 766 mg (0.89 mmol) portion of the compound obtained in the above step (1) was dissolved in 4 ml of DMF, and the resulting solution was mixed with 277 µl of methyl iodide (4.5 mmol) and the mixture was stirred overnight at room temperature. Thereafter, the solvent was evaporated under a reduced pressure, and the resulting residue was solidified by adding ethyl ether, collected by filtration and then dried to obtain 827 mg (93%) of p-methoxybenzyl 7β-[(Z)-2-(2-tritylamino-1,2,4-thiadiazol-3-yl)-2-methoxyiminoacetamido]-3-[(E)-2-(2,2-dimethyl-5-isooxazolidinio)vinyl]-3-cephem-4-carboxylate·iodide.
Nuclear magnetic resonance spectrum (DMSO-d₆, TMS internal standard)
δ (ppm): 2.5 - 2.9 (2 H, m), 3.4 - 3.6 (9 H, m), 3.75 (3 H, s), 3.8 (2 H, ABq), 3.90 (3 H, s), 4.0 - 4.2 (2 H, m), 5.1 - 5.2 (3 H, m), 5.8 (1 H, m), 6.2 (1 H, m), 6.83 (1 H, d, J = 16 Hz), 6.94 (2 H, ABq, J = 8 Hz), 7.2 - 7.5 (17 H, m), 9.56 (1 H, d, J = 8 Hz), 10.0 (1 H, s)
Positive ion-FAB-mass spectrum: m/z; 872 (M-I)⁺
Infrared absorption spectrum νₘₐₓ (KBr): 3456, 2952, 1784, 1724, 1670, 1520, 1392, 1039 cm⁻¹
(3) A 785 mg (0.79 mmol) portion of the compound obtained in the above step (2) was dissolved in 9 ml of methylene chloride and 2 ml of anisole, the solution was cooled to 10°C and mixed with 15 ml of trifluoroacetic acid. Then, the mixture was stirred for 1.5 hours at 10°C to room temperature. With cooling in an ice bath, this was mixed with 5 ml of water and stirred for 1 hour. The reaction solution was mixed with 50 ml of n-hexane to separate the water layer. The remaining organic layer was extracted twice with water, and the water layers were combined with the above water layer. The thus combined water layer was concentrated under a reduced pressure, the resulting residue was applied to an HP-20 column. Elution was carried out with water-acetonitrile and fractions containing the compound of interest were pooled. The pooled fraction was concentrated under a reduced pressure and then freeze-dried to obtain 270 mg (68%) of 7β-[(Z)-2-(5-amino-1,2,4-thiadiazol-3-yl)-2-methoxyiminoacetamido]-3-[(E)-2-(2,2-dimethyl-5-isooxazolidinio)vinyl]-3-cephem-4-carboxylate. Since this product was a 1:1 mixture of the 5-position isooxazole-based isomers (isomers 14A and 14B), each isomer was purified by a high performance liquid chromatography.

### Physicochemical properties (isomer 14A)

Nuclear magnetic resonance spectrum (DMSO-d₆, TMS internal standard)
δ (ppm): 2.5 - 2.7 (1 H, m), 3.3 - 3.5 (2 H, m), 3.52 (3 H, m), 3.55 (3 H, s), 3.91 (3 H, s), 4.07 - 4.16 (2 H, m), 5.01 (1 H, d, J = 5 Hz), 5.18 (1 H, m), 5.54 - 5.62 (2 H, m), 7.10 (1 H, d, J = 16 Hz), 8.14 (1 H, s), 9.51 (1 H, d, J = 8.5 Hz)
Positive ion-FAB-mass spectrum: m/z; 510 (M + H)⁺
Infrared absorption spectrum νₘₐₓ (KBr): 3428, 1770, 1670, 1608, 1532, 1394, 1354, 1042 cm⁻¹

### Physicochemical properties (isomer 14B)

Nuclear magnetic resonance spectrum (DMSO-d₆, TMS internal standard)
δ (ppm): 2.5 - 2.7 (2 H, m), 3.4 - 3.5 (2 H, m), 3.51 (3 H, s), 3.55 (1 H, s), 3.91 (3 H, s), 4.07 - 4.19 (2 H, m), 5.04 (1 H, d, J = 5 Hz), 5.17 (1 H, m), 5.5 - 5.6 (2 H, m), 7.10 (1 H, d, J = 16 Hz), 8.13 (1 H, s), 9.51 (1 H, d, J = 8.5 Hz)
Positive ion-FAB-mass spectrum: m/z; 510 (M + H)⁺
Infrared absorption spectrum νₘₐₓ (KBr): 3424, 1770, 1670, 1610, 1530, 1464, 1356, 1040 cm⁻¹

### Example 15

A 600 mg (0.70 mmol) portion of the compound obtained in Example 14 (1) was deprotected in the same manner as described in Example 14 and purified by HP-20 column chromatography to obtain 269 mg (78%) of 7β-[(Z)-2-(5-amino-1,2,4-thiadiazol-3-yl)-2-methoxyiminoacetamido]-3-[(E)-2-(2-methyl-5-isooxazolidinyl)vinyl]-3-cephem-4-carboxylic acid. Since this product was a 1:1 mixture of the 5-position isooxazolidine-based isomers (isomers 15A and 15B), each isomer was purified by a high performance liquid chromatography.

### Physicochemical properties (isomer 15A)

Nuclear magnetic resonance spectrum (DMSO-d₆, TMS internal standard)
δ (ppm): 1.9 - 2.0 (1 H, m), 2.3 - 2.7 (6 H, m), 3.52 (1 H, ABq, J = 17 Hz), 3.72 (1 H, ABq, J = 17 Hz), 3.91 (3 H, s), 4.36 and 4.63 (1 H, m), 5.11 (1 H, d, J = 5 Hz), 5.72 (2 H, q, J = 8.5 Hz), 5.9 (1 H, m), 6.82 (1 H, d, J = 15 Hz), 8.13 (2 H, brs), 9.56 (1 H, d, J = 8 Hz)
Positive ion-FAB-mass spectrum: m/z; 496 (M + H)⁺
Infrared absorption spectrum νₘₐₓ (KBr): 3432, 1772, 1678, 1604, 1530, 1398, 1042 cm⁻¹

### Physicochemical properties (isomer 15B)

Nuclear magnetic resonance spectrum (DMSO-d₆, TMS internal standard)
δ (ppm): 1.8 - 2.0 (1 H, m), 2.3 - 2.7 (6 H, m), 3.50 (1 H, ABq, J = 16 Hz), 3.68 (1 H, ABq, J = 16 Hz), 4.37 and 4.63 (1 H, m), 5.09 (1 H, d, J = 5 Hz), 5.72 (2 H, q, J = 9.5 Hz), 5.86 (1 H, m), 6.82 (1 H, d, J = 16 Hz), 8.13 (2 H, brs), 9.56 (1 H, d, J = 9 Hz)
Positive ion-FAB-mass spectrum: m/z; 496 (M + H)⁺
Infrared absorption spectrum νₘₐₓ (KBr): 3432, 1772, 1678, 1530, 1396, 1042 cm⁻¹

### Example 16

(1) A 1.2 g (1.5 mmol) portion of p-methoxybenzyl 7β-[(Z)-2-(2-tritylamino-4-thiazolyl)-2-(methoxyimino)acetamido]-3-[(E)-1,3-butadienyl]-3-cephem-4-carboxylate, 308 mg (1.5 mmol) of dibromoformaldoxime and 638 mg (7.5 mmol) of sodium bicarbonate were suspended in 20 ml of methylene chloride, and the suspension was stirred for 5 hours at room temperature. Thereafter, this was mixed with saturated ammonium chloride aqueous solution and washed twice with saturated ammonium chloride aqueous solution. After drying the thus obtained organic layer over anhydrous magnesium sulfate, the solvent was evaporated under a reduced pressure, the resulting residue was subjected to silica gel column chromatography and elution was carried out with methylene chloride-ethyl acetate (10:1, v/v). Fractions containing the compound of interest were pooled and then the solvent was evaporated under a reduced pressure to obtain 1.04 g (76%) of p-methoxybenzyl 7β-[(Z)-2-(2-tritylamino-4-thiazolyl)-2-(methoxyimino)acetamido]-3-[(E)-2-(4H,5H-3-bromo-5-isooxazolinyl)vinyl]-3-cephem-4-carboxylate as a mixture of isomers.

### Physicochemical properties

Nuclear magnetic resonance spectrum (DMSO-d₆, TMS internal standard)
δ (ppm): 3.1 (1 H, m), 3.5 - 3.6 (2 H, m), 3.75 (3 H, s), 3.81 (3 H, s), 3.9 (1 H, m), 5.70 (1 H, dd, J = 7.8, 4.9 Hz), 6.17 (1 H, dd, J = 15.6, 7.3 Hz), 6.71 (1 H; s), 6.77 (1 H, d, 15.6 Hz), 6.93 (2 H, m), 7.2 - 7.4 (17 H, m), 8.84 (1 H, brs), 9.6 (1 H, m)
Positive ion-FAB-mass spectrum: m/z; 920 (M + 1)⁺
Infrared absorption spectrum νₘₐₓ (KBr): 3320, 1788, 1728, 1688, 1518, 1250, 1220, 1038, 702 cm⁻¹
(2) In an atmosphere of argon, 200 mg of p-methoxybenzyl 7β-[(Z)-2-(2-tritylamino-4-thiazolyl)-2-(methoxyimino)acetamido]-3-[(E)-2-(4H,5H-3-bromo-5-isooxazolinyl)vinyl]-3-cephem-4-carboxylate was dissolved in 2 ml of methylene chloride and 1 ml of anisole. To the resulting solution was added dropwise 5 ml of trifluoroacetic acid at 10°C to room temperature. After 60 minutes of stirring at room temperature, the solvent and trifluoroacetic acid were evaporated under a reduced pressure, and the thus obtained residue was solidified by adding ethyl ether-hexane and collected by filtration. This was added to 5 ml of trifluoroacetic acid cooled at 10°C, followed by dropwise addition of 2.5 ml of water at room temperature or lower temperature. After 60 minutes of stirring at room temperature, trifluoroacetic acid was evaporated under a reduced pressure, and the resulting residue was mixed with 10 ml of ethyl alcohol and subjected to azeotropic distillation to remove water. The residue was solidified by adding 10 ml of ethyl ether and collected by filtration. The thus obtained powder was dissolved in saturated sodium bicarbonate aqueous solution and purified by HP-20 column chromatography to obtain 56 mg of sodium 7β-[(Z)-2-(2-tritylamino-4-thiazolyl)-2-(methoxyimino)acetamido]-3-[(E)-2-(4H,5H-3-bromo-5-isooxazolinyl)vinyl]-3-cephem-4-carboxylate as a mixture of isomers.

### Physicochemical properties

Nuclear magnetic resonance spectrum (DMSO-d₆)
δ (ppm): 3.0 - 3.1 (1 H, m), 3.84 (3 H, s), 5.59 (1 H, dd, J = 7.3, 4.4 Hz), 7.23 (2 H, brs), 9.57 (1 H, m)
Positive ion-FAB-mass spectrum: m/z; 581 (M + 2)⁺
Infrared absorption spectrum νₘₐₓ (KBr): 3448, 1766, 1670, 1612, 1538, 1386, 1040 cm⁻¹

### Example 17

(1) A 485 mg (0.61 mmol) portion of p-methoxybenzyl 7β-[(Z)-2-(2-tritylamino-4-thiazolyl)-2-(methoxyimino)acetamido]-3-[(E)-1,3-butadienyl]-3-cephem-4-carboxylate and 236 mg (1.22 mmol) of 4-pyridylhydroxamoyl chloride hydrochloride were suspended in 5 ml of dioxane. Then, 2 ml of dioxane solution containing 338 µl (2.44 mmol) of triethylamine was added dropwise thereto at room temperature spending 15 minutes. After completion of the dropwise addition and subsequent 1 hour of stirring at room temperature, insoluble materials were removed by filtration, and the resulting filtrate was concentrated. The thus obtained residue was subjected to silica gel column chromatography and elution was carried out with chloroform. Fractions containing the compound of interest were pooled and then the solvent was evaporated under a reduced pressure to obtain 275 mg (49%) of p-methoxybenzyl 7β-[(Z)-2-(2-tritylamino-4-thiazolyl)-2-(methoxyimino)acetamido]-3-[(E)-2- (4H,5H-3-(4-pyridinyl)-5-isooxazolinyl)vinyl]-3-cephem-4-carboxylate as a mixture of isomers.

### Physicochemical properties

Nuclear magnetic resonance spectrum (DMSO-d₆)
δ (ppm): 3.1 - 3.3 (2 H, m), 3.5 - 3.7 (3 H, m), 3.74 (3 H, s), 3.81 (3 H, s), 5.3 (1 H, m), 5.7 (1 H, m), 6.2 - 6.3 (1 H, m), 7.6 (2 H, m), 8.68 (2 H, m), 8.84 (1 H, brs), 9.6 (1 H, m)
Positive ion-FAB-mass spectrum: m/z; 918 (M + 1)⁺
Infrared absorption spectrum νₘₐₓ (KBr): 1786, 1724, 1686, 1600, 1520, 1218, 1038, 754, 702 cm⁻¹
(2) In an atmosphere of argon, 92 mg of p-methoxybenzyl 7β-[(Z)-2-(2-tritylamino-4-thiazolyl)-2-(methoxyimino)acetamido]-3-[(E)-2-(4H,5H-3-(4-pyridinyl)-5-isooxazolinyl)vinyl]-3-cephem-4-carboxylate was dissolved in 2 ml of methylene chloride and 1 ml of anisole. To the resulting solution was added dropwise 5 ml of trifluoroacetic acid at 10°C to room temperature. After 60 minutes of stirring at room temperature, the solvent and trifluoroacetic acid were evaporated under a reduced pressure, and the thus obtained residue was solidified by adding ethyl ether-hexane and collected by filtration. This was added to 5 ml of trifluoroacetic acid cooled at 10°C, followed by dropwise addition of 2.5 ml of water at room temperature or lower temperature. After 60 minutes of stirring at room temperature, trifluoroacetic acid was evaporated under a reduced pressure, and the resulting residue was mixed with 10 ml of ethyl alcohol and subjected to azeotropic distillation to remove water. The thus obtained residue was solidified by adding 10 ml of ethyl ether and collected by filtration. The thus obtained powder was dissolved in saturated sodium bicarbonate aqueous solution and purified by HP-20 column chromatography to obtain 21 mg of sodium 7β-[(Z)-2-(2-tritylamino-4-thiazolyl)-2-(methoxyimino)acetamido]-3-[(E)-2-(4H,5H-3-(4-pyridinyl)-5-isooxazolinyl)vinyl]-3-cephem-4-carboxylate as a mixture of isomers.

### Physicochemical properties

Nuclear magnetic resonance spectrum (DMSO-d₆)
δ (ppm): 5.2 (1 H, m), 7.2 (2 H, brs), 7.6 (2 H, m), 8.6 (2 H, m), 9.6 (1 H, m)
Positive ion-FAB-mass spectrum: m/z; 556 (M + 2)⁺, 278 (M + Na + 2)⁺, 600 (M + Na + Na + 2)⁺
Infrared absorption spectrum νₘₐₓ (KBr): 3432, 1766, 1672, 1608, 1540, 1386, 1040 cm⁻¹

### Example 18

(1) In an atmosphere of argon, 148 mg (0.16 mmol) of p-methoxybenzyl 7β-[(Z)-2-(2-tritylamino-4-thiazolyl)-2 (methoxyimino)acetamido]-3-[(E)-2-(4H,5H-3-(pyridinyl)-5-isooxazolinyl)vinyl]-3-cephem-4-carboxylate was dissolved in 2 ml of dimethylformamide, and the solution was mixed with methyl iodide and the mixture was stirred for 17 hours at room temperature. After completion of the reaction, the solvent was evaporated under a reduced pressure and the resulting foamy product was solidified by adding ethyl ether-hexane to obtain 166 mg of p-methoxybenzyl 7β-[(Z)-2-(2-tritylamino-4-thiazolyl)-2-(methoxyimino)acetamido]-3-[(E)-2-(4H,5H-3-(1-methyl-4-pyridinio)-5-isooxazolinyl)vinyl]-3-cephem-4-carboxylate·iodide as a mixture of isomers with almost quantitative yield.

### Physicochemical properties

Nuclear magnetic resonance spectrum (DMSO-d₆)
δ (ppm): 3.75 (3 H, s), 3.81 (3 H, s), 4.35 (3 H, s), 5.48 (1 H, m), 6.24 (1 H, m), 8.28 (2 H, dd, J = 14.6, 6.7 Hz), 8.84 (1 H, s), 9.02 (2 H, d, J = 6.7 Hz), 9.59 (1 H, m)
Positive ion-FAB-mass spectrum: m/z; 932 (M)⁺
Infrared absorption spectrum νₘₐₓ (KBr): 3432, 1784, 1684, 1520, 1250, 1220, 1176, 1036, 704 cm⁻¹
(2) In an atmosphere of argon, 154 mg of p-methoxybenzyl 7β-[(Z)-2-(2-tritylamino-4-thiazolyl)-2-(methoxyimino)acetamido]-3-[(E)-2-(4H,5H-3-(1-methyl-4-pyridinio)-5-isooxazolinyl)vinyl]-3-cephem-4-carboxylate·iodide was dissolved in 2 ml of methylene chloride and 1 ml of anisole. To the resulting solution was added dropwise 5 ml of trifluoroacetic acid at 10°C to room temperature. After 60 minutes of stirring at room temperature, the solvent and trifluoroacetic acid were evaporated under a reduced pressure, and the thus obtained residue was solidified by adding ethyl ether-hexane and collected by filtration. This was added to 5 ml of trifluoroacetic acid cooled at 10°C, followed by dropwise addition of 2.5 ml of water at room temperature or lower temperature. After 60 minutes of stirring at room temperature, trifluoroacetic acid was evaporated under a reduced pressure, and the resulting residue was mixed with 10 ml of ethyl alcohol and subjected to azeotropic distillation to remove water. The thus obtained residue was solidified by adding 10 ml of ethyl ether and collected by filtration. The thus obtained powder was dissolved in saturated sodium bicarbonate aqueous solution and purified by HP-20 column chromatography to obtain 53 mg of 7β-[(Z)-2-(2-tritylamino-4-thiazolyl)-2-(methoxyimino)acetamido]-3-[(E)-2-(4H,5H-3-(1-methyl-4-pyridinio)-5-isooxazolinyl)vinyl]-3-cephem-4-carboxylate as a mixture of isomers.

### Physicochemical properties

Nuclear magnetic resonance spectrum (DMSO-d₆)
δ (ppm): 3.83 (3 H, s), 4.35 (3 H, s), 5.03 (1 H, m), 5.3 - 5.5 (2 H, m), 5.58 (1 H, dd, J = 8.3, 4.9 Hz), 5.72 (1 H, m), 6.73, 6.79 (1 H, s), 7.0 - 7.1 (1 H, m), 7.24 (2 H, brs), 8.28 (2 H, m), 9.04 (2 H, m), 9.56 (1 H, m)
Positive ion-FAB-mass spectrum: m/z; 570 (M + 1)⁺
Infrared absorption spectrum νₘₐₓ (KBr): 3416, 1770, 1642, 1608, 1534, 1386, 1036, 932 cm⁻¹

### Example 19

(1) To 8 ml of tetrahydrofuran solution containing 400 mg of p-methoxybenzyl 7β-[(Z)-2-(2-tritylamino-4-thiazolyl)-2-(methoxyimino)acetamido]-3-[(E)-1,3-butadienyl]-3-cephem-4-carboxylate, 62 mg of anhydrous sodium acetate and 0.09 ml of 38% aqueous formalin was added dropwise 80% ethanol aqueous solution containing 127 mg of N-carbamoylmethylhydroxylamine hydrochloride, followed by 5 hours of heating under reflux. Thereafter, the solvent was evaporated and the resulting residue was dissolved in methylene chloride. This was washed with saturated sodium chloride aqueous solution and dried over anhydrous magnesium sulfate and then the solvent was evaporated. The thus obtained residue was subjected to silica gel column chromatography and elution was carried out with chloroform-methanol (20:1, v/v). Fractions containing the compound of interest were pooled and then the solvent was evaporated to obtain 280 mg of p-methoxybenzyl 7β-[(Z)-2-(2-tritylamino-4-thiazolyl)-2-(methoxyimino)acetamido]-3-[(E)-2-(2-carbamoylmethyl-5-isooxazolidinyl)vinyl]-3-cephem-4-carboxylate.

### Physicochemical properties

Nuclear magnetic resonance spectrum (DMSO-d₆, TMS internal standard)
δ (ppm): 1.8 - 2.0 (1 H, m), 2.3 - 2.5 (1 H, m), 2.8 - 3.4 (4 H, m), 3.56 (1 H, m), 3.75 (3 H, s), 3.81 (3 H, s), 3.85 (1 H, m), 4.52 (1 H, m), 5.1 - 5.3 (3 H, m), 5.7 (1 H, m), 6.1 (1 H, m), 6.67 (1 H, d, J = 16 Hz), 6.71 (1 H, s), 6.93 (2 H, d, J = 8.6 Hz), 7.2 - 7.4 (17 H, m), 8.83 (1 H, s), 9.58 (1 H, m)
Positive ion-FAB-mass spectrum: m/z; 900 (M + H)⁺
Infrared absorption spectrum νₘₐₓ (KBr): 3400, 2952, 1786, 1684, 1520, 1036, 704 cm⁻¹
(2) A 260 mg portion of the compound obtained in the above step (1) was dissolved in 6 ml of methylene chloride and 4 ml of anisole, the resulting solution cooled in an ice bath was mixed with 10 ml of trifluoroacetic acid and the mixture was stirred for 1 hour at 0°C to room temperature. After evaporating the solvent, the resulting residue was mixed with ethyl ether and the thus formed precipitate was collected by filtration. The thus obtained powder was suspended in 10 ml of water, 10 ml of trifluoroacetic acid was added to the suspension cooled in an ice bath and then the mixture was stirred for 1 hour at 0°C to room temperature. After evaporation of the solvent, ethyl ether was added to the residue and the thus formed precipitate was collected by filtration. The thus obtained powder was dissolved in saturated sodium bicarbonate aqueous solution and purified by HP-20 high performance liquid separation chromatography to obtain two isomers 19A (30 mg) and 19B (25 mg) of sodium salt of 7β-[(Z)-2-(2-amino-4-thiazolyl)-2-(methoxyimino)acetamido]-3-[(E)-2-(2-carbamoylmethyl-5-isooxazolidinyl)vinyl]-3-cephem-4-carboxylic acid.

### Physicochemical properties (isomer 19A)

Nuclear magnetic resonance spectrum (DMSO-d₆, TMS internal standard)
δ (ppm): 1.96 (1 H, m), 2.46 (1 H, m), 2.8 - 3.4 (4 H, m), 3.50 (1 H, d, J = 17.1 Hz), 3.66 (1 H, d, J = 17.1 Hz), 3.84 (3 H, s), 4.4 - 4.6 (1 H, m), 5.10 (1 H, d, J = 4.9 Hz), 5.66 (1 H, m), 5.7 - 5.9 (1 H, m), 6.6 (1 H, d, J = 16 Hz), 6.75 (1 H, s), 6.85 (2 H, d, J = 16.1 Hz), 7.12 (2 H, d, J = 14.2 Hz), 7.21 (1 H, m), 9.58 (1 H, d, J = 8.3 Hz)
Positive ion-FAB-mass spectrum: m/z; 538 (M + H)⁺
Infrared absorption spectrum νₘₐₓ (KBr): 3546, 1768, 1670, 1394, 1040 cm⁻¹

### Physicochemical properties (isomer 19B)

Nuclear magnetic resonance spectrum (DMSO-d₆, TMS internal standard)
δ (ppm): 1.94 (1 H, m), 2.3 - 2.6 (1 H, m), 2.8 - 3.4 (4 H, m), 3.52 (1 H, d, J = 17.1 Hz), 3.69 (1 H, d, J = 17.1 Hz), 3.84 (3 H, s), 4.58 (1 H, m), 5.11 (1 H, d, J = 4.4 Hz), 5.69 (1 H, m), 5.89 (1 H, m), 6.74 (1 H, s), 6.87 (1 H, d, J = 16 Hz), 7.12 (2 H, d, J = 15.1 Hz), 7.21 (1 H, m), 9.58 (1 H, d, J = 8.3 Hz)
Positive ion-FAB-mass spectrum: m/z; 538 (M + H)⁺
Infrared absorption spectrum νₘₐₓ (KBr): 3480, 1774, 1676, 1388, 1040 cm⁻¹

### Example 20

(1) A 400 mg portion of p-methoxybenzyl 7β-[(Z)-2-(2-tritylamino-4-thiazolyl)-2-(methoxyimino)acetamido]-3-[(E)-1,3-butadienyl]-3-cephem-4-carboxylate was dissolved in 5 ml of acetonitrile. Then, 213 mg of 4-(R,S)-(t-butoxycarbonylamino)-3-oxo-1-(methylene)-1,2-pyrazolidinium ylide, followed by 5 hours of heating under reflux. Thereafter, the solvent was evaporated, the resulting residue was subjected to silica gel column chromatography and elution was carried out with chloroform-methanol (20:1, v/v). Fractions containing the compound of interest were pooled and then the solvent was evaporated to obtain 215 mg of p-methoxybenzyl 3-[(E)-2-(1R or 1S, 6R or 6S)-6-(t-butoxycarbonylamino)-7-oxo-2,3,7,6-1H,5H-pyrazolo[1,2-a]pyrazol-1-yl)vinyl]-7β-[(Z)-2-(2-tritylamino-4-thiazolyl)-2-(methoxyimino)acetamido]-3-cephem-4-carboxylate.

### Physicochemical properties

Nuclear magnetic resonance spectrum (DMSO-d₆, TMS internal standard)
δ (ppm): 1.38 (9 H, s), 1.8 - 2.0 (1 H, m), 2.3 - 2.7 (3 H, m), 3.1 - 3.2 (1 H, m), 3.4 - 3.9 (9 H, m), 4.2 - 4.6 (2 H, m), 5.1 - 5.3 (3 H, m), 5.68 (1 H, m), 5.9 - 6.1 (1 H, m), 6.6 - 6.8 (2 H, m), 6.93 (2 H, d, J = 8.6 Hz), 7.1 - 7.6 (17 H, m), 8.83 (1 H, m), 9.59 (1 H, m)
Positive ion-FAB-mass spectrum: m/z; 1011 (M + H)⁺
Infrared absorption spectrum νₘₐₓ (KBr): 3424, 2988, 1786, 1696, 1520, 1166, 1038, 704 cm⁻¹
(2) Using 320 mg of the compound obtained in the above step (1), the procedure of Example 19 (2) was repeated to obtain isomers 20A (30 mg) and 20B (28 mg), as well as 12C (58 mg) which is a 36:64 mixture of isomers different from 20A and 20B, of 3-[(E)-2-(1R or 1S, 6R or 6S)-6-amino-7-oxo-2,3,7,6-1H,5H-pyrazolo[1,2-a]pyrazol-1-yl)vinyl]-7β-[(Z)-2-(2-amino-4-thiazolyl)-2-(methoxyimino)acetamido]-3-cephem-4-carboxylic acid.

### Physicochemical properties (isomer 20A)

Nuclear magnetic resonance spectrum (DMSO-d₆, TMS internal standard)
δ (ppm): 1.98 (1 H, m), 2.3 - 2.7 (3 H, m), 3.23 (1 H, m), 3.47 (1 H, d, J = 17.1 Hz), 3.60 (1 H, d, J = 17.1 Hz), 3.81 (1 H, m), 3.84 (3 H, s), 4.01 (1 H, m), 4.45 (1 H, m), 5.08 (1 H, d, J = 4.9 Hz), 5.6 - 5.8 (2 H, m), 6.74 (1 H, s), 6.90 (2 H, d, J = 8.6 Hz), 7 (1 H, d, J = 15.6 Hz), 7.22 (2 H, s), 9.58 (1 H, d, J = 7.9 Hz)
Positive ion-FAB-mass spectrum: m/z; 549 (M + H)⁺
Infrared absorption spectrum νₘₐₓ (KBr): 3424, 2988, 1786, 1696, 1520, 1166, 1038, 704 cm⁻¹

### Physicochemical properties (isomer 20B)

Nuclear magnetic resonance spectrum (DMSO-d₆, TMS internal standard)
δ (ppm): 1.94 (1 H, m), 2.4 - 3.2 (4 H, m), 3.48 (1 H, d, J = 17.1 Hz), 3.63 (1 H, d, J = 17.1 Hz), 3.84 (3 H, s), 4.06 (1 H, m), 4.39 (1 H, m), 5.10 (1 H, d, J = 4.9 Hz), 5.65 (1 H, m), 5.76 (1 H, dd, J = 6.7, 9.1 Hz), 6.74 (1 H, s), 6.90 (1 H, d, J = 16.1 Hz), 7.21 (1 H, m), 9.58 (1 H, d, J = 7.9 Hz)
Positive ion-FAB-mass spectrum: m/z; 549 (M + H)⁺
Infrared absorption spectrum νₘₐₓ (KBr): 3452, 1768, 1674, 1540, 1388, 1040 cm⁻¹

### Physicochemical properties (36:64 mixture of two isomers, 20C)

Nuclear magnetic resonance spectrum (DMSO-d₆, TMS internal standard)
δ (ppm): 1.8 - 2.0 (1 H, m), 2.3 - 3.1 (4 H, m), 3.2 - 4.0 (7 H, m), 4.3 - 4.5 (1 H, m), 5.0 - 5.1 (1 H, m), 5.6 - 5.8 (2 H, m), 6.74 (1 H, s), 6.8 - 7.1 (1 H, m), 7.2 (2 H, s), 9.5 - 9.6 (1 H, m)
Positive ion-FAB-mass spectrum: m/z; 549 (M + H)⁺
Infrared absorption spectrum νₘₐₓ (KBr): 3456, 1768, 1684, 1538, 1390, 1038 cm⁻¹

### Example 21

(1) A 3441 mg portion of [7-(2-tritylaminothiazol-4-yl)-2-methoxyimino-2-acetamido-4-(p-methoxybenzyloxycarbonyl)-3-cephem-3-ylmethyl]triphenylphosphonium iodide was dissolved in 100 ml of chloroform, and the solution was mixed with 12 ml of 1 N NaOH, followed by stirring for 10 minutes. To this was added saturated sodium chloride aqueous solution to separate the organic layer, which was subsequently dried over anhydrous magnesium sulfate. To the above solution was added a chloroform solution containing 1.38 g of 5-formyl-2-isooxazolidine, followed by overnight stirring at room temperature. The reaction solution was washed with water and saturated sodium chloride aqueous solution and then dried over anhydrous magnesium sulfate. After concentrating the solvent under a reduced pressure, the thus obtained crude product was subjected to silica gel column chromatography and elution was carried out with chloroform-methanol (100:0 to 99:1 to 95:5, v/v). Fractions containing the compound of interest were pooled and then the solvent was evaporated to obtain 517 mg of p-methoxybenzyl 7β-[(Z)-2-(2-tritylamino-4-thiazolyl)-2-(methoxyimino)acetamido]-3-[(E,Z)-2-(2-methyl-5-isooxazolidinyl)vinyl]-3-cephem-4-carboxylate. This was a mixture of isomers based on (E,Z) and the isooxazole at the 5-position (RS).

### Physicochemical properties

Nuclear magnetic resonance spectrum (DMSO-d₆, TMS internal standard)
δ (ppm): 1.7 - 2.0 (1 H, m), 2.3 - 2.7 (5 H, m), 3.1 (1 H, m), 3.50 (1 H, m), 3.74 (3 H, s), 3.80 (3 H, s), 4.2 - 4.7 (1 H, m), 5.1 - 5.2 (2 H, m), 5.20 - 5.25 (1 H, m), 5.48 - 5.57 (1 H, m), 5.70 (1 H, m), 6.2 (1 H, m), 6.71 (1 H, s), 6.9 (2 H, m), 7.2 - 7.7 (17 H, m), 8.84 (1 H, s), 9.56 (1 H, d)
Positive ion-FAB-mass spectrum: m/z; 857 (M + H)⁺
(2) In an atmosphere of argon, 261 mg of the compound obtained in the above step (1) was dissolved in 5 ml of methylene chloride and 0.2 ml of anisole, the resulting solution was cooled at 10°C and mixed with 5 ml of trifluoroacetic acid. Then, the mixture was stirred for 60 minutes at 10°C to room temperature. Methylene chloride and trifluoroacetic acid were evaporated under a reduced pressure, and the thus obtained residue was again cooled to 10°C and mixed with 5 ml of trifluoroacetic acid and 3 ml of water. After 1 hour of stirring, the solvent was evaporated and the resulting residue was mixed with ethyl ether to obtain crude product. The E/Z ratio in the crude product was found to be 1:4 when analyzed by the analytical high performance liquid separation chromatography. The crude product was purified by high performance liquid separation chromatography to obtain 7β-[(Z)-2-(2-amino-4-thiazolyl)-2-(methoxyimino)acetamido]-3-[(Z)-2-(2-methyl-5- isooxazolidinyl)vinyl]-3-cephem-4-carboxylic acid (isomers 21A and 21B). At room temperature, each isomer showed two respective conformers on NMR.

### Physicochemical properties (isomer 21A)

Nuclear magnetic resonance spectrum (DMSO-d₆, TMS internal standard)
δ (ppm): 1.85 (1 H, m), 2.30 (1 H, m), 2.51 (3 H, s), 3.13 (1 H, m), 3.45 (1 H, m), 3.46 (1 H, ABq, J = 17 Hz), 3.60 (1 H, ABq, J = 17 Hz), 3.84 (3 H, s), 4.51 and 4.76 (each 0.5 H, m), 5.17 (1 H, d, J = 5 Hz), 5.43 (1 H, dd, J = 12, 10 Hz), 5.73 (1 H, dd, J = 8, 5 Hz), 6.31 (1 H, m), 6.74 (1 H, s), 7.2 (1 H, m), 9.59 (1 H, d, J = 8 Hz)
Positive ion-FAB-mass spectrum: m/z; 495 (M + H)⁺
Infrared absorption spectrum νₘₐₓ (KBr): 3424, 1722, 1670, 1610, 1538, 1388, 1354, 1040 cm⁻¹

### Physicochemical properties (isomer 21B)

Nuclear magnetic resonance spectrum (DMSO-d₆, TMS internal standard)
δ (ppm): 1.90 (1 H, m), 2.3 (1 H, m), 2.50 (3 H, m), 2.55 (1 H, m), 3.14 (1 H, m), 3.37 (2 H, m), 3.71 (2 H, m), 3.85 (3 H, s), 4.58 and 4.86 (each 0.5 H, m), 5.09 (1 H, d, J = 5 Hz), 5.30 (1 H, m), 5.61 (1 H, dd, J = 8, 5 Hz), 6.52 (1 H, m; 1 H, d, J = 13 Hz under heating at 80°C), 6.75 (1 H, s), 7.21 (1 H, m), 9.55 (1 H, d, J = 8 Hz)
Positive ion-FAB-mass spectrum: m/z; 495 (M + H)⁺
Infrared absorption spectrum νₘₐₓ (KBr): 3432, 1772, 1670, 1606, 1540, 1394, 1356, 1040 cm⁻¹

### Example 22

A 227 mg portion of the compound obtained in Example 21 (1) was dissolved in 4 ml of dimethylformamide, the resulting solution was mixed with 50 µl of methyl iodide, and the mixture was stirred overnight at room temperature. After evaporation of the solvent under a reduced pressure, the thus obtained solid substance was dissolved in 5 ml of methylene chloride, mixed with 0.2 g of anisole and 5 ml of trifluoroacetic acid, and the mixture was stirred for 1 hour at room temperature. Next, methylene chloride was evaporated under a reduced pressure, and the reaction solution was cooled to 10°C, mixed with 8 ml of trifluoroacetic acid and 3 ml of water, followed by stirring for 1 hour. Water and trifluoroacetic acid were evaporated and the resulting residue was solidified by adding ethyl ether, collected by filtration and then dried to obtain 165 mg of crude product. This was purified by high performance liquid chromatography to obtain 7β-[(Z)-2-(2-amino-4-thiazolyl)-2-(methoxyimino)acetamido]-3-[(Z)-2-(2,2-dimethyl-5-isooxazolidinio)vinyl]-3-cephem-4-carboxylate (isomers 22A and 22B).

### Physicochemical properties (isomer 22A)

Nuclear magnetic resonance spectrum (DMSO-d₆ + CD₃OD, TMS internal standard)
δ (ppm): 2.5 - 2.8 (2 H, m), 3.36 (1 H, ABq, J = 17 Hz), 3.53 (3 H, s), 3.56 (3 H, s), 3.69 (1 H, ABq, J = 17 Hz), 3.90 (3 H, s), 4.07 (1 H, m), 4.20 (1 H, m), 5.10 (1 H, d, J = 5 Hz), 5.39 (1 H, m), 5.44 (1 H, m), 5.68 (1 H, d, J = 5 Hz), 6.70 (1 H, d, J = 12 Hz), 6.80 (1 H, s)
Positive ion-FAB-mass spectrum: m/z; 509 (M + H)⁺
Infrared absorption spectrum νₘₐₓ (KBr): 3488, 1770, 1666, 1608, 1538, 1388, 1356, 1038 cm⁻¹

### Physicochemical properties (isomer 22B)

Nuclear magnetic resonance spectrum (DMSO-d₆, TMS internal standard)
δ (ppm): 2.5 - 2.8 (2 H, m), 3.30 (1 H, ABq, J = 17 Hz), 3.51 (3 H, s), 3.54 (3 H, s), 3.61 (1 H, ABq, J = 17 Hz), 3.83 (1 H, s), 4.03 (1 H, m), 4.18 (1 H, m), 5.07 (1 H, d, 1H, d, J = 5 Hz), 5.34 (1 H, m), 5.48 (1 H, m), 5.62 (1 H, dd, J = 8, 5 Hz), 6.62 (1 H, d, J = 12 Hz), 7.21 (1 H, s), 9.57 (1 H, d, J = 8 Hz)
Positive ion-FAB-mass spectrum: m/z; 509 (M + H)⁺
Infrared absorption spectrum νₘₐₓ (KBr): 3432, 1776, 1662, 1608, 1540, 1464, 1392, 1360, 1040 cm⁻¹

### Example 23

(1) To a 20 ml tetrahydrofuran solution containing 925 mg (1 mmol) of p-methoxybenzyl 7β-[(Z)-2-(2-tritylamino-4-thiazolyl)-2-(1-tert-butoxycarbonyl-1-methylethoxyimino)acetamido]-3-[(E)-1,3-butadienyl]-3-cephem-4-carboxylate obtained in Example (5), 123 mg (1 mmol) of sodium acetate and 0.17 ml (2 mmol) of 38% formalin aqueous solution was added dropwise 2.5 ml of 80% ethanol aqueous solution containing 126 mg (1.5 mmol) of N-methylhydroxylamine hydrochloride, and the mixture was stirred for 30 minutes at room temperature and then for 3 hours with heating under reflux. Thereafter, insoluble materials thus formed were removed by filtration, and the solvent was evaporated. The thus obtained residue was subjected to silica gel column chromatography and elution was carried out with chloroform-methanol (15:1, v/v). Fractions containing the compound of interest were pooled and then the solvent was evaporated to obtain 800 mg (81.3%) of p-methoxybenzyl 7β-[(Z)-2-(2-tritylamino-4-thiazolyl)-2-(1-tert-butoxycarbonyl-1-methylethoxyimino)acetamido]-3-[(E)-2-(2-methyl-5-isooxazolidinyl)vinyl]-3-cephem-4-carboxylate.

### Physicochemical properties

Nuclear magnetic resonance spectrum (DMSO-d₆, TMS internal standard)
δ (ppm): 1.3 - 1.4 (15 H, m), 1.8 - 2.1 (1 H, m), 2.3 - 2.7 (5 H, m), 3.13 (1 H, m), 3.55 (1 H, m), 3.74 (3 H, s), 3.84 (1 H, m), 4.3 - 4.7 (1 H, m), 5.1 - 5.3 (3 H, m), 5.7 (1 H, m), 6.0 - 6.2 (1 H, m), 6.66 (1 H, d, J = 15.6 Hz), 6.69 (1 H, s), 6.93 (2 H, d, J = 6.8 Hz), 7.1 - 7.4 (17 H, m), 8.80 (1 H, s), 9.35 (1 H, m)
Positive ion-FAB-mass spectrum: m/z; 999 (M - 1)⁺
Infrared absorption spectrum νₘₐₓ (KBr): 3420, 2992, 1790, 1728, 1690, 1520, 1372, 1306, 1252, 1146, 704 cm⁻¹
(2) To a 6 ml dimethylformamide solution containing 492 mg (0.5 mmol) of p-methoxybenzyl 7β-[(Z)-2-(2-tritylamino-4-thiazolyl)-2-(1-tert-butoxycarbonyl-1-methylethoxyimino)acetamido]-3-[(E)-2-(2-methyl-5-isooxazolidinyl)vinyl]-3-cephem-4-carboxylate obtained in the above step (1) was added dropwise 355 mg (2.5 mmol) of methyl iodide. After overnight stirring at room temperature, the solvent was evaporated and the thus obtained residue was solidified with ethyl ether to quantitatively obtain 560 mg of p-methoxybenzyl 7β-[(Z)-2-(2-tritylamino-4-thiazolyl)-2-(1-tert-butoxycarbonyl-1-methylethoxyimino)acetamido]-3-[(E)-2-(2,2-dimethyl-5-isooxazolidinio)vinyl]-3-cephem-4-carboxylate·iodide.

### Physicochemical properties

Nuclear magnetic resonance spectrum (DMSO-d₆, TMS internal standard)
δ (ppm): 1.2 - 1.6 (15 H, m), 2.5 - 2.6 (1 H, m), 2.7 - 3.0 (1 H, m), 3.4 - 3.7 (8 H, m), 3.76 (3 H, m), 3.88 (1 H, d, J = 16.0), 4.0 - 4.3 (2 H, m), 5.1 - 5.3 (3 H, m), 5.73 (1 H, m), 6.21 (1 H, m), 6.69 (1 H, s), 6.84 (1 H, d, J = 15.6 Hz), 6.94 (2 H, d, J = 8.3 Hz), 7.1 - 7.5 (17 H, m), 8.80 (1 H, s), 9.37 (1 H, d, J = 7.8 Hz)
Positive ion-FAB-mass spectrum: m/z; 985 (M + 1)⁺
Infrared absorption spectrum νₘₐₓ (KBr): 3432, 2992, 1788, 1726, 1686, 1520, 1388, 1372, 1306, 1250, 1222, 1178, 1146, 970, 704 cm⁻¹
(3) A 530 mg (0.47 mmol) portion of p-methoxybenzyl 7β-[(Z)-2-(2-tritylamino-4-thiazolyl)-2-(1-tert-butoxycarbonyl-1-methylethoxyimino)acetamido]-3-[(E)-2-(2,2-dimethyl-5-isooxazolidinio)vinyl]-3-cephem-4-carboxylate·iodide obtained in the above step (2) was dissolved in 9 ml of methylene chloride and 6 ml of anisole, the resulting solution was cooled in an ice bath and mixed with 15 ml of trifluoroacetic acid. Then, the mixture was stirred for 1 hour at 0°C to room temperature. The solvent was evaporated and the thus obtained residue was solidified by adding ethyl ether and collected by filtration. The thus obtained powder was suspended in 15 ml of water, mixed with 15 ml of trifluoroacetic acid while cooling in ac ice bath and then stirred for 1 hour at room temperature. After evaporation of the solvent, the resulting residue was mixed with ethyl ether to collect the formed powder by filtration. The thus obtained powder was dissolved in water and purified by HP-20 high performance liquid chromatography to obtain two isomers 23A (52 mg) and 23B (37 mg) of 7β-[(Z)-2-(2-amino-4-thiazolyl)-2-(1-carboxy-1-methylethoxyimino)acetamido]-3-[(E)-2-(2,2-dimethyl-5-isooxazolidinio)vinyl]-3-cephem-4-carboxylate.

### Physicochemical properties (isomer 23A)

Nuclear magnetic resonance spectrum (DMSO-d₆, TMS internal standard)
δ (ppm): 1.44 (3 H, s), 1.46 (3 H, s), 2.4 - 2.6 (1 H, m), 2.75 (1 H, m), 3.3 - 3.7 (9 H, m), 4.0 - 4.3 (2 H, m), 5.08 (1 H, d, J = 4, 9 Hz), 5.21 (1 H, m), 5.6 - 5.8 (2 H, m), 6.73 (1 H, s), 7.07 (1 H, d, J = 15.6 Hz), 7.26 (2 H, brs), 9.7 - 10.0 (1 H, brs)
Positive ion-FAB-mass spectrum: m/z; 581 (M + 1)⁺
Infrared absorption spectrum νₘₐₓ (KBr): 3452, 3052, 1774, 1670, 1598, 1540, 1470, 1392, 1164, 980 cm⁻¹

### Physicochemical properties (isomer 23B)

Nuclear magnetic resonance spectrum (DMSO-d₆, TMS internal standard)
δ (ppm): 1.42 (3 H, s), 1.47 (3 H, s), 2.4 - 2.7 (2 H, m), 3.4 - 3.7 (9 H, m), 4.07 (1 H, m), 4.25 (1 H, m), 5.12 (1 H, d, J = 4, 9 Hz), 5.35 (1 H, m), 5.63 (1 H, m), 5.70 (1 H, m), 6.72 (1 H, s), 7.05 (1 H, d, J = 15.6 Hz), 7.23 (2 H, brs)
Positive ion-FAB-mass spectrum: m/z; 581 (M + 1)⁺
Infrared absorption spectrum νₘₐₓ (KBr): 3448, 3052, 1772, 1672, 1598, 1538, 1470, 1394, 1164, 980 cm⁻¹

### Example 24

(1) Using 816 mg (1 mmol) of p-methoxybenzyl 7β-[(Z)-2-(2-tritylamino-4-thiazolyl)-2-fluoromethoxyimino)acetamido]-3-[(E)-1,3-butadienyl]-3-cephem-4-carboxylate obtained in Example 7 as the starting material, the procedure of Example 12 (1) was repeated to obtain 630 mg (72.0%) of p-methoxybenzyl 7β-[(Z)-2-(2-tritylamino-4-thiazolyl)-2-fluoromethoxyimino)acetamido]-3-[(E)-2-(2-methyl-5-isooxazolidinyl)vinyl]-3-cephem-4-carboxylate.

### Physicochemical properties

Nuclear magnetic resonance spectrum (DMSO-d₆, TMS internal standard)
δ (ppm): 1.7 - 2.1 (1 H, m), 2.3 - 2.8 (5 H, m), 3.13 (1 H, m), 3.57 (1 H, d, J = 17.6 Hz), 3.75 (3 H, s), 3.83 (1 H, d, J = 17.6 Hz), 4.2 - 4.7 (1 H, m), 5.1 - 5.3 (3 H, m), 5.62 (1 H, s), 5.69 (1 H, m), 5.76 (1 H, s), 6.09 (1 H, m), 6.6 - 6.8 (1 H, m), 6.89 (1 H, s), 6.93 (2 H, d, J = 7.8 Hz), 7.2 - 7.5 (17 H, m), 8.92 (1 H, s), 9.7 - 9.9 (1 H, m)
Positive ion-FAB-mass spectrum: m/z; 875 (M + 1)⁺
Infrared absorption spectrum νₘₐₓ (KBr): 3412, 2972, 1786, 1726, 1684, 1616, 1520, 1388, 1306, 1250, 1220, 1098, 988, 702 cm⁻¹
(2) Using 438 mg (0.5 mmol) of p-methoxybenzyl 7β-[(Z)-2-(2-tritylamino-4-thiazolyl)-1-methyl-1-methoxyethoxyimino)acetamido]-3-[(E)-2-(2-methyl-5-isooxazolidinyl)vinyl]-3-cephem-4-carboxylate obtained in (1) as the starting material, the procedure of Example 13 (1) was repeated to quantitatively obtain 510 mg of p-methoxybenzyl 7β-[(Z)-2-(2-tritylamino-4-thiazolyl)-2-fluoromethoxyimino)acetamido]-3-[(E)-2-(2,2-dimethyl-5-isooxazolidinio)vinyl]-3-cephem-4-carboxylate·iodide.

### Physicochemical properties

Nuclear magnetic resonance spectrum (DMSO-d₆, TMS internal standard)
δ (ppm): 2.5 - 3.0 (2 H, m), 3.4 - 3.7 (8 H, m), 3.75 (3 H, s), 3.86 (1 H, d, J = 17.6 Hz), 4.0 - 4.3 (2 H, m), 5.1 - 5.3 (3 H, m), 5.63 (1 H, s), 5.73 (1 H, m), 5.77 (1 H, s), 6.19 (1 H, m), 6.89 (1 H, s), 6.95 (2 H, d, J = 7.8 Hz), 7.1 - 7.5 (17 H, m), 8.93 (1 H, s), 9.78 (1 H, d, J = 7.8 Hz)
Positive ion-FAB-mass spectrum: m/z; 889 (M - 1)⁺
Infrared absorption spectrum νₘₐₓ (KBr): 3456, 2976, 1786, 1724, 1670, 1616, 1520, 1390, 1250, 1220, 1222, 1178, 1098, 984, 704 cm⁻¹
(3) Using 500 mg (0.49 mmol) of p-methoxybenzyl 7β-[(Z)-2-(2-tritylamino-4-thiazolyl)-2-fluoromethoxyimino)acetamido]-3-[(E)-2-(2,2-dimethyl-5-isooxazolidinio)vinyl)-3-cephem-4-carboxylate·iodide obtained in the above step (2) as the starting material, the procedure of Example 13 (2) was repeated to respectively obtain 85 mg and 87 mg of two isomers 24A and 24B of 7β-[(Z)-2-(2-amino-4-thiazolyl)-2-fluoromethoxyimino)acetamido]-3-[(E)-2-(2,2-dimethyl-5-isooxazolidinio)vinyl]-3-cephem-4-carboxylate.

### Physicochemical properties (isomer 24A)

Nuclear magnetic resonance spectrum (DMSO-d₆, TMS internal standard)
δ (ppm): 2.4 - 3.0 (2 H, m), 3.2 - 3.6 (8 H, m), 4.0 - 4.3 (2 H, m), 5.07 (1 H, d, J = 4.9 Hz), 5.21 (1 H, m), 5.5 - 5.7 (3 H, m), 5.80 (1 H, s), 6.19 (1 H, m), 6.91 (1 H, s), 7.10 (1 H, d, J = 15.6 Hz), 7.30 (2 H, brs), 9.77 (1 H, d, J = 8.4 Hz)
Positive ion-FAB-mass spectrum: m/z; 527 (M + 1)⁺
Infrared absorption spectrum νₘₐₓ (KBr): 3448, 1770, 1670, 1612, 1540, 1388, 1098, 984 cm⁻¹

### Physicochemical properties (isomer 24B)

Nuclear magnetic resonance spectrum (DMSO-d₆, TMS internal standard)
δ (ppm): 2.4 - 3.0 (2 H, m), 3.2 - 3.6 (8 H, m), 4.0 - 4.3 (2 H, m), 5.08 (1 H, d, J = 4.9 Hz), 5.20 (1 H, m), 5.5 - 5.7 (3 H, m), 5.80 (1 H, s), 6.19 (1 H, m), 6.90 (1 H, s), 7.10 (1 H, d, J = 15.6 Hz), 7.29 (2 H, brs), 9.77 (1 H, d, J = 8.4 Hz)
Positive ion-FAB-mass spectrum: m/z; 527 (M + 1)⁺
Infrared absorption spectrum νₘₐₓ (KBr): 3456, 2940, 1774, 1674, 1612, 1540, 1390, 1098, 970 cm⁻¹

### Example 25

(1) Using 856 mg (1 mmol) of p-methoxybenzyl 7β-[(Z)-2-(2-tritylamino-4-thiazolyl)-2-(1-methoxy-1-methyl)ethoxyiminoacetamido]-3-[(E)-1,3-butadienyl]-3-cephem-4-carboxylate obtained in Example 6 as the starting material, the procedure of Example 12 (1) was repeated to obtain 640 mg (70.0%) of p-methoxybenzyl 7β-[(Z)-2-(2-tritylamino-4-thiazolyl)-2-(1-methoxy-1-methyl)ethoxyiminoacetamido]-3- [(E)-2-((RS)-2-methyl-5-isooxazolidinyl)vinyl]-3-cephem-4-carboxylate.

### Physicochemical properties

Nuclear magnetic resonance spectrum (DMSO-d₆, TMS internal standard)
δ (ppm): 1.39 (6 H, s), 1.7 - 2.1 (1H, m), 2.2 - 2.8 (5 H, m), 3.0 - 3.3 (4 H, m), 3.5 - 3.7 (1 H, m), 3.75 (3 H, s), 3.86 (1 H, d, J = 17.6 Hz), 4.2 - 4.7 (1 H, m), 5.1 - 5.3 (3 H, m), 5.68 (1 H, s), 6.09 (1 H, m), 6.6 - 6.8 (2 H, m), 6.93 (2 H, d, J = 7.8 Hz), 7.2 - 7.5 (17 H, m), 8.86 (1 H, s), 9.5 - 9.6 (1 H, m)
Positive ion-FAB-mass spectrum: m/z; 915 (M + 1)⁺
Infrared absorption spectrum νₘₐₓ (KBr): 3416, 3072, 3004, 2968, 1788, 1726, 1688, 1618, 1520, 1376, 1306, 1452, 1376, 1306, 1250, 1218, 1178, 1156, 1070, 958, 894, 754, 702 cm⁻¹
(2) Using 458 mg (0.5 mmol) of p-methoxybenzyl 7β-[(Z)-2-(2-tritylamino-4-thiazolyl)-2-(1-methoxy-1-methyl)ethoxyiminoacetamido]-3-[(E)-2-((RS)-2-methyl-5-isooxazolidinyl)vinyl]-3-cephem-4-carboxylate obtained in the above step (1) as the starting material, the procedure of Example 13 (1) was repeated to quantitatively obtain 520 mg of p-methoxybenzyl 7β-[(Z)-2-(2-tritylamino-4-thiazolyl)-2-(1-methoxy-1-ethyl)ethoxyiminoacetamido)-3-[(E)-2-((RS)-2,2- dimethyl-5-isooxazolidinio)vinyl]-3-cephem-4-carboxylate·iodide.

### Physicochemical properties

Nuclear magnetic resonance spectrum (DMSO-d₆, TMS internal standard)
δ (ppm): 1.39 (6 H, s), 2.5 - 2.9 (2 H, m), 3.11 (3 H, s), 3.4 - 3.7 (8 H, m), 3.76 (3 H, s), 3.93 (1 H, d, J = 17.6 Hz), 4.0 - 4.3 (2 H, m), 5.1 - 5.3 (3 H, m), 5.70 (1 H, m), 6.20 (1 H, m), 6.73 (1 H, s), 6.84 (1 H, d, J = 15.6 Hz), 6.94 (2 H, d, J = 7.8 Hz), 7.1 - 7.5 (17 H, m), 8.87 (1 H, s), 9.54 (1 H, d, J = 7.8 Hz)
Positive ion-FAB-mass spectrum: m/z; 929 (M - 1)⁺
Infrared absorption spectrum νₘₐₓ (KBr): 3448, 3012, 1786, 1728, 1672, 1618, 1520, 1388, 1250, 1220, 1180, 1068, 976, 894, 754, 704 cm⁻¹
(3) Using 500 mg (0.47 mmol) of p-methoxybenzyl 7β-[(Z)-2-(2-tritylamino-4-thiazolyl)-2-(1-methoxy-1-methyl)ethoxyiminoacetamido]-3-[(E)-2-((RS)-2,2-dimethyl-5-isooxazolidinio)vinyl]-3-cephem-4-carboxylate·iodide obtained in the above step (2) as the starting material, the procedure of Example 13 (2) was repeated to obtain 35 mg of 7β-[(Z)-2-(2-amino-4-thiazolyl)-2-(hydroxyimino)acetamido]-3-[(E)-2-(2,2-dimethyl-5-isooxazolidinio)vinyl]-3-cephem-4-carboxylate (compound 25A) and 20 mg of 7β-[(E)-2-(2-amino-4-thiazolyl)-2-(hydroxyimino)acetamido]-3-[(E)-2-(2,2-dimethyl-5-isooxazolidinio)vinyl]-3-cephem-4-carboxylate (compound 25B).

### Physicochemical properties (compound 25A)

Nuclear magnetic resonance spectrum (DMSO-d₆, TMS internal standard)
δ (ppm): 2.4 - 2.6 (1 H, m), 2.7 - 2.9 (1 H, m), 3.2 - 3.6 (8 H, m), 4.0 - 4.3 (2 H, m), 5.03 (1 H, m), 5.18 (1 H, m), 5.5 - 5.7 (2 H, m), 5.80 (1 H, s), 6.65 (1 H, s), 6.91 (1 H, s), 7.0 - 7.2 (3 H, s), 9.40 (1 H, d, J = 7.8 Hz), 11.33 (1 H, brs)
Positive ion-FAB-mass spectrum: m/z; 495 (M + 1)⁺
Infrared absorption spectrum νₘₐₓ (KBr): 3448, 3048, 1772, 1608, 1540, 1394, 1184, 1048, 970 cm⁻¹

### Physicochemical properties (compound 25B)

Nuclear magnetic resonance spectrum (DMSO-d₆, TMS internal standard)
δ (ppm): 2.4 - 2.6 (1 H, m), 2.6 - 2.9 (1 H, m), 3.2 - 3.6 (8 H, m), 4.0 - 4.3 (2 H, m), 5.04 (1 H, m), 5.21 (1 H, m), 5.5 - 5.7 (2 H, m), 7.11 (1 H, d, J = 15.6 Hz), 7.20 (2 H, s), 7.50 (1 H, s), 9.27 (1 H, m), 12.61 (1 H, m)
Positive ion-FAB-mass spectrum: m/z; 495 (M + 1)⁺
Infrared absorption spectrum νₘₐₓ (KBr): 3448, 2360, 1768, 1672, 1610, 1534, 1394, 1180, 1004, 970 cm⁻¹

### Example 26

(1) A 351 mg (0.49 mmol) portion of the compound obtained in Example 8 was dissolved in 8 ml of tetrahydrofuran, and the resulting solution was mixed with 60 mg (0.735 mmol) of sodium acetate and 0.077 ml (0.98 mmol) of 38% formalin aqueous solution. To this solution cooled in an ice bath was added dropwise 62 mg (0.735 mmol) of N-methylhydroxylamine hydrochloride which has been dissolved in 80% ethanol, followed by 3 hours of heating under reflux. Thereafter, the solvent was evaporated, the resulting residue was subjected to silica gel chromatography and elution was carried out with chloroform-methanol (100:2, v/v). Fractions containing the compound of interest were pooled and then the solvent was evaporated to obtain 244 mg of 4-methoxybenzyl 7β-[(Z)-2-(5-tert-butoxycarbonylamino-1,2,4-thiadiazol-3-yl)-2-(1-methyl-1-methoxy)ethoxyiminoacetamido]-3-[(E)-2-((RS)-2-methyl-5-isooxazolidinyl)vinyl]-3-cephem-4-carboxylate.

### Physicochemical properties

Nuclear magnetic resonance spectrum (DMSO-d₆, TMS internal standard)
δ (ppm): 1.46 (6 H, s), 1.51 (9 H, s), 1.8 - 2.0 (1 H, m), 2.3 - 2.7 (3 H, m), 2.5 (3 H, s), 3.16 (2 H, m), 3.28 and 3.31 (3 H, each s), 3.58 (1 H, ABq), 3.76 (3 H, s), 3.90 (1 H, ABq), 4.2 - 4.6 (1 H, m), 5.19 - 5.24 (3 H, m), 5.88 (1 H, q, J = 5, 8 Hz), 6.1 (1 H, m), 6.64 (1 H, d, J = 15 Hz), 6.94, 7.36 (4 H, ABq), 9.68 (1 H, m), 12.4 (1 H, brs)
Positive ion-FAB-mass spectrum: m/z; 774 (M + H)⁺
Infrared absorption spectrum νₘₐₓ (KBr): 1790, 1722, 1548, 1520, 1374, 1250, 1222, 1176, 1156 cm⁻¹
(2) A 230 mg (0.30 mmol) portion of the compound obtained in the above step (1) was dissolved in 4 ml of DMF, the solution was mixed with 93 µl (1.5 mmol) of methyl iodide, and the mixture was stirred overnight at room temperature. The solvent was evaporated under a reduced pressure, and the resulting residue was solidified by adding ethyl ether, collected by filtration and then dried to obtain 251 mg of p-methoxybenzyl 7β-[(Z)-2-(5-tert-butoxycarbonylamino-1,2,4-thiadiazol-3-yl)-2-(1-methoxy-1-methyl)ethoxyiminoacetamido]-3-[(E)-2-((RS)-2,2-dimethyl-5-isooxazolidinio)vinyl]-3-cephem-4-carboxylate·iodide.

### Physicochemical properties

Nuclear magnetic resonance spectrum (DMSO-d₆, TMS internal standard)
δ (ppm): 1.46 (6 H, s), 1.51 (9 H, s), 2.5 (1 H, m), 2.8 (1 H, m), 3.16 (3 H, s), 3.54 (1 H, ABq), 3.6 (6 H, m), 3.76 (3 H, s), 3.9 (1 H, ABq), 4.0 - 4.3 (2 H, m), 5.1 - 5.2 (4 H, m), 5.9 (1 H, m), 6.2 (1 H, m), 6.84 (1 H, d, J = 16 Hz), 6.95 (2 H, ABq, J = 8 Hz), 7.36 (2 H, ABq, J = 8 Hz), 9.70 (1 H, d, J = 8 Hz)
Positive ion-FAB-mass spectrum: m/z; 788 (M - I)⁺
Infrared absorption spectrum νₘₐₓ (KBr): 1788, 1720, 1552, 1388, 1248, 1154 cm⁻¹
(3) A 251 mg (0.27 mmol) portion of the compound obtained in the above step (2) and 0.2 ml of anisole were dissolved in 2 ml of methylene chloride, the resulting solution was mixed with 2 ml of trifluoroacetic acid, and the mixture was stirred for 1 hour at room temperature. The reaction solution was concentrated under a reduced pressure, the residue was again mixed with 2 ml of trifluoroacetic acid and 4 ml of methylene chloride, and the mixture was stirred for 1 hour at room temperature. The reaction solution was concentrated under a reduced pressure, and the resulting residue was mixed with ethyl ether to obtain 167 mg of crude product. This was purified by HP-20 column chromatography to obtain 135 mg of 7β-[(EZ)-2-(5-amino-1,2,4-thiadiazol-3-yl)-2-(hydroxyiminoacetamido]-3-[(E)-2-((RS)-2,2-dimethyl-5-isooxazolidinio)vinyl]-3-cephem-4-carboxylate. This was further purified by a high performance liquid chromatography to obtain two components (26A and 26B) based on the oxime E,Z of the above compound. These were diastereomer mixtures based on the isooxazolidine at the 5-position.

### Physicochemical properties (component 26A)

Nuclear magnetic resonance spectrum (DMSO-d₆, TMS internal standard)
δ (ppm): 2.6 - 2.7 (2 H, m), 3.3 - 3.6 (8 H, m), 4.0 - 4.18 (2 H, m), 5.01, 5.04 (1 H, each d, J = 5 Hz), 5.18 - 5.20 (1 H, m), 5.56 - 5.66 (1 H, m), 7.08 (1 H, d, J = 16 Hz), 8.06 (2 H, brs), 9.38 (1 H, d, J = 8 Hz), 11.95 (1 H, brs)
Positive ion-FAB-mass spectrum: m/z; 496 (M + H)⁺
Infrared absorption spectrum νₘₐₓ (KBr): 3416, 1770, 1670, 1604, 1526, 1392, 1178 cm⁻¹

### Physicochemical properties (component 26B)

Nuclear magnetic resonance spectrum (DMSO-d₆, TMS internal standard)
δ (ppm): 2.7 - 2.8 (2 H, m), 3.4 - 3.7 (8 H, m), 4.07 - 4.18 (2 H, m), 5.04 and 5.05 (1 H, each d, J = 5 Hz), 5.19 - 5.21 (1 H, m), 5.19 - 5.21 (1 H, m), 5.58 - 5.66 (2 H, m), 7.10 (1 H, d, J = 16 Hz), 8.07 (1 H, s), 8.78 (1 H, d, J = 8 Hz), 12.7 (1 H, brs)
Positive ion-FAB-mass spectrum: m/z; 496 (M + H)⁺
Infrared absorption spectrum νₘₐₓ (KBr): 3432, 1770, 1680, 1606, 1518, 1394, 1356 cm⁻¹

### Example 27

(1) Using 1.4 g (1.3 mmol) of p-methoxybenzyl 7β-[(Z)-2-(5-tritylamino-1,2,4-thiadiazol-3-yl)-2-fluoromethoxyimino)acetamido]-3-[(E)-4-diphenylphosphoryloxy-2-butenyl]-3-cephem-4-carboxylate obtained in Reference Example 10 as the starting material, the procedure of Example 4 was repeated to obtain 840 mg (79.1%) of p-methoxybenzyl 7β-[(Z)-2-(5-tritylamino-1,2,4-thiadiazol-3-yl)-2-fluoromethoxyimino)acetamido]-3-[(E)-1,3-butadienyl]-3-cephem-4-carboxylate.

### Physicochemical properties

Nuclear magnetic resonance spectrum (DMSO-d₆, TMS internal standard)
δ (ppm): 3.60 (1 H, d, J = 17.6 Hz), 3.75 (3 H, s), 3.90 (1 H, d, J = 17.6 Hz), 5.09 - 5.20 (1 H, m), 5.19 (2 H, s), 5.25 (1 H, d, J = 10.7 Hz), 5.38 (1 H, d, J = 17.1 Hz), 5.70 (1 H, s), 5.80 (1 H, m), 5.84 (1 H, s), 6.3 - 6.45 (1 H, m), 6.34 - 6.76 (2 H, m), 6.94 (2 H, d, J = 8.3 Hz), 7.2 - 7.4 (17 H, m), 9.75 (1 H, d, J = 8.3 Hz), 10.08 (1 H, s)
Positive ion-FAB-mass spectrum: m/z; 817 (M + 1)⁺
Infrared absorption spectrum νₘₐₓ (KBr): 3308, 3040, 2980, 1780, 1698, 1616, 1520, 1450, 1390, 1366, 1306, 1250, 1222, 1168, 1124, 1076, 1004, 756, 702 cm⁻¹

### Example 28

(1) Using 810 mg (0.99 mmol) of p-methoxybenzyl 7β-[(Z)-2-(5-tritylamino-1,2,4-thiadiazol-3-yl)-2-fluoromethoxyimino)acetamido]-3-[(E)-1,3-butadienyl]-3-cephem-4-carboxylate obtained in Example 27 as the starting material, the procedure of Example 14 (1) was repeated to obtain 678 mg (78.2%) of p-methoxybenzyl 7β-[(Z)-2-(5-tritylamino-1,2,4-thiadiazol-3-yl)-2-fluoromethoxyimino)acetamido]-3-[(E)-2-(2-methyl-5-isooxazolidinyl)vinyl]-3-cephem-4-carboxylate.

### Physicochemical properties

Nuclear magnetic resonance spectrum (DMSO-d₆, TMS internal standard)
δ (ppm): 1.7 - 2.1 (1 H, m), 2.2 - 2.8 (5 H, m), 3.13 (1 H, m), 3.55 (1 H, m), 3.75 (3 H, s), 3.84 (1 H, m), 4.2 - 4.7 (1 H, m), 5.0 - 5.3 (3 H, m), 5.70 (1 H, s), 5.79 (1 H, m), 5.84 (1 H, s), 6.08 (1 H, m), 6.6 - 6.7 (1 H, m), 6.93 (2 H, d, J = 8.8 Hz), 7.1 - 7.4 (17 H, m), 9.75 (1 H, m), 10.08 (1 H, s)
Positive ion-FAB-mass spectrum: m/z; 876 (M + 1)⁺
Infrared absorption spectrum νₘₐₓ (KBr): 3416, 2976, 1788, 1724, 1696, 1616, 1520, 1450, 1392, 1372, 1306, 1250, 1222, 1178, 1124, 1076, 1006, 754, 702 cm⁻¹
(2) Using 640 mg (0.73 mmol) of p-methoxybenzyl 7β-[(Z)-2-(5-tritylamino-1,2,4-thiadiazol-3-yl)-2-fluoromethoxyimino)acetamido]-3-[(E)-2-(2-methyl-5-isooxazolidinyl)vinyl]-3-cephem-4-carboxylate obtained in the above step (1) as the starting material, the procedure of Example 14 (2) was repeated to obtain 700 mg (94.2%) of p-methoxybenzyl 7β-[(Z)-2-(5-tritylamino-1,2,4-thiadiazol-3-yl)-2-fluoromethoxyimino)acetamido]-3-[(E)-2-(2,2-dimethyl-5-isooxazolidinyl)vinyl]-3-cephem-4-carboxylate·iodide.

### Physicochemical properties

Nuclear magnetic resonance spectrum (DMSO-d₆, TMS internal standard)
δ (ppm): 2.4 - 2.6 (1 H, m), 2.7 - 2.9 (1 H, m), 3.4 - 3.7 (7 H, m), 3.75 (3 H, m), 3.8 - 3.9 (1 H, m), 4.0 - 4.3 (2 H, m), 5.1 - 5.3 (3 H, m), 5.70 (1 H, s), 5.8 - 5.9 (2 H, m), 6.1 - 6.3 (1 H, m), 6.84 (1 H, d, J = 15.6 Hz), 6.94 (2 H, m), 7.2 - 7.5 (17 H, m), 9.76 (1 H, m), 10.09 (1 H, s)
Positive ion-FAB-mass spectrum: m/z; 890 (M + 1)⁺
Infrared absorption spectrum νₘₐₓ (KBr): 3464, 3040, 2976, 1786, 1724, 1684, 1616, 1520, 1452, 1394, 1366, 1306, 1250, 1222, 1178, 1124, 1074, 1004, 860, 756, 704 cm⁻¹
(3) Using 690 mg (0.68 mmol) of p-methoxybenzyl 7β-[(Z)-2-(5-tritylamino-1,2,4-thiadiazol-3-yl)-2-fluoromethoxyimino)acetamido]-3-[(E)-2-(2,2-dimethyl-5-isooxazolidinio)vinyl]-3-cephem-4-carboxylate·iodide obtained in the above step (2) as the starting material, the procedure of Example 14 (3) was repeated to obtain two isomers A (81 mg) and B (115 mg) of 7β-[(Z)-2-(5-amino-1,2,4-thiadiazol-3-yl)-2-fluoromethoxyimino)acetamido]-3-[(E)-2-(2,2-dimethyl-5-isooxazolidinio)vinyl]-3-cephem-4-carboxylate.

### Physicochemical properties

### Isomer 28A

Nuclear magnetic resonance spectrum (DMSO-d₆, TMS internal standard)
δ (ppm): 2.43 - 2.6 (1 H, m), 2.65 - 2.8 (1 H, m), 3.3 - 3.6 (8 H, m), 4.0 - 4.23 (2 H, m), 5.04 (1 H, d, J = 4.9 Hz), 5.20 (1 H, m), 5.55 - 5.65 (2 H, m), 5.72 (1 H, s), 5.86 (1 H, s), 7.10 (1 H, d, J = 15.6 Hz), 8.24 (2 H, s), 9.71 (1 H, d, J = 8.3 Hz)
Positive ion-FAB-mass spectrum: m/z; 528 (M + 1)⁺
Infrared absorption spectrum νₘₐₓ (KBr): 3448, 3056, 1772, 1676, 1610, 1532, 1454, 1398, 1354, 1180, 1144, 1086, 1064, 992, 864 cm⁻¹

### Isomer 28B

Nuclear magnetic resonance spectrum (DMSO-d₆, TMS internal standard)
δ (ppm): 2.45 - 2.6 (1 H, m), 2.68 - 2.8 (1 H, m), 3.3 - 3.7 (9 H, m), 4.0 - 4.23 (2 H, m), 5.05 (1 H, d, J = 4.9 Hz), 5.19 (1 H, m), 5.55 - 5.66 (2 H, m), 5.72 (1 H, s), 5.86 (1 H, s), 7.09 (1 H, d, J = 15.6 Hz), 8.26 (1 H, s), 9.72 (1 H, d, J = 8.3 Hz)
Positive ion-FAB-mass spectrum: m/z; 528 (M + 1)⁺
Infrared absorption spectrum νₘₐₓ (KBr): 3452, 1770, 1674, 1610, 1532, 1464, 1398, 1354, 1182, 1140, 1088, 1062, 998, 860 cm⁻¹

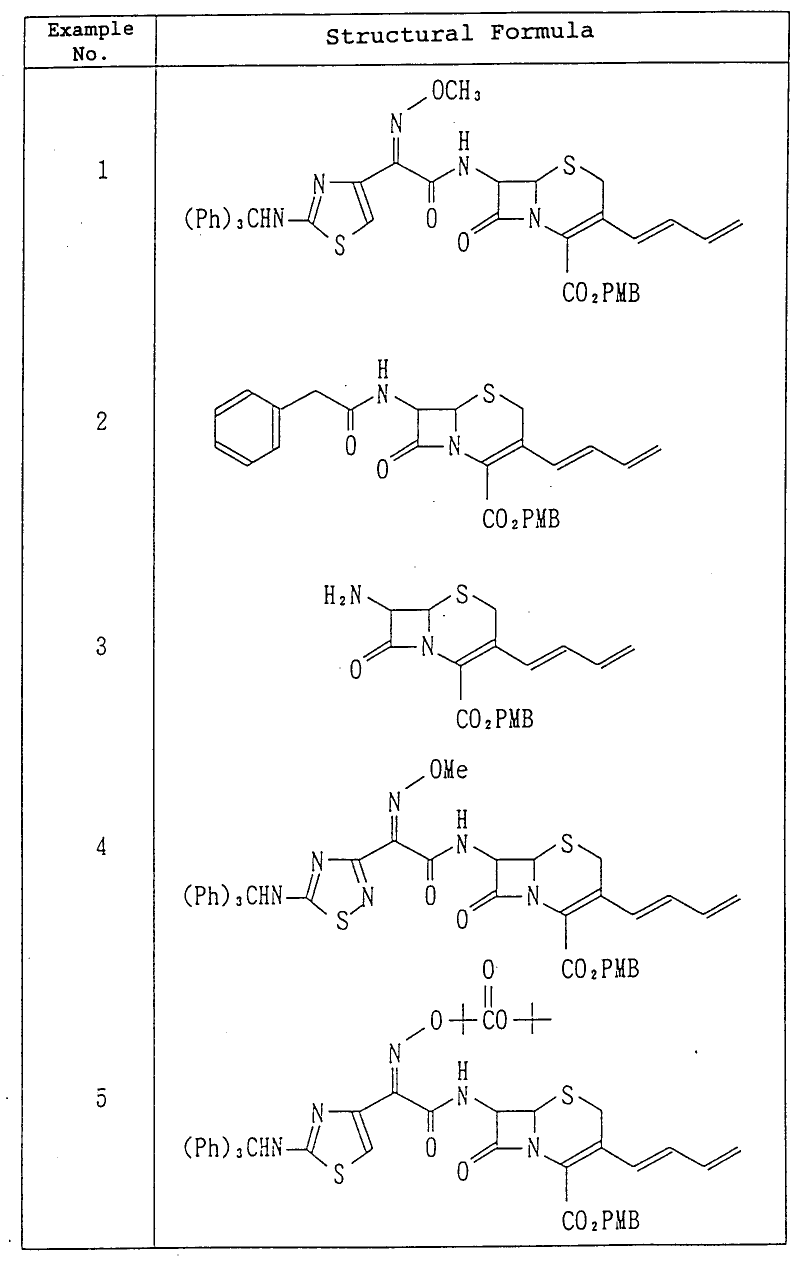

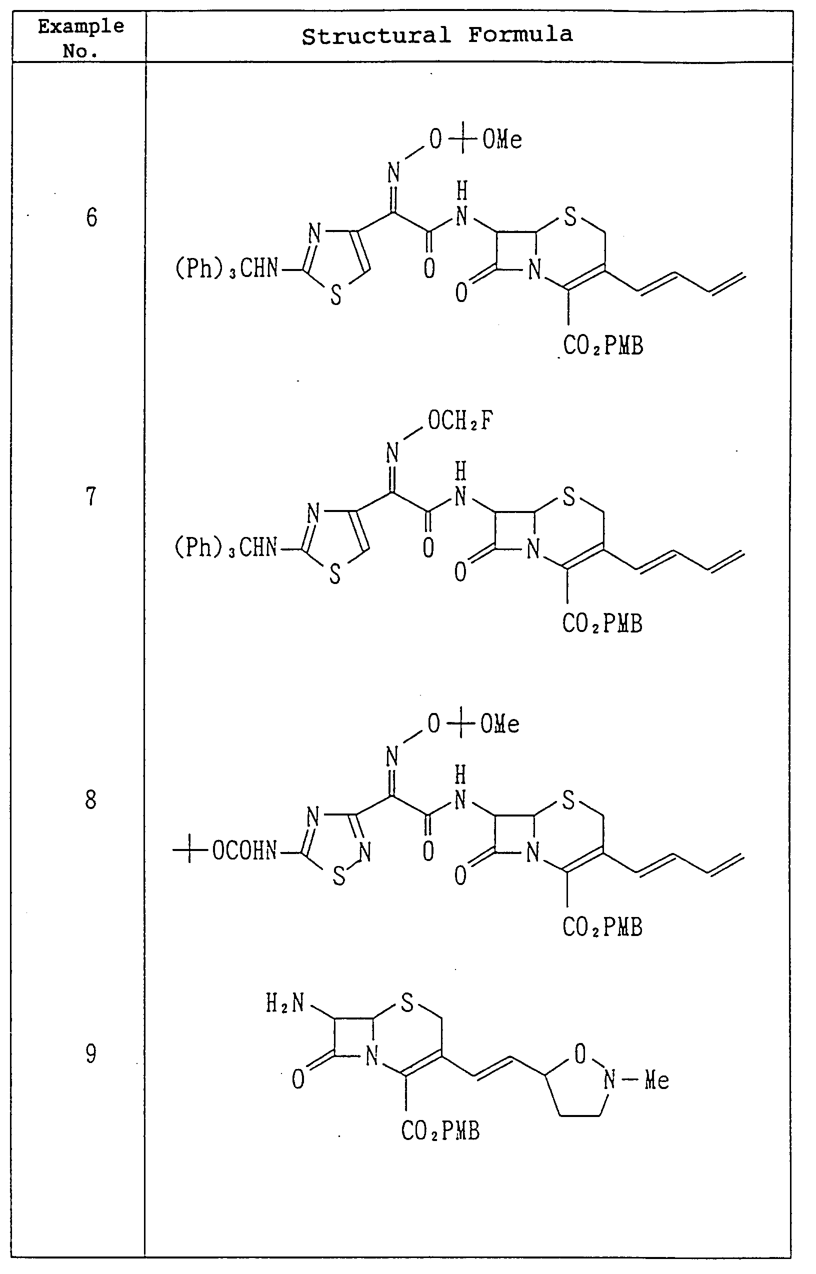

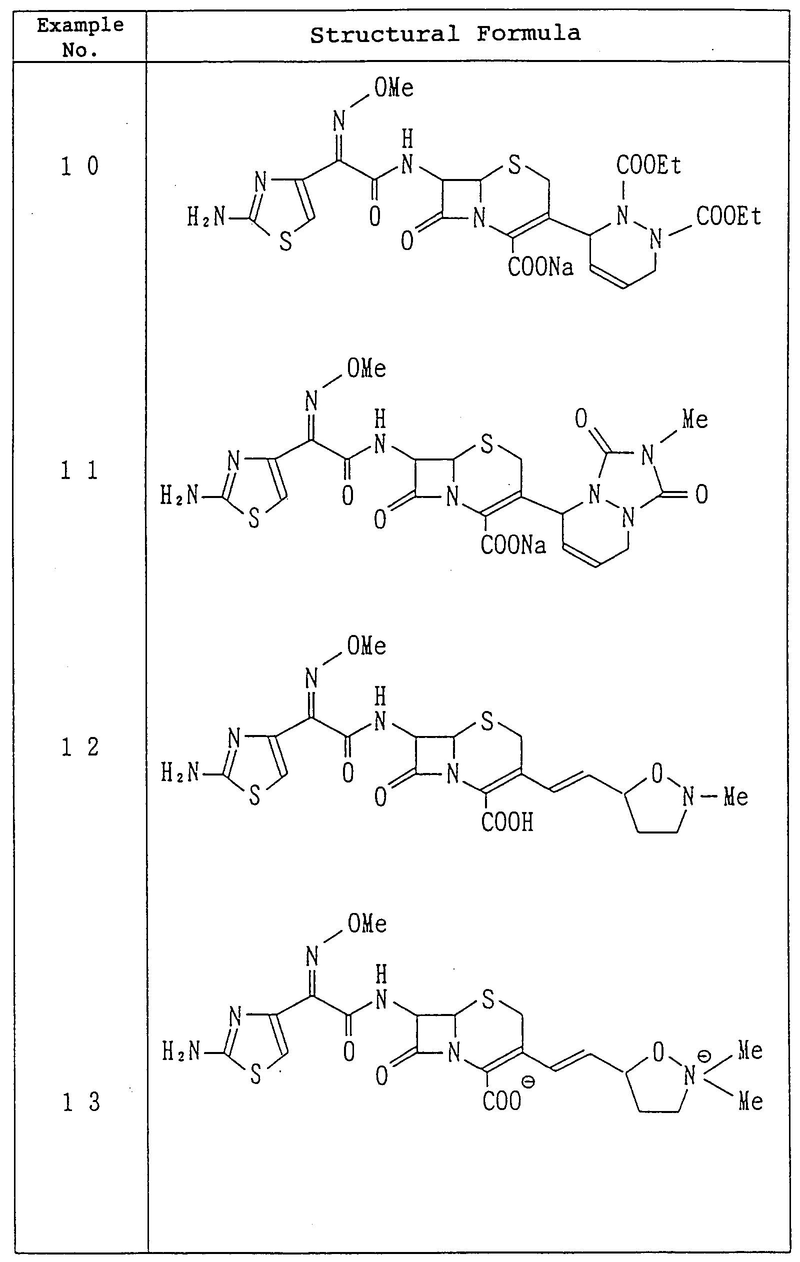

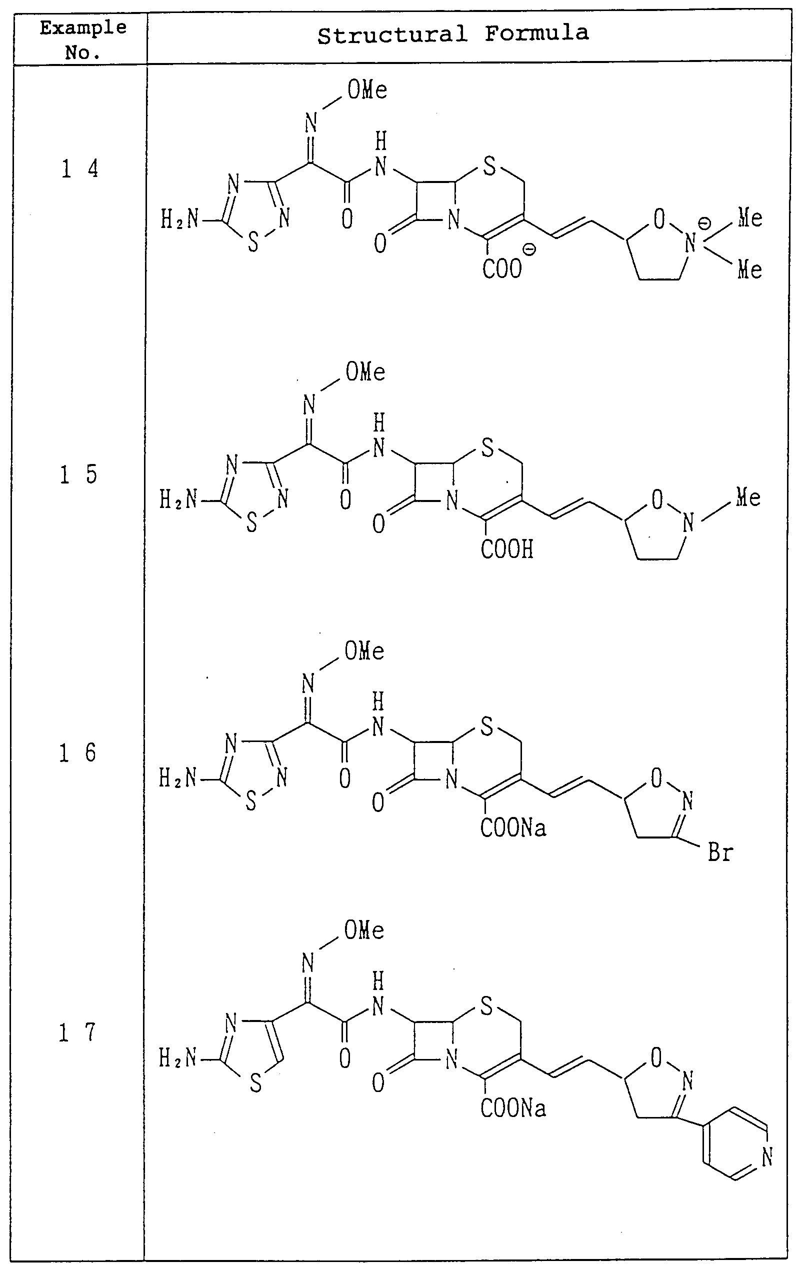

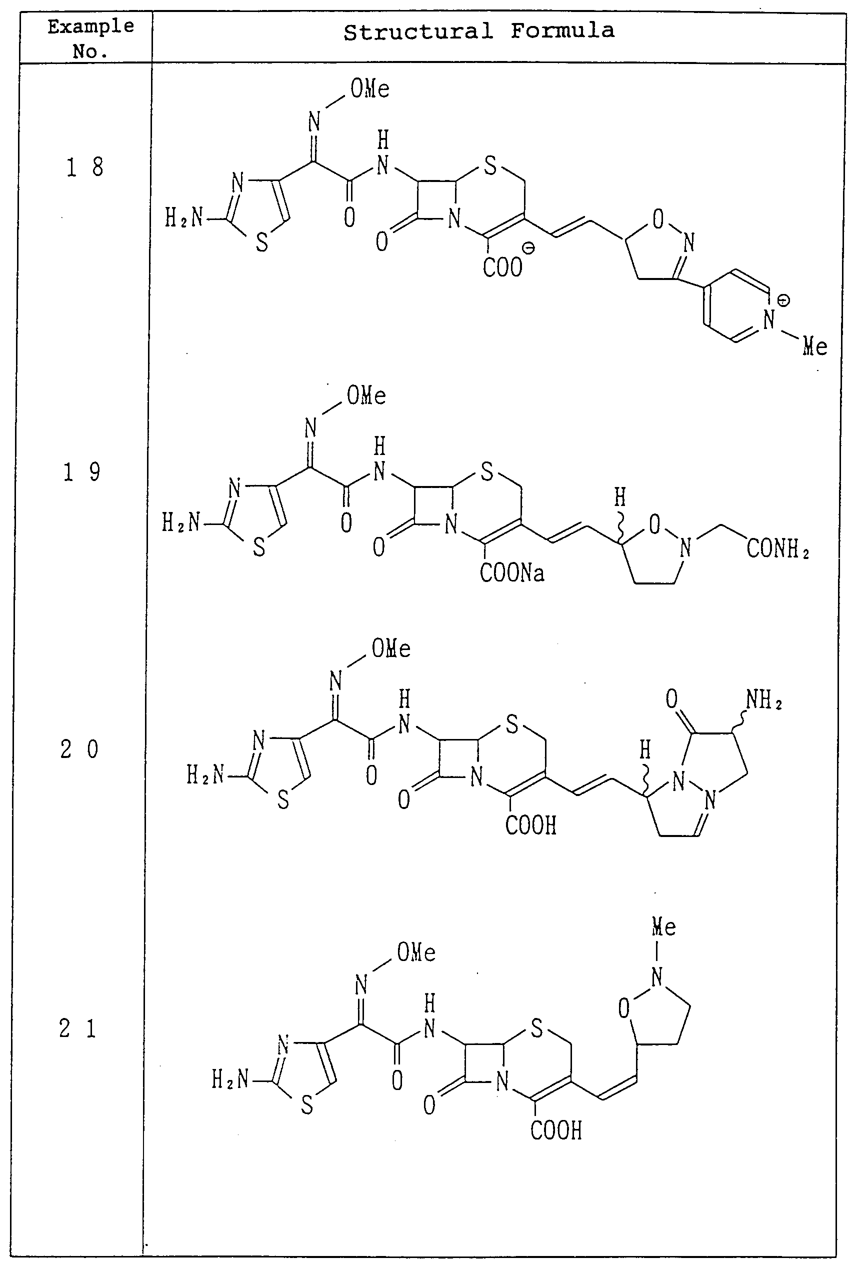

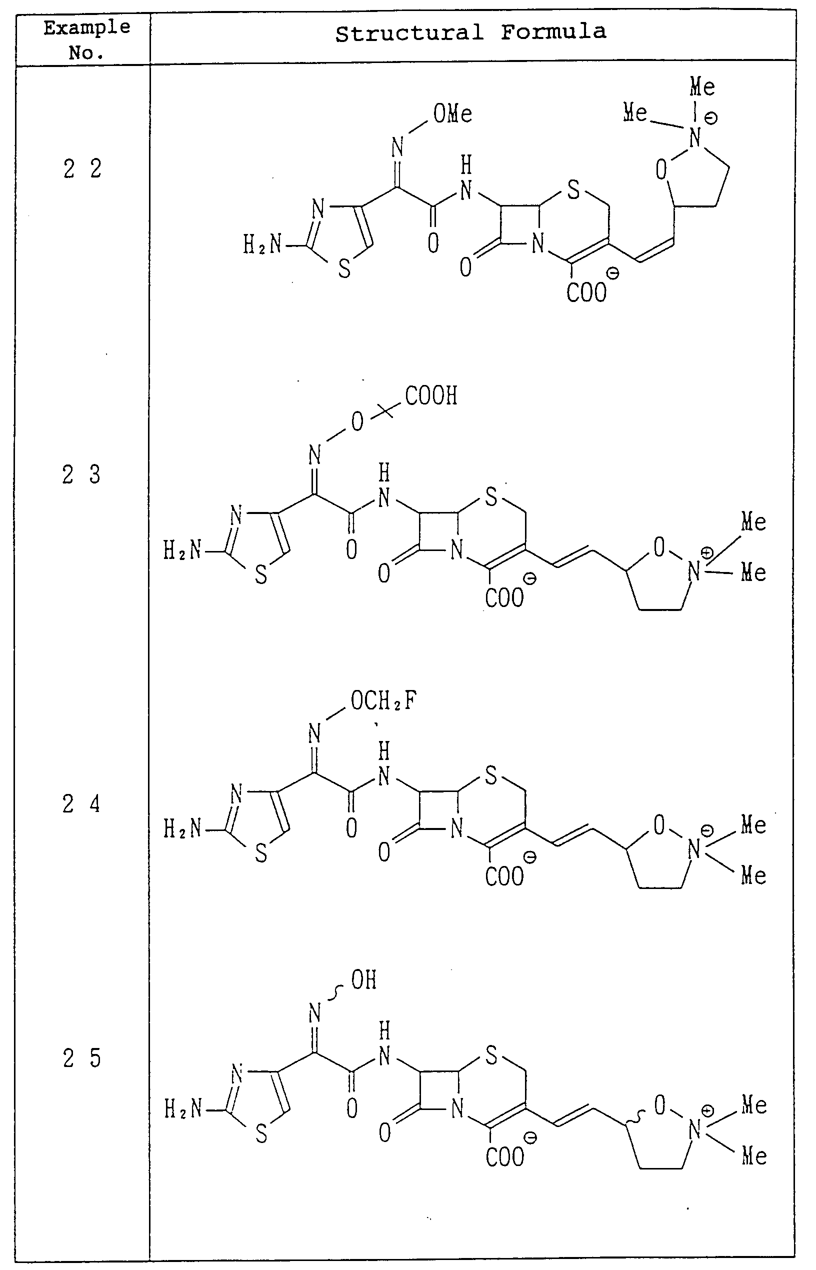

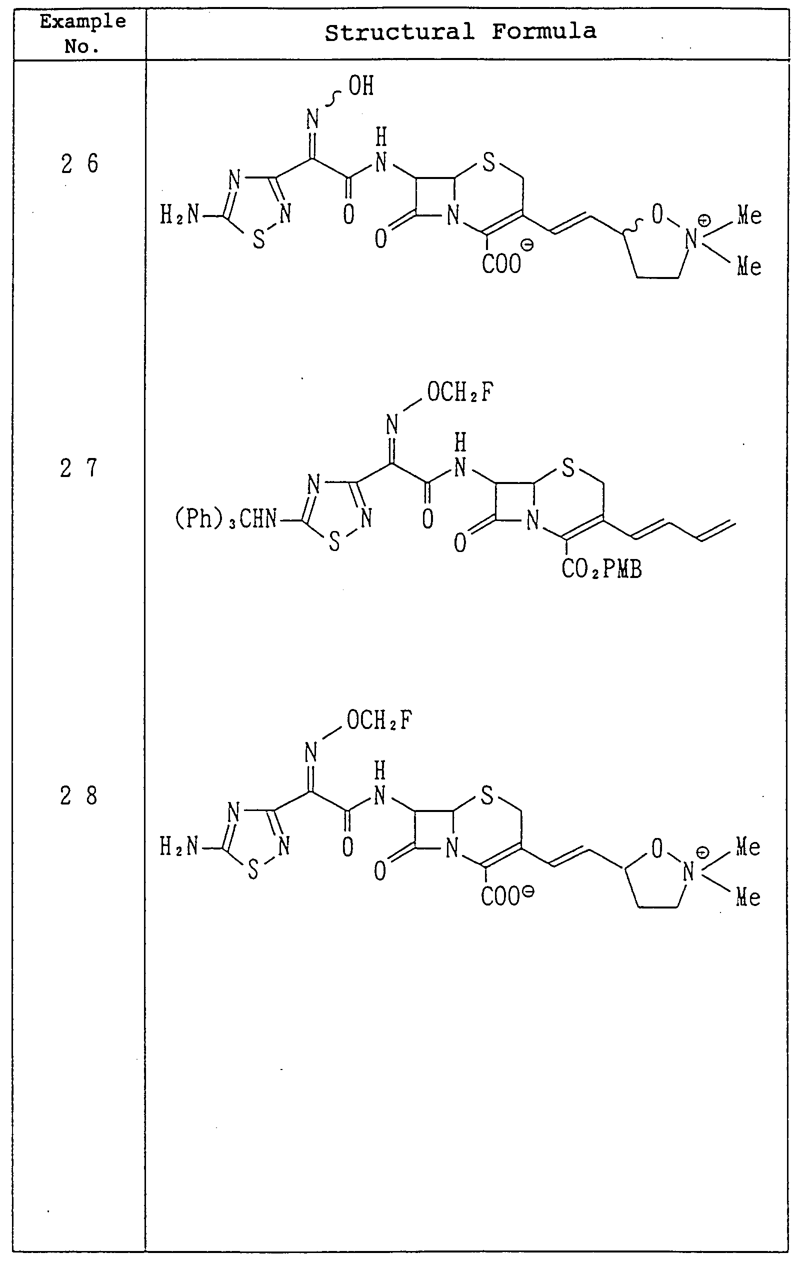

## Claims

1. A cephalosporin derivative represented by the following general formula (I) 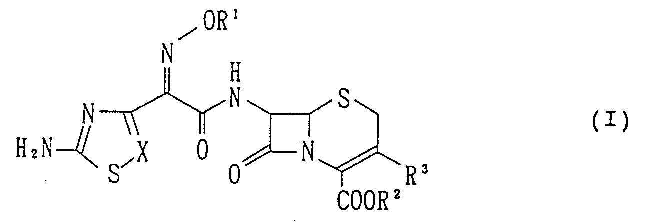 (symbols in the formula have the following meanings
R¹: a hydrogen atom, a lower alkenyl group, a lower alkynyl group, a cycloalkyl group, an protective group for the hydroxyl group or a substituted or unsubstituted lower alkyl group,
R²: a hydrogen atom, an ester residue or a negative charge,
R³: (1) a group represented by the following formula 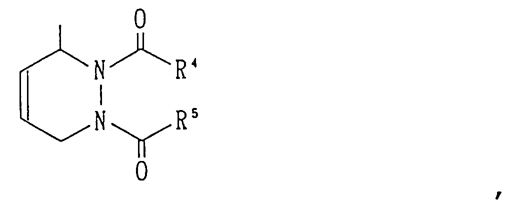 R⁴, R⁵: the same or different from each other and each represents a lower alkoxy group or a substituted or unsubstituted amino group, or R⁴ and R⁵ may be combined together with the neighboring tetrahydropyridazine ring form a substituted or unsubstituted condensed five- or six-membered cyclic group, or
(2) a group represented by the following formula 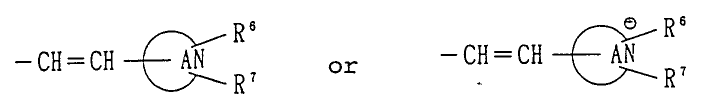 R⁶, R⁷: the same or different from each other and each represents a hydrogen atom, a halogen atom, an amino group, an azide group, a carbamoyl group, an unsubstituted or amino-, azide-, carbamoyl- or halogen-substituted lower alkyl group, a carbamoyl group or a substituted or unsubstituted five- or six-membered heterocyclic group (nitrogen atom of the hetero ring may be substituted to form a quaternary amine together with substituent groups),
X: a methine group (-CH=) or a nitrogen atom, and
ring A: a four- or five-membered nitrogen-containing hetero ring which may contain an oxygen atom) or a salt thereof.

2. The cephalosporin derivative or salt thereof according to claim 1, wherein R³ is represented by the following general formula 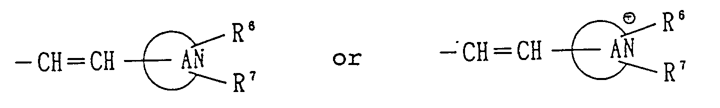 R⁶, R⁷: the same or different from each other and each represents a hydrogen atom, a halogen atom, an amino group, an azide group, an unsubstituted or amino-, azide-, carbamoyl- or halogen-substituted lower alkyl group, a carbamoyl group or a substituted or unsubstituted five- or six-membered heterocyclic group (nitrogen atom of the hetero ring may be substituted to form a quaternary amine together with substituent groups),
X: a methine group (-CH=) or a nitrogen atom, and
A ring: a four- or five-membered nitrogen-containing hetero ring which may contain an oxygen atom.

3. The compound according to claim 1 wherein R³ is represented by the following formula
-CH=CH-Y
(symbols in the formula have the following meanings
Y: a group represented by the following formula 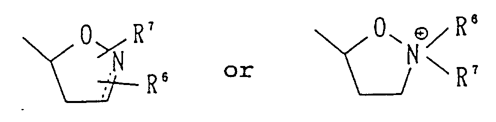 the dotted line may form a double bond, and
R⁶, R⁷: the same or different from each other and each represents a hydrogen atom, a halogen atom, an amino group, an azide group, an unsubstituted or amino-, azide-, carbamoyl- or halogen-substituted lower alkyl group, a carbamoyl group or a substituted or unsubstituted five- or six-membered heterocyclic group (nitrogen atom of the hetero ring may be substituted to form a quaternary amine together with substituent groups)).

4. The compound according to any one of claims 1 to 3, wherein R¹ is a hydrogen atom, a lower alkyl group, a halogeno-lower alkyl group or a carboxy-lower alkyl group.

5. 7β-[(Z)-2-(2-Amino-4-thiazolyl)-2-(methoxyimino)acetamido]-3-[(E)-2-(2,2-dimethyl-5-isooxazolidinio)vinyl]-3-cephem-4-carboxylate or a pharmaceutically acceptable salt thereof.

6. 7β-[(Z)-5-Amino-1,2,4-thiadiazol-3-yl)-2-(methoxyimino)acetamido]-3-[(E)-2-(2,2-dimethyl-5-isooxazolidinio)vinyl]-3-cephem-4-carboxylate or a pharmaceutically acceptable salt thereof.

7. 7β-[(Z)-2-(2-Amino-4-thiazolyl)-2-(fluoromethoxyimino)acetamido]-3-[(E)-2-(2,2-dimethyl-5-isooxazolidinio)vinyl]-3-cephem-4-carboxylate or a pharmaceutically acceptable salt thereof.

8. 7β-[2-(2-Amino-4-thiazolyl)-2-(hydroxyimino)acetamido]-3-[(E)-2-(2,2-dimethyl-5-(isooxazolidinio)vinyl]-3-cephem-4-carboxylate or a pharmaceutically acceptable salt thereof.

9. 7β-[2-(5-Amino-1,2,4-thiadiazol-3-yl)-2-(hydroxyimino)acetamido]-3-[(E)-2-((RS)-2,2-dimethyl-5-isooxazolidinio)vinyl]-3-cephem-4-carboxylate or a pharmaceutically acceptable salt thereof.

10. 7β-[(Z)-2-(5-Amino-1,2,4-thiadiazol-3-yl)-2-(fluoromethoxyimino)acetamido]-3-[(E)-2-(2,2-dimethyl-5-isooxazolidinio)vinyl]-3-cephem-4-carboxylate or a pharmaceutically acceptable salt thereof.

11. 7β-[(Z)-2-(2-Amino-4-thiazolyl)-2-(methoxyimino)acetamido]-3-[(E)-2-(2-methyl-5-isooxazolidinyl)vinyl]-3-cephem-4-carboxylate or a pharmaceutically acceptable salt thereof.

12. 7β-[(Z)-2-(5-Amino-1,2,4-thiadiazol-3-yl)-2-(methoxyimino)acetamido]-3-[(E)-2-(2-methyl-5-isooxazolidinyl)vinyl]-3-cephem-4-carboxylate or a pharmaceutically acceptable salt thereof.

13. A cephalosporin derivative represented by the following general formula (II) 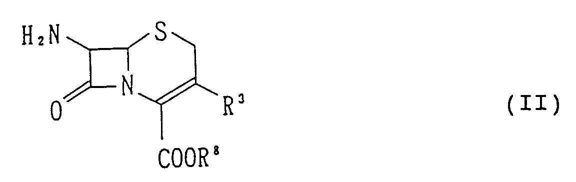 (symbols in the formula have the following meanings
R⁸: a hydrogen atom, a negative charge or a protective group for the carboxyl group,
R³: (1) a group represented by the following formula 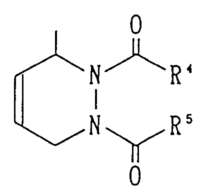 R⁴, R⁵: the same or different from each other and each represents a lower alkoxy group or a substituted or unsubstituted amino group, or R⁴ and R⁵ may be combined together with the neighboring tetrahydropyridazine ring to form a substituted or unsubstituted condensed five- or six-membered cyclic group, or
(2) a group represented by the following formula, 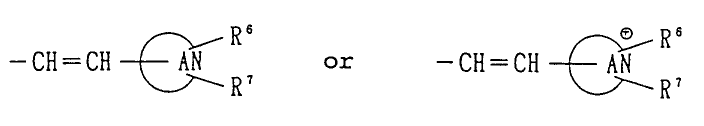 R⁶, R⁷: the same or different from each other and each represents a hydrogen atom, a halogen atom, an amino group, an azide group, an unsubstituted or amino-, azide-, carbamoyl- or halogen-substituted lower alkyl group, a carbamoyl group or a substituted or unsubstituted five- or six-membered heterocyclic group (nitrogen atom of the hetero ring may be substituted to form a quaternary amine together with substituent groups), and
ring A: a four- or five-membered nitrogen-containing hetero ring which may be substituted to contain an oxygen atom), or a pharmaceutically acceptable salt thereof.

14. A cephalosporin derivative represented by the following general formula 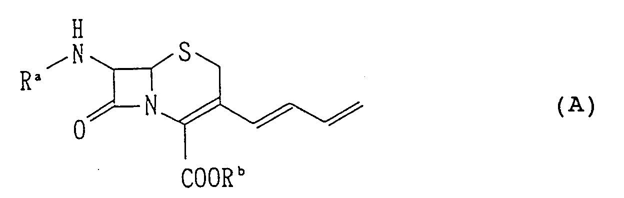 (R^{a}: a hydrogen atom, a phenylacetyl group, a group represented by the following formula 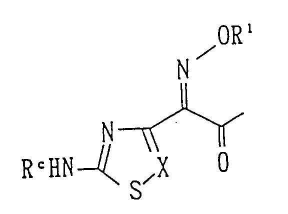 or a protective group for the amino group,
R^{c}: a hydrogen atom or a protective group for the amino group,
X: a methine group (-CH=) or a nitrogen atom,
R¹: a hydrogen atom, a lower alkenyl group, a lower alkynyl group, a cycloalkyl group, an protective group for the hydroxyl group or a substituted or unsubstituted lower alkyl group, and
R^{b}: a hydrogen atom or a protective group for the carboxyl group),
or a pharmaceutically acceptable salt thereof.

15. The cephalosporin derivative or pharmaceutically acceptable salt thereof according to claim 14, wherein R¹ is a hydrogen atom or a substituted or unsubstituted lower alkyl group.
